# EUROPEAN PATENT SPECIFICATION

(11) **EP 2 278 019 B1**
(45) Date of publication and mention of the grant of the patent: **04.06.2014**
(21) Application number: 10010423.1
(22) Date of filing: 22.06.2006
(51) Int. Cl.: C12N 15/82, C07K 14/415

(54) **Nucleotide sequences mediating plant male fertility and method of using same**
Männliche Fruchtbarkeit in Pflanzen vermittelnde Nukleotidsequenzen und Verwendungsverfahren dafür
Séquences de nucléotides facilitant la fertilité mâle des plantes et son procédé d'utilisation

(30) Priority: 24.06.2005 US 166609
(43) Date of publication of application: 26.01.2011
(62) Divisional of application: 06785327.5
(73) Proprietor: PIONEER HI-BRED INTERNATIONAL, INC., Johnston, IA 50131-1014 (US)
(72) Inventor: Albertsen, Marc C., Grimes, Iowa 50111 (US); Fox, Timothy W., Des Moines, Iowa 50310 (US); Hershey, Howard P., Cumming, Iowa 50061 (US); Huffman, Gary A., Des Moines, Iowa 50311 (US); Trimnell, Mary, West Des Moines, Iowa 50266 (US); Wu, Yongzhong, Johnston, Iowa 50131 (US)
(74) Representative: Bentham, Andrew

(56) References cited:
- YOUNG TODD E ET AL: "Cloning of an alpha-amylase cDNA from aleurone tissue of germinating maize seed" PLANT PHYSIOLOGY (ROCKVILLE), vol. 105, no. 2, 1994, pages 759-760, XP002608437 ISSN: 0032-0889

## Description

### BACKGROUND OF THE INVENTION

Development of hybrid plant breeding has made possible considerable advances in quality and quantity of crops produced. Increased yield and combination of desirable characteristics, such as resistance to disease and insects, heat and drought tolerance, along with variations in plant composition are all possible because of hybridization procedures. These procedures frequently rely heavily on providing for a male parent contributing pollen to a female parent to produce the resulting hybrid.

Field crops are bred through techniques that take advantage of the plant's method of pollination. A plant is self-pollinating if pollen from one flower is transferred to the same or another flower of the same plant. A plant is cross-pollinated if the pollen comes from a flower on a different plant.

In *Brassica,* the plant is normally self-sterile and can only be cross-pollinated. In self-pollinating species, such as soybeans and cotton, the male and female plants are anatomically juxtaposed. During natural pollination, the male reproductive organs of a given flower pollinate the female reproductive organs of the same flower.

Maize plants (*Zea mays* L.) present a unique situation in that they can be bred by both self-pollination and cross-pollination techniques. Maize has male flowers, located on the tassel, and female flowers, located on the ear, on the same plant. It can self or cross pollinate. Natural pollination occurs in maize when wind blows pollen from the tassels to the silks that protrude from the tops of the incipient ears.

A reliable method of controlling fertility in plants would offer the opportunity for improved plant breeding. This is especially true for development of maize hybrids, which relies upon some sort of male sterility system and where a female sterility system would reduce production costs.

The development of maize hybrids requires the development of homozygous inbred lines, the crossing of these lines, and the evaluation of the crosses. Pedigree breeding and recurrent selection are two of the breeding methods used to develop inbred lines from populations. Breeding programs combine desirable traits from two or more inbred lines or various broad-based sources into breeding pools from which new inbred lines are developed by selfing and selection of desired phenotypes. A hybrid maize variety is the cross of two such inbred lines, each of which may have one or more desirable characteristics lacked by the other or which complement the other. The new inbreds are crossed with other inbred lines and the hybrids from these crosses are evaluated to determine which have commercial potential. The hybrid progeny of the first generation is designated F₁. In the development of hybrids only the F₁ hybrid plants are sought. The F₁ hybrid is more vigorous than its inbred parents. This hybrid vigor, or heterosis, can be manifested in many ways, including increased vegetative growth and increased yield.

Hybrid maize seed can be produced by a male sterility system incorporating manual detasseling. To produce hybrid seed, the male tassel is removed from the growing female inbred parent, which can be planted in various alternating row patterns with the male inbred parent. Consequently, providing that there is sufficient isolation from sources of foreign maize pollen, the ears of the female inbred will be fertilized only with pollen from the male inbred. The resulting seed is therefore hybrid (F₁) and will form hybrid plants.

Environmental variation in plant development can result in plants tasseling after manual detasseling of the female parent is completed. Or, a detasseler might not completely remove the tassel of a female inbred plant. In any event, the result is that the female plant will successfully shed pollen and some female plants will be self-pollinated. This will result in seed of the female inbred being harvested along with the hybrid seed which is normally produced. Female inbred seed is not as productive as F₁ seed. In addition, the presence of female inbred seed can represent a germplasm security risk for the company producing the hybrid.

Alternatively, the female inbred can be mechanically detasseled by machine. Mechanical detasseling is approximately as reliable as hand detasseling, but is faster and less costly. However, most detasseling machines produce more damage to the plants than hand detasseling. Thus, no form of detasseling is presently entirely satisfactory, and a need continues to exist for alternatives which further reduce production costs and to eliminate self-pollination of the female parent in the production of hybrid seed.

A reliable system of genetic male sterility would provide advantages. The laborious detasseling process can be avoided in some genotypes by using cytoplasmic male-sterile (CMS) inbreds. In the absence of a fertility restorer gene, plants of a CMS inbred are male sterile as a result of factors resulting from the cytoplasmic, as opposed to the nuclear, genome. Thus, this characteristic is inherited exclusively through the female parent in maize plants, since only the female provides cytoplasm to the fertilized seed. CMS plants are fertilized with pollen from another inbred that is not male-sterile. Pollen from the second inbred may or may not contribute genes that make the hybrid plants male-fertile. Usually seed from detasseled normal maize and CMS produced seed of the same hybrid must be blended to insure that adequate pollen loads are available for fertilization when the hybrid plants are grown and to insure cytoplasmic diversity.

There can be other drawbacks to CMS. One is an historically observed association of a specific variant of CMS with susceptibility to certain crop diseases. This problem has discouraged widespread use of that CMS variant in producing hybrid maize and has had a negative impact on the use of CMS in maize in general.

One type of genetic sterility is disclosed in U.S. Patents 4,654,465 and 4,727,219 to Brar, et al. However, this form of genetic male sterility requires maintenance of multiple mutant genes at separate locations within the genome and requires a complex marker system to track the genes and make use of the system convenient. Patterson also described a genic system of chromosomal translocations which can be effective, but which are complicated. (See, U.S. Patents No. 3,861,709 and 3,710,511.)

Many other attempts have been made to improve on these drawbacks. For example, Fabijanski, et al., developed several methods of causing male sterility in plants (see EPO 89/3010153.8 publication no. 329,308 and PCT application PCT/CA90/00037 published as WO 90/08828). One method includes delivering into the plant a gene encoding a cytotoxic substance associated with a male tissue specific promoter. Another involves an antisense system in which a gene critical to fertility is identified and an antisense to the gene inserted in the plant. Mariani, et al. also shows several cytotoxic antisense systems. See EP 89/401, 194. Still other systems use "repressor" genes which inhibit the expression of another gene critical to male sterility. PCT/GB90/00102, published as WO 90/08829.

A still further improvement of this system is one described at U.S. Patent No. 5,478,369 in which a method of imparting controllable male sterility is achieved by silencing a gene native to the plant that is critical for male fertility and replacing the native DNA with the gene critical to male fertility linked to an inducible promoter controlling expression of the gene. The plant is thus constitutively sterile, becoming fertile only when the promoter is induced and its attached male fertility gene is expressed.

In a number of circumstances, a male sterility plant trait is expressed by maintenance of a homozygous recessive condition. Difficulties arise in maintaining the homozygous condition, when a transgenic restoration gene must be used for maintenance. For example, a natural mutation in a gene critical to male sterility can impart a male sterility phenotype to plants when this mutant allele is in the homozygous state. This sterility can be restored when the non-mutant form of the gene is introduced into the plant. However, this form of restoration removes the desired homozygous recessive condition, restores full male fertility and prevents maintenance of pure male sterile maternal lines. This issue can be avoided where production of pollen containing the restoration gene is eliminated, thus providing a maintainer plant producing only pollen not containing the restoration gene, and the progeny retains the homozygous condition. An example of one approach is shown in Dellaporta et al., 6,743,968, in which a plant is produced having a hemizygotic construct comprising a gene that produces a product fatal to a cell, linked with a pollen-specific promoter, and the restoration gene. When crossed with the homozygous recessive male sterile plant, the progeny thus retains the homozygous recessive condition.

As noted, an essential aspect of much of the work underway with male sterility systems is the identification of genes impacting male fertility.

Such a gene can be used in a variety of systems to control male fertility including those described herein. Previously, a male fertility gene has been identified in *Arabidopsis thaliana* and used to produce a male sterile plant. Aarts, et al., "Transposon Tagging of a Male Sterility Gene in Arabidopsis", Nature, 363:715-717 (Jun. 24, 1993). U.S. Patent No. 5,478,369 discloses therein one such gene impacting male fertility.

### SUMMARY OF THE INVENTION

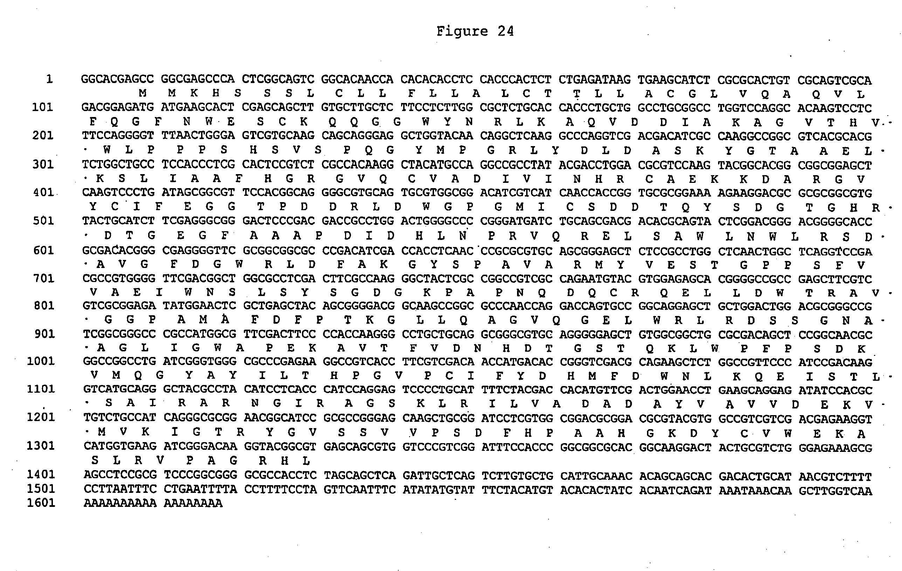

The present invention provides:
[1] An expression cassette comprising an alpha-amylase 1 sequence comprising:
   (a) the alpha-amylase 1 coding region from Zea *mays* as set forth in Figure 24 (SEQ ID NO: 26); or
   (b) a sequence having at least 90% sequence identity thereto.
      operably linked to a male tissue-preferred promoter.
[2] A plant or plant cell comprising an expression cassette of 1.
[3] Use of an expression cassette of 1 in the repression of pollen development.

The plant is preferably a maize, sorghum or soybean plant.

### BRIEF DESCRIPTION OF THE DRAWINGS

Figure 1 is a locus map of the male fertility gene *Ms26.*
Figure 2A is a Southern blot of the *ms26-m2*::*Mu8* family hybridized with a *Mu8* probe;
Figure 2B is a Southern blot of the *ms26-m2*::*Mu8* family hybridized with a Pstl fragment isolated from the *ms26* clone.
Figure 3. is a Northern Blot analysis gel hybridized with a *PstI* fragment isolated from the *Ms26* gene.
Figure 4A-4D is the sequence of *Ms26* (The cDNA is SEQ ID NO: 1, the protein is SEQ ID NOS: 2)
Figure 5A-5C is the genomic *Ms26* sequence (also referred to as SEQ ID NO: 7).
Figure 6A-6D is a comparison of the genomic *Ms26* sequence (Residues 1051-3326 of SEQ ID NO: 7) with the cDNA of *Ms26* (SEQ ID NO: 1).
Figure 7A is a Northern analysis gel showing expression in various plant tissues and Figure 7B is a gel showing expression stages of microsporogenesis
Figure 8 is the full length promoter of *Ms26* (SEQ ID NO: 5)
Figure 9 is a bar graph showing luciferase activity after deletions of select regions of the *Ms26* promoter.
Figure 10 shows essential regions of the *Ms26* promoter (SEQ ID NO: 6).
Figure 11 is a bar graph showing luciferase activity after substitution by restriction site linker scanning of select small (9-10bp) regions of the *Ms26* essential promoter fragment.
Figure 12A and 12B is a comparison of the nucleotide sequence (SEQ ID NO: 3) from the *Ms26* orthologue from a sorghum panicle and *Ms26* maize cDNA (Residues 201-750 of SEQ ID NO: 1), and the sorghum protein sequence (SEQ ID NO: 4) and *Ms26* maize protein (Residues 87-244 of SEQ ID NO: 2).
Figure 13 is a representation of the mapping of the male sterility gene ms26.
Figure 14 shows a sequence comparison of the region of excision of the *ms26-ref* allele (SEQ ID NO: 8) with wild-type *Ms26* (SEQ ID NO: 9).
Figure 15 shows the transposon sequence within *ms26-ref* (SEQ ID NO: 10).
Figure 16 shows the entire *ms26*-*ref* sequence (SEQ ID NO: 11).
Figure 17A shows a translated protein sequence alignment between regions of the CYP704B1, a P450 gene (SEQ ID NO: 12) and *Ms26* (SEQ ID NO: 13); Figure 17B shows the phylogenetic tree analysis of select P450 genes.
Figure 18 demonstrates the heme binding domain frame shift, showing the translated sequence alignment of regions of the *Ms26* cDNA (SEQ ID NOS: 14 and 28-29), the genomic regions of exon 5 in fertile plants (SEQ ID NOS: 15 and 30-31) and sterile plants (SEQ ID NOS: 16 and 32-33).
Figure 19 shows the rice *Ms26* cDNA (SEQ ID NO: 17) and protein (SEQ ID NO: 18)..
Figure 20 shows alignment of the *Ms26* promoter of corn (Residues 650-1089 of SEQ ID NO: 5), sorghum (SEQ ID NO: 19) and rice (SEQ ID NO: 20).
Figure 21 shows alignment of the maize *Ms26* protein (SEQ ID NO: 21); rice *Ms26* protein (SEQ ID NO: 18) and sorghum *Ms26* protein (SEQ ID NO: 22) along with a consensus sequence.
Figure 22 is a plasmid map of PHP 18091, containing Ms45 fertility gene with a pollen promoter, cytotoxic gene and selectable marker.
Figure 23 is a plasmid map of PHP 24101, containing the *Ms26* fertility gene with a pollen promoter, cytotoxic gene and selectable marker.
Figure 24 shows a sequence of the *Zea mays* α-amylase 1 coding region.

### DISCLOSURE OF THE INVENTION

Unless defined otherwise, all technical and scientific terms used herein have the same meaning as commonly understood by one of ordinary skill in the art to which this invention belongs. Unless mentioned otherwise, the techniques employed or contemplated therein are standard methodologies well known to one of ordinary skill in the art. The materials, methods and examples are illustrative only and not limiting.

Genetic male sterility results from a mutation, suppression, or other impact to one of the genes critical to a specific step in microsporogenesis, the term applied to the entire process of pollen formulation. These genes can be collectively referred to as male fertility genes (or, alternatively, male sterility genes). There are many steps in the overall pathway where gene function impacts fertility. This seems aptly supported by the frequency of genetic male sterility in maize. New alleles of male sterility mutants are uncovered in materials that range from elite inbreds to unadapted populations.

The sequences may be isolated from any plant, including, but not limited to corn (*Zea mays*), canola (*Brassica napus, Brassica rapa ssp*.), alfalfa (*Medicago sativa*), rice (*Oryza sativa*), rye (*Secale cereale*), sorghum (*Sorghum bicolor, Sorghum vulgare*), sunflower (*Helianthus annuus*), wheat (*Triticum aestivum*), soybean (*Glycine max*), tobacco (*Nicotiana tabacum*), millet (*Panicum spp*.), potato (*Solanum tuberosum*), peanuts (*Arachis hypogaea*), cotton (*Gossypium hirsutum*), sweet potato (*Ipomoea batatus*), cassava (*Manihot esculenta*), coffee (*Cofea spp*.), coconut (*Cocos nucifera*), pineapple (*Ananas comosus*), citrus trees (*Citrus spp*.), cocoa (*Theobroma cacao*), tea (*Camellia sinensis*), banana (*Musa spp*.), avocado (*Persea americana*), fig (*Ficus casica*), guava (*Psidium guajava*), mango (*Mangifera indica*), olive (*Olea europaea*), oats (*Avena sativa*), barley (*Hordeum vulgare*), vegetables, ornamentals, and conifers. Preferably, plants include corn, soybean, and sorghum.

Sequences from other plants may be isolated according to well-known techniques based on their sequence homology to the homologous coding region of the coding sequences set forth herein. In these techniques, all or part of the known coding sequence is used as a probe which selectively hybridizes to other sequences present in a population of cloned genomic DNA fragments (i.e. genomic libraries) from a chosen organism. Methods are readily available in the art for the hybridization of nucleic acid sequences. An extensive guide to the hybridization of nucleic acids is found in Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel, et al., Eds., Greene Publishing and Wiley-Interscience, New York (1995).

The following terms are used to describe the sequence relationships between two or more nucleic acids or polynucleotides: (a) "reference sequence", (b) "comparison window", (c) "sequence identity", and (d) "percentage of sequence identity."
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (*i.e*., gaps) compared to the reference sequence (which does not comprise additions or deletions) for optimal alignment of the two sequences. Generally, the comparison window is at least 20 contiguous nucleotides in length, and optionally can be 30, 40, 50, or 100 nucleotides in length, or longer. Those of skill in the art understand that to avoid a high similarity to a reference sequence due to inclusion of gaps in the polynucleotide sequence a gap penalty is typically introduced and is subtracted from the number of matches.

Methods of aligning sequences for comparison are well-known in the art. Thus, the determination of percent sequence identity between any two sequences can be accomplished using a mathematical algorithm. Non-limiting examples of such mathematical algorithms are the algorithm of Myers and Miller (1988) CABIOS 4: 11-17; the local alignment algorithm of Smith et al. (1981) Adv. Appl. Math. 2: 482; the global alignment algorithm of Needleman and Wunsch (1970) J. Mol. Biol. 48: 443-453; the search-for-local-alignment-method of Pearson and Lipman (1988) Proc. Natl. Acad. Sci. 85: 2444-2448; the algorithm of Karlin and Altschul (1990) Proc. Natl. Acad. Sci. USA 87: 2264, modified as in Karlin and Altschul (1993) Proc. Natl. Acad. Sci. USA 90: 5873-5877.

Computer implementations of these mathematical algorithms can be utilized for comparison of sequences to determine sequence identity. Such implementations include, but are not limited to: CLUSTAL in the PC/Gene program.. (available from Intelligenetics, Mountain View, California); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, California, USA). Alignments using these programs can be performed using the default parameters. The CLUSTAL program is well described by Higgins et al. (1988) Gene 73: 237-244 (1988); Higgins et al. (1989) CABIOS 5: 151-153; Corpet et al. (1988) Nucleic Acids Res. 16: 10881-90; Huang et al. (1992) CABIOS 8: 155-65; and Pearson et al. (1994) Meth. Mol. Biol. 24: 307-331. The ALIGN program is based on the algorithm of Myers and Miller (1988) *supra.* A PAM120 weight residue table, a gap length penalty of 12, and a gap penalty of 4 can be used with the ALIGN program when comparing amino acid sequences. The BLAST programs of Altschul et al (1990) J. Mol. Biol. 215: 403 are based on the algorithm of Karlin and Altschul (1990) *supra.* BLAST nucleotide searches can be performed with the BLASTN program, score = 100, wordlength = 12, to obtain nucleotide sequences homologous to a nucleotide sequence encoding a protein. BLAST protein searches can be performed with the BLASTX program, score = 50, wordlength = 3, to obtain amino acid sequences homologous to a protein or polypeptide. To obtain gapped alignments for comparison purposes, Gapped BLAST (in BLAST 2.0) can be utilized as described in Altschul et al. (1997) Nucleic Acids Res. 25: 3389. Alternatively, PSI-BLAST (in BLAST 2.0) can be used to perform an iterated search that detects distant relationships between molecules. See Altschul *et al.* (1997) *supra.* When utilizing BLAST, Gapped BLAST, PSI-BLAST, the default parameters of the respective programs (*e.g*., BLASTN for nucleotide sequences, BLASTX for proteins) can be used. See http://www.ncbi.nlm.nih.gov. Alignment may also be performed manually by inspection.

Unless otherwise stated, sequence identity/similarity values provided herein refer to the value obtained using GAP Version 10 using the following parameters: % identity and % similarity for a nucleotide sequence using GAP Weight of 50 and Length Weight of 3 and the nwsgapdna.cmp scoring matrix; % identity and % similarity for an amino acid sequence using GAP Weight of 8 and Length Weight of 2; and the BLOSUM62 scoring matrix or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by GAP Version 10.

GAP uses the algorithm of Needleman and Wunsch (1970) J. Mol. Biol. 48: 443-453, to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the GCG Wisconsin Genetics Software Package for protein sequences are 8 and 2, respectively. For nucleotide sequences the default gap creation penalty is 50 while the default gap extension penalty is 3. The gap creation and gap extension penalties can be expressed as an integer selected from the group of integers consisting of from 0 to 200. Thus, for example, the gap creation and gap extension penalties can be 0, 1, 2, 3, 4, 5, 6, 7, 8, 9, 10, 15, 20, 25, 30, 35, 40, 45, 50, 55, 60, 65 or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the GCG Wisconsin Genetics Software Package is BLOSUM62 (see Henikoff and Henikoff (1989) Proc. Natl. Acad. Sci. USA 89:10915).
(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, *e.g*., as implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (*i.e*., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

Identity to the sequence of the present invention would mean a polynucleotide sequence having at least 90%, 91%, 92%, 93%, 94%, 95%, 96%, 97%, 98%, 99% sequence identity.

Promoter regions can be readily identified by one skilled in the art. The putative start codon containing the ATG motif is identified and upstream from the start codon is the presumptive promoter. By "promoter" is intended a regulatory region of DNA usually comprising a TATA box capable of directing RNA polymerase II to initiate RNA synthesis at the appropriate transcription initiation site for a particular coding sequence. A promoter can additionally comprise other recognition sequences generally positioned upstream or 5' to the TATA box, referred to as upstream promoter elements, which influence the transcription initiation rate. It is recognized that having identified the nucleotide sequences for the promoter region disclosed herein, it is within the state of the art to isolate and identify further regulatory elements in the region upstream of the TATA box from the particular promoter region identified herein. Thus the promoter region disclosed herein is generally further defined by comprising upstream regulatory elements such as those responsible for tissue and temporal expression of the coding sequence, enhancers and the like. In the same manner, the promoter elements which enable expression in the desired tissue such as male tissue can be identified, isolated, and used with other core promoters to confirm male tissue-preferred expression. By core promoter is meant the minimal sequence required to initiate transcription, such as the sequence called the TATA box which is common to promoters in genes encoding proteins. Thus the upstream promoter of *Ms26* can optionally be used in conjunction with its own or core promoters from other sources. The promoter may be native or non-native to the cell in which it is found.

The isolated promoter sequence of the present disclosure can be modified to provide for a range of expression levels of the heterologous nucleotide sequence. Less than the entire promoter region can be utilized and the ability to drive anther-preferred expression retained. However, it is recognized that expression levels of mRNA can be decreased with deletions of portions of the promoter sequence. Thus, the promoter can be modified to be a weak or strong promoter. Generally, by "weak promoter" is intended a promoter that drives expression of a coding sequence at a low level. By "low level" is intended levels of about 1/10,000 transcripts to about 1/100,000 transcripts to about 1/500,000 transcripts. Conversely, a strong promoter drives expression of a coding sequence at a high level, or at about 1/10 transcripts to about 1/100 transcripts to about 1/1,000 transcripts. Generally, at least about 30 nucleotides of an isolated promoter sequence will be used to drive expression of a nucleotide sequence. It is recognized that to increase transcription levels, enhancers can be utilized in combination with the promoter regions of the disclosure. Enhancers are nucleotide sequences that act to increase the expression of a promoter region. Enhancers are known in the art and include the SV40 enhancer region, the 35S enhancer element, and the like.

The promoter of the present disclosure can be isolated from the 5' region of its native coding region of 5' untranslation region (5'UTR). Likewise the terminator can be isolated from the 3' region flanking its respective stop codon. The term "isolated" refers to material such as a nucleic acid or protein which is substantially or essentially free from components which normally accompany or interact with the material as found in it naturally occurring environment or if the material is in its natural environment, the material has been altered by deliberate human intervention to a composition and/or placed at a locus in a cell other than the locus native to the material. Methods for isolation of promoter regions are well known in the art.

"Functional variants" of the regulatory sequences are also encompassed by the compositions of the present invention. Functional variants include, for example, the native regulatory sequences of the invention having one or more nucleotide substitutions, deletions or insertions. Functional variants of the invention may be created by site-directed mutagenesis, induced mutation, or may occur as allelic variants (polymorphisms).

As used herein, a "functional fragment" of the regulatory sequence is a nucleotide sequence that is a regulatory sequence variant formed by one or more deletions from a larger sequence. For example, the 5' portion of a promoter up to the TATA box near the transcription start site can be deleted without abolishing promoter activity, as described by Opsahl-Sorteberg, H-G. et al., "Identification of a 49-bp fragment of the HvLTP2 promoter directing aleruone cell specific expression" Gene 341:49-58 (2004). Such variants should retain promoter activity, particularly the ability to drive expression in male tissues. Activity can be measured by Northern blot analysis, reporter activity measurements when using transcriptional fusions, and the like. See, for example, Sambrook et al. (1989) Molecular Cloning: A Laboratory Manual (2nd ed. Cold Spring Harbor Laboratory, Cold Spring Harbor, N.Y.).

Functional fragments can be obtained by use of restriction enzymes to cleave the naturally occurring regulatory element nucleotide sequences disclosed herein; by synthesizing a nucleotide sequence from the naturally occurring DNA sequence; or can be obtained through the use of PCR technology See particularly, Mullis et al. (1987) Methods Enzymol. 155:335-350, and Erlich, ed. (1989) PCR Technology (Stockton Press, New York).

Sequences which hybridize to the regulatory sequences of the present invention are within the scope of the invention. Sequences that correspond to the promoter sequences of the present invention and hybridize to the promoter sequences disclosed herein will be at least 50% homologous, 70% homologous, and even 85% 86%, 87%, 88%, 89%, 90%, 91%, 92%, 93%, 94%, 95%, 96%, 97%, 98%, 99% homologous or more with the native regulatory sequence.

Promoter sequences from other plants may be isolated according to well-known techniques based on their sequence homology to the promoter sequence set forth herein. In these techniques, all or part of the known promoter sequence is used as a probe which selectively hybridizes to other sequences present in a population of cloned genomic DNA fragments (i.e. genomic libraries) from a chosen organism. Methods are readily available in the art for the hybridization of nucleic acid sequences.

The entire promoter sequence or portions thereof can be used as a probe capable of specifically hybridizing to corresponding promoter sequences. To achieve specific hybridization under a variety of conditions, such probes include sequences that are unique and are preferably at least about 10 nucleotides in length, and most preferably at least about 20 nucleotides in length. Such probes can be used to amplify corresponding promoter sequences from a chosen organism by the well-known process of polymerase chain reaction (PCR). This technique can be used to isolate additional promoter sequences from a desired organism or as a diagnostic assay to determine the presence of the promoter sequence in an organism. Examples include hybridization screening of plated DNA libraries (either plaques or colonies; see e.g. Innis et al., eds., (1990) PCR Protocols, A Guide to Methods and Applications, Academic Press).

Further, a promoter of the present disclosure can be linked with nucleotide sequences to express other heterologous nucleotide sequences. The nucleotide sequence for the promoter of the disclosure, as well as fragments and variants thereof, can be provided in expression cassettes along with heterologous nucleotide sequences for expression in the plant of interest, more particularly in the male tissue of the plant. Such an expression cassette is provided with a plurality of restriction sites for insertion of the nucleotide sequence to be under the transcriptional regulation of the promoter. These expression cassettes are useful in the genetic manipulation of any plant to achieve a desired phenotypic response.

The range of available plant compatible promoters includes tissue specific and inducible promoters. An inducible regulatory element is one that is capable of directly or indirectly activating transcription of one or more DNA sequences or genes in response to an inducer. In the absence of an inducer the DNA sequences or genes will not be transcribed. Typically the protein factor that binds specifically to an inducible regulatory element to activate transcription is present in an inactive form which is then directly or indirectly converted to the active form by the inducer. The inducer can be a chemical agent such as a protein, metabolite, growth regulator, herbicide or phenolic compound or a physiological stress imposed directly by heat, cold, salt, or toxic elements or indirectly through the actin of a pathogen or disease agent such as a virus. A plant cell containing an inducible regulatory element may be exposed to an inducer by externally applying the inducer to the cell or plant such as by spraying, watering, heating or similar methods.

Tissue-preferred promoters can be utilized to target enhanced transcription and/or expression within a particular plant tissue.

The promoters are those which preferentially express to the male tissue of the plant. The invention does not require that any particular male tissue-preferred promoter be used in the process, and any of the many such promoters known to one skilled in the art may be employed. The native *Ms26* promoter described herein is one example of a useful promoter. Another such promoter is the 5126 promoter, which preferentially directs expression of the gene to which it is linked to male tissue of the plants, as described in U.S. Patents Nos. 5,837,851 and 5,689,051. Other examples include the *Ms45* promoter described at US Patent No. 6,037,523; SF3 promoter described at U.S. Patent No. 6,452,069; the BS92-7 promoter described at WO 02/063021; a SGB6 regulatory element described at U.S. Patent No. 5,470,359; the TA29 promoter (Koltunow et al. (1990) "Different temporal and spatial gene expression patterns occur during anther development." Plant Cell 2:1201-1224; Goldberg, R. B., Beals, T. P. and Sanders, P. M., (1993) "Anther development: basic principles and practical applications" Plant Cell 5:1217-1229; and US Patent No. 6,399,856); the type 2 metallothionein-like gene promoter (Charbonnel-Campaa et al., Gene (2000) 254:199-208); and the *Brassica* Bca9 promoter (Lee et al., Plant Cell Rep. (2003) 22:268-273).

Male gamete preferred promoters include the PG47 promoter, *supra* as well as ZM13 promoter (Hamilton et al., Plant Mol. Biol. (1998) 38:663-669); actin depolymerizing factor promoters (such as Zmabp1, Zmabp2; see for example Lopez et al. Proc. Natl. Acad. Sci. USA (1996) 93: 7415-7420); the promoter of the maize petctin methylesterase-liked gene, ZmC5 ( Wakeley et al. Plant Mol. Biol. (1998) 37:187-192); the profiling gene promoter Zmpro1 (Kovar et al., The Plant Cell (2000) 12:583-598); the sulphated pentapeptide phytosulphokine gene ZmPSK1 ( Lorbiecke et al., Journal of Experimental Botany (2005) 56(417): 1805-1819); the promoter of the calmodulin binding protein Mpcbp (Reddy et al. J. Biol. Chem. (2000) 275(45):35457-70).

Other components of the vector may be included, also depending upon intended use of the gene. Examples include selectable markers, targeting or regulatory sequences, stabilizing or leader sequences, introns etc. General descriptions and examples of plant expression vectors and reporter genes can be found in Gruber, et al., "Vectors for Plant Transformation" in Method in Plant Molecular Biology-and Biotechnology, Glick et al eds;CRC Press pp. 89-119 (1993). The selection of an appropriate expression vector will depend upon the host and the method of introducing the expression vector into the host. The expression cassette will also include at the 3' terminus of the heterologous nucleotide sequence of interest, a transcriptional and translational termination region functional in plants. The termination region can be native with the promoter nucleotide sequence of the present invention, can be native with the DNA sequence of interest, or can be derived from another source. Convenient termination regions are available from the Ti-plasmid of *A*. *tumefaciens,* such as the octopine synthase and nopaline synthase termination regions. See also, Guerineau et al. Mol. Gen. Genet. 262:141-144 (1991); Proudfoot, Cell 64:671-674 (1991); Sanfacon et al. Genes Dev. 5:141-149 (1991); Mogen et al. Plant Cell 2:1261-1272 (1990); Munroe et al. Gene 91:151-158 (1990); Ballas et al. Nucleic Acids Res. 17:7891-7903 (1989); Joshi et al. Nucleic Acid Res. 15:9627-9639 (1987).

The expression cassettes can additionally contain 5' leader sequences. Such leader sequences can act to enhance translation. Translation leaders are known in the art and include by way of example, picornavirus leaders, EMCV leader (Encephalomyocarditis 5' noncoding region), Elroy-Stein et al. Proc. Nat Acad. Sci. USA 86:6126-6130 (1989); potyvirus leaders, for example, TEV leader (Tobacco Etch Virus), Allison et al.; MDMV leader (Maize Dwarf Mosaic Virus), Virology 154:9-20 (1986); human immunoglobulin heavy-chain binding protein (BiP), Macejak et al. Nature 353:90-94 (1991); untranslated leader from the coat protein mRNA of alfalfa mosaic virus (AMV RNA 4), Jobling et al. Nature 325:622-625 (1987); Tobacco mosaic virus leader (TMV), Gallie et al. (1989) Molecular Biology of RNA, pages 237-256; and maize chlorotic mottle virus leader (MCMV) Lommel et al. Virology 81:382-385 (1991). See also Della-Cioppa et al. Plant Physiology 84:965-968 (1987). The cassette can also contain sequences that enhance translation and/or mRNA stability such as introns.

In those instances where it is desirable to have the expressed product of the heterologous nucleotide sequence directed to a particular organelle, particularly the plastid, amyloplast, or to the endoplasmic reticulum, or secreted at the cell's surface or extracellularly, the expression cassette can further comprise a coding sequence for a transit peptide. Such transit peptides are well known in the art and include, but are not limited to, the transit peptide for the acyl carrier protein, the small subunit of RUBISCO, plant EPSP synthase, *Zea mays* Brittle-1 chloroplast transit peptide (Nelson et al. Plant physiol 117(4):1235-1252 (1998); Sullivan et al. Plant Cell 3(12):1337-48; Sullivan et al., Planta (1995) 196(3):477-84; Sullivan et al., J. Biol. Chem. (1992) 267(26):18999-9004) and the like. One skilled in the art will readily appreciate the many options available in expressing a product to a particular organelle. For example, the barley alpha amylase sequence is often used to direct expression to the endoplasmic reticulum (Rogers, J. Biol. Chem. 260:3731-3738 (1985)). Use of transit peptides is well known (*e.g*., *see* U.S. Patents Nos. 5,717,084; 5,728,925).

In preparing the expression cassette, the various DNA fragments can be manipulated, so as to provide for the DNA sequences in the proper orientation and, as appropriate, in the proper reading frame. Toward this end, adapters or linkers can be employed to join the DNA fragments or other manipulations can be involved to provide for convenient restriction sites, removal of superfluous DNA, removal of restriction sites, or the like. For this purpose, *in vitro* mutagenesis, primer repair, restriction digests, annealing, and resubstitutions, such as transitions and transversions, can be involved.

As noted herein, the present disclosure provides vectors capable of expressing genes of interest. In general, the vectors should be functional in plant cells. At times, it may be preferable to have vectors that are functional in *E*. *coli* (e.g., production of protein for raising antibodies, DNA sequence analysis, construction of inserts, obtaining quantities of nucleic acids). Vectors and procedures for cloning and expression in *E*. *coli* are discussed in Sambrook et al. (*supra*).

The transformation vector comprising the promoter sequence of the present disclosure operably linked to a heterologous nucleotide sequence in an expression cassette, can also contain at least one additional nucleotide sequence for a gene to be cotransformed into the organism. Alternatively, the additional sequence(s) can be provided on another transformation vector.

Reporter genes can be included in the transformation vectors. Examples of suitable reporter genes known in the art can be found in, for example, Jefferson et al. (1991) in Plant Molecular Biology Manual, ed. Gelvin et al. (Kluwer Academic Publishers), pp. 1-33; DeWet et al. Mol. Cell. Biol. 7:725-737 (1987); Goff et al. EMBO J. 9:2517-2522 (1990); Kain et al. BioTechniques 19:650-655 (1995); and Chiu et al. Current Biology 6:325-330 (1996).

Selectable reporter genes for selection of transformed cells or tissues can be included in the transformation vectors. These can include genes that confer antibiotic resistance or resistance to herbicides. Examples of suitable selectable marker genes include, but are not limited to, genes encoding resistance to chloramphenicol, Herrera Estrella et al. EMBO J. 2:987-992(1983); methotrexate, Herrera Estrella et al. Nature 303:209-213(1983); Meijer et al. Plant Mol. Biol. 16:807-820 (1991); hygromycin, Waldron et al. Plant Mol. Biol. 5:103-108 (1985), Zhijian et al. Plant Science 108:219-227 (1995); streptomycin, Jones et al. Mol. Gen. Genet. 210:86-91(1987); spectinomycin, Bretagne-Sagnard et al. Transgenic Res. 5:131-137 (1996); bleomypin, Hille et al. Plant Mol. Biol. 7:171-176 (1990); sulfonamide, Guerineau et al. Plant Mol. Biol. 15:127-136(1990); bromoxynil, Stalker et al. Science 242:419-423 (1988); glyphosate, Shaw et al. Science 233:478-481(1986); and phosphinothricin, DeBlock et al. EMBO J. 6:2513-2518 (1987).

Scorable or screenable markers may also be employed, where presence of the sequence produces a measurable product. Examples include a β- glucuronidase, or uidA gene (GUS), which encodes an enzyme for which various chromogenic substrates are known (for example, US Patents 5,268,463 and 5,599,670); chloramphenicol acetyl transferase (Jefferson et al. The EMBO Journal vol. 6 No. 13 pp. 3901-3907); and alkaline phosphatase. Other screenable markers include the anthocyanin/flavonoid genes in general (See discussion at Taylor and Briggs, The Plant Cell (1990)2:115-127) including, for example, a *R*-locus gene, which encodes a product that regulates the production of anthocyanin pigments (red color) in plant tissues (Dellaporta et al., in Chromosome Structure and Function, Kluwer Academic Publishers, Appels and Gustafson eds., pp. 263-282 (1988)); the genes which control biosynthesis of flavonoid pigments, such as the maize *C1* gene (Kao et al., Plant Cell (1996) 8: 1171-1179; Scheffler et al. Mol. Gen. Genet. (1994) 242:40-48) and maize *C2* (Wienand et al., Mol. Gen. Genet. (1986) 203:202-207); the B gene (Chandler et al., Plant Cell (1989) 1:1175-1183), the *p1* gene (Grotewold et al, Proc. Natl. Acad. Sci USA (1991) 88:4587-4591; Grotewold et al., Cell (1994) 76:543-553; Sidorenko et al., Plant Mol. Biol. (1999)39:11-19); the *bronze* locus genes (Ralston et al., Genetics (1988) 119:185-197; Nash et al., Plant Cell (1990) 2(11): 1039-1049), among others. Yet further examples of suitable markers include the cyan fluorescent protein (CYP) gene (Bolte et al. (2004) J. Cell Science 117: 943-54 and Kato et al. (2002) Plant Physiol 129: 913-42), the yellow fluorescent protein gene (PhiYFP™ from Evrogen; see Bolte et al. (2004) J. Cell Science 117: 943-54); a lux gene, which encodes a luciferase, the presence of which may be detected using, for example, X-ray film, scintillation counting, fluorescent spectrophotometry, low-light video cameras, photon counting cameras or multiwell luminometry (Teeri et al. (1989) EMBO J. 8:343); a green fluorescent protein (GFP) gene (Sheen et al., Plant J. (1995) 8(5):777-84); and DsRed2 where plant cells transformed with the marker gene are red in color, and thus visually selectable (Dietrich et al. (2002) Biotechniques 2(2):286-293). Additional examples include a p-lactamase gene (Sutcliffe, Proc. Nat'l. Acad. Sci. U.S.A. (1978) 75:3737), which encodes an enzyme for which various chromogenic substrates are known (e.g., PADAC, a chromogenic cephalosporin); a xyIE gene (Zukowsky et al., Proc. Nat'l. Acad. Sci. U.S.A. (1983) 80:1101), which encodes a catechol dioxygenase that can convert chromogenic catechols; an α-amylase gene (Ikuta et al., Biotech. (1990) 8:241); and a tyrosinase gene (Katz et al., J. Gen. Microbiol. (1983) 129:2703), which encodes an enzyme capable of oxidizing tyrosine to DOPA and dopaquinone, which in turn condenses to form the easily detectable compound melanin. Clearly, many such markers are available to one skilled in the art.

The method of transformation/transfection is not critical to the present disclosure; various methods of transformation or transfection are currently available. As newer methods are available to transform crops or other host cells they may be directly applied. Accordingly, a wide variety of methods have been developed to insert a DNA sequence into the genome of a host cell to obtain the transcription or transcript and translation of the sequence to effect phenotypic changes in the organism. Thus, any method which provides for efficient transformation/transfection may be employed.

Methods for introducing expression vectors into plant tissue available to one skilled in the art are varied and will depend on the plant selected. Procedures for transforming a wide variety of plant species are well known and described throughout the literature. See, for example, Miki et al, "Procedures for Introducing Foreign DNA into Plants" in Methods in Plant Molecular Biotechnology, *supra*; Klein et al, Bio/Technology 10:268 (1992); and Weising et al., Ann. Rev. Genet. 22: 421-477 (1988). For example, the DNA construct may be introduced into the genomic DNA of the plant cell using techniques such as microprojectile-mediated delivery, Klein et al., Nature 327: 70-73 (1987); electroporation, Fromm et al., Proc. Natl. Acad. Sci. 82: 5824 (1985); polyethylene glycol (PEG) precipitation, Paszkowski et al., EMBO J. 3: 2717-2722 (1984); direct gene transfer WO 85/01856 and EP No. 0 275 069; *in vitro* protoplast transformation, U.S. Patent No. 4,684,611; and microinjection of plant cell protoplasts or embryogenic callus, Crossway, Mol. Gen. Genetics 202:179-185 (1985). Co-cultivation of plant tissue with *Agrobacterium tumefaciens* is another option, where the DNA constructs are placed into a binary vector system. *See e.g.,* U.S. Patent No. 5,591,616; Ishida et al., "High Efficiency Transformation of Maize (Zea mays L.) mediated by Agrobacterium tumefaciens" Nature Biotechnology 14:745-750 (1996). The virulence functions of the *Agrobacterium tumefaciens* host will direct the insertion of the construct into the plant cell DNA when the cell is infected by the bacteria. See, for example Horsch et al., Science 233: 496-498 (1984), and Fraley et al., Proc. Natl. Acad. Sci. 80: 4803 (1983).

Standard methods for transformation of canola are described at Moloney et al. "High Efficiency Transformation of Brassica napus using Agrobacterium Vectors" Plant Cell Reports 8:238-242 (1989). Corn transformation is described by Fromm et al, Bio/Technology 8:833 (1990) and Gordon-Kamm et al, *supra. Agrobacterium* is primarily used in dicots, but certain monocots such as maize can be transformed by *Agrobacterium. See supra* and U.S. Patent No. 5,550,318. Rice transformation is described by Hiei et al., "Efficient Transformation of Rice (Oryza sativs L.) Mediated by Agrobacterium and Sequence Analysis of the Boundaries of the T-DNA" The Plant Journal 6(2): 271-282 (1994, Christou et al, Trends in Biotechnology 10:239 (1992) and Lee et al, Proc. Nat'l Acad. Sci. USA 88:6389 (1991). Wheat can be transformed by techniques similar to those used for transforming corn or rice. Sorghum transformation is described at Casas et al, *supra* and sorghum by Wan et al, Plant Physicol. 104:37 (1994). Soybean transformation is described in a number of publications, including U.S. Patent No. 5,015,580.

When referring to "introduction" of the nucleotide sequence into a plant, it is meant that this can occur by direct transformation methods, such as *Agrobacterium* transformation of plant tissue, microprojectile bombardment, electroporation, or any one of many methods known to one skilled in the art; or, it can occur by crossing a plant having the heterologous nucleotide sequence with another plant so that progeny have the nucleotide sequence incorporated into their genomes. Such breeding techniques are well known to one skilled in the art.

The plant breeding methods used herein are well known to one skilled in the art. For a discussion of plant breeding techniques, see Poehlman (1987) Breeding Field Crops. AVI Publication Co., Westport Conn. Many of the plants which would be most preferred in this method are bred through techniques that take advantage of the plant's method of pollination.

Backcrossing methods may be used to introduce a gene into the plants. This technique has been used for decades to introduce traits into a plant. An example of a description of this and other plant breeding methodologies that are well known can be found in references such as Plant Breeding Methodology, edit. Neal Jensen, John Wiley & Sons, Inc. (1988). In a typical backcross protocol, the ' original variety of interest (recurrent parent) is crossed to a second variety (nonrecurrent parent) that carries the single gene of interest to be transferred. The resulting progeny from this cross are then crossed again to the recurrent parent and the process is repeated until a plant is obtained wherein essentially all of the desired morphological and physiological characteristics of the recurrent parent are recovered in the converted plant, in addition to the single transferred gene from the nonrecurrent parent.

In certain situations, it is desirable to maintain the male sterile homozygous recessive condition of a male sterile plant, when using a transgenic restoration approach, while decreasing the number of plants, plantings and steps needed for maintenance plant with such traits. Homozygosity is a genetic condition existing when identical alleles reside at corresponding loci on homologous chromosomes. Heterozygosity is a genetic condition existing when different alleles reside at corresponding loci on homologous chromosomes. Hemizygosity is a genetic condition existing when there is only one copy of a gene (or set of genes) with no allelic counterpart on the sister chromosome. In an embodiment, the homozygous recessive condition results in conferring on the plant a trait of interest, which can be any trait desired and which results from the recessive genotype, such as increased drought or cold tolerance, early maturity, changed oil or protein content, or any of a multitude of the many traits of interest to plant breeders. The homozygous recessive condition may confer male sterility upon the plant. When the sequence which is the functional complement of the homozygous condition is introduced into the plant (that is, a sequence which, when introduced into and expressed in the plant having the homozygous recessive condition, restores the wild-type condition), fertility is restored by virtue of restoration of the wild-type fertile phenotype.

Maintenance of the homozygous recessive condition is achieved by introducing a restoration transgene construct into a plant that is linked to a sequence which interferes with the function or formation of male gametes of the plant to create a maintainer or donor plant. The restoring transgene, upon introduction into a plant that is homozygous recessive for the genetic trait, restores the genetic function of that trait, with the plant producing only viable pollen containing a copy of the recessive allele but does not contain the restoration transgene. The transgene is kept in the hemizygous state in the maintainer plant. By transgene, it is meant any nucleic acid sequence which is introduced into the genome of a cell by genetic engineering techniques. A transgene may be a native DNA sequence, or a heterologous DNA sequence (i.e., "foreign DNA"). The term native DNA sequence refers to a nucleotide sequence which is naturally found in the cell but that may have been modified from its original form. The pollen from the maintainer can be used to fertilize plants that are homozygous for the recessive trait, and the progeny will therefore retain their homozygous recessive condition. The maintainer plant containing the restoring transgene construct is propagated by self-fertilization, with the resulting seed used to produce further plants that are homozygous recessive plants and contain the restoring transgene construct.

The maintainer plant serves as a pollen donor to the plant having the homozygous recessive trait. The maintainer is optimally produced from a plant having the homozygous recessive trait and which also has nucleotide sequences introduced therein which would restore the trait created by the homozygous recessive alleles. Further, the restoration sequence is linked to nucleotide sequences which interfere with the function or formation of male gametes. The gene can operate to prevent formation of male gametes or prevent function of the male gametes.

In this invention, an α-amylase gene is used with a male tissue-preferred promoter. During the initial germinating period of cereal seeds, the aleurone layer cells will synthesize α-amylase, which participates in hydrolyzing starch to form glucose and maltose, so as to provide the nutrients needed for the growth of the germ (J. C. Rogers and C. Milliman, J. Biol. Chem., 259 (19): 12234-12240, 1984; Rogers, J. C., J. Biol. Chem., 260: 3731-3738, 1985). The α-amylase gene used can be the *Zea mays* α-amylase-1 gene. Young et al. "Cloning of an α-amylase cDNA from aleurone tissue of germinating maize seed" Plant Physiol. 105(2) 759-760 and GenBank accession No. L25805, GI:426481). Sequences encoding α-amylase are not typically found in pollen cells, and when expression is directed to male tissue, the result is a breakdown of the energy source for the pollen grains, and repression of pollen development.

One skilled in this area readily appreciates the methods described herein are applicable to any other crops which have the potential to outcross. By way of example, but not limitation it can include maize, soybean, sorghum, or any plant with the capacity to outcross.

Ordinarily, to produce more plants having the recessive condition, one might cross the recessive plant with another recessive plant. This may not be desirable for some recessive traits and may be impossible for recessive traits affecting reproductive development. Alternatively, one could cross the homozygous plant with a second plant having the restoration gene, but this requires further crossing to segregate away the restoring gene to once again reach the recessive phenotypic state. Instead, in one process the homozygous recessive condition can be maintained, while crossing it with the maintainer plant. This method can be used with any situation in which is it desired to continue the recessive condition. This results in a cost-effective system that is relatively easy to operate to maintain a population of homozygous recessive plants.

Further detailed description is provided below by way of instruction and illustration and is not intended to limit the scope of the invention.

### REFERENCE EXAMPLE 1

### Identification and Cosegregation of ms26-m2::Mu8

Families of plants from a Mutator (*Mu*) population were identified that segregated for plants that were mostly male sterile, with none or only a few extruded abnormal anthers, none of which had pollen present. Male sterility is expected to result from those instances where a *Mu* element has randomly integrated into a gene responsible for some step in microsporogenesis, disrupting its expression. Plants from a segregating F₂ family in which the male sterile mutation was designated *ms26**-SBMu200, were grown and classified for male fertility/sterility based on the above criteria. Leaf samples were taken and DNA subsequently isolated on approximately 20 plants per phenotypic classification, that is male fertility vs. male sterility.

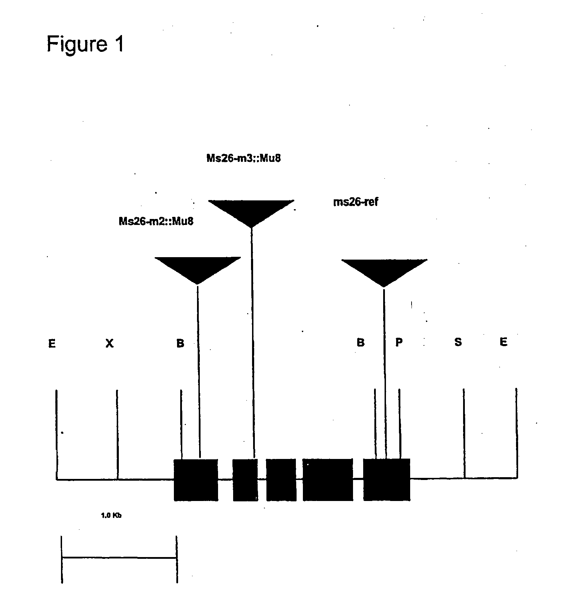

Southern analysis was performed to confirm association of *Mu* with sterility. Southern analysis is a well known technique to those skilled in the art. This common procedure involves isolating the plant DNA, cutting with restriction endonucleases, fractioning the cut DNA by molecular weight on an agarose gel, and transferring to nylon membranes to fix the separated DNA. These membranes are subsequently hybridized with a probe fragment that was radioactively labeled with P³²P-dCTP, and washed in an SDS solution. Southern, E., "Detection of Specific Sequences Among DNA Fragments by Gel Electrophoresis," J. Mol. Biol. 98:503-317 (1975). Plants from a segregating F₂ *ms26**-SBMu200 family were grown and classified for male fertility/sterility. Leaf samples and subsequent DNA isolation was conducted on approximately 20 plants per phenotypic classification. DNA (~7ug) from 5 fertile and 12 sterile plants was digested with EcoRI and electrophoresed through a 0.75% agarose gel. The digested DNA was transferred to nylon membrane via Southern transfer. The membrane was hybridized with an internal fragment from the *Mu8* transposon. Autoradiography of the membrane revealed cosegregation of an approximately 5.6 Kb EcoRI fragment with the sterility phenotype as shown in Figure 1. This EcoRI band segregated in the fertile plants suggesting a heterozygous wild type condition for the allele

### REFERENCE EXAMPLE 2

### Library Construction, Screening, and Mapping

The process of genomic library screenings is commonly known among those skilled in the art and is described at Sambrook, J., Fritsch, E.F., Maniatis T., et al., Molecular Cloning: A Laboratory Manual, Cold Spring Harbor Laboratory, Cold Spring Harbor Lab Press, Plainview, NY (1989). Libraries were created as follows.

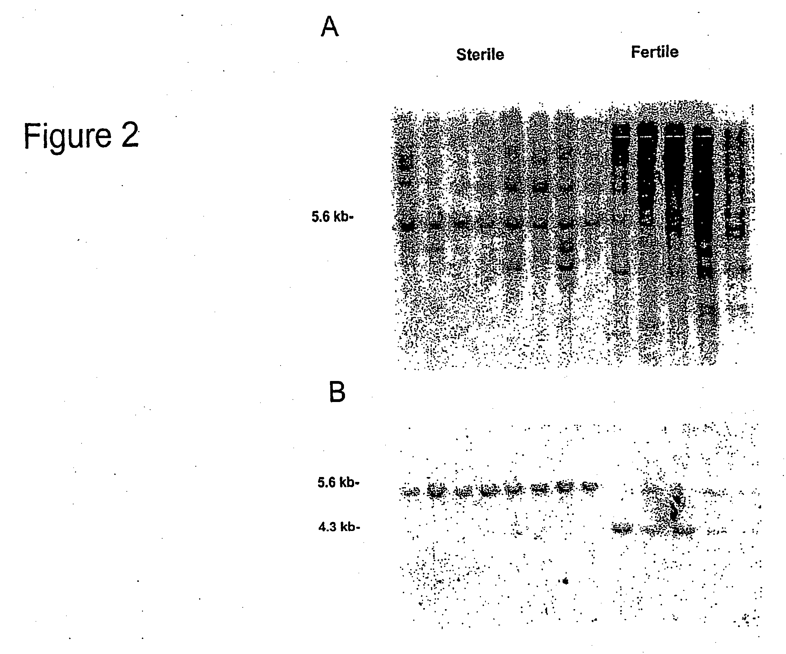

DNA from a sterile plant was digested with *Eco*RI and run on a preparative gel. DNA with a molecular weight between 5.0 and 6.0 Kb was excised from the gel, electroeluted and ethanol precipitated. This DNA was ligated into the Lambda Zap vector (Stratagene™) using the manufacturer's protocol. The ligated DNA was packaged into phage particles using Gigapack Gold (Stratagene™). Approximately 500,000 PFU were plated and lifted onto nitrocellulose membranes. Membranes were hybridized with the *Mu8* probe. A pure clone was obtained after 3 rounds of screening. The insert was excised from the phage as a plasmid and designated SBMu200-3.1. A *Pst*I border fragment from this clone was isolated and used to reprobe the orginal *Eco*RI cosegregation blot as shown in Figure 2B. The approximately 5.6 kb *EcoR*I fragment is homozygous in all the sterile plants, which confirms that the correct *Mu* fragment was isolated. Three of the fertile plants are heterozygous for the 5.5 kb *Eco*RI band and a 4.3 Kb *Eco*RI band. Two of the fertile plants are homozygous for the 4.3 kb *Eco*RI band, presumably the wild type allele.

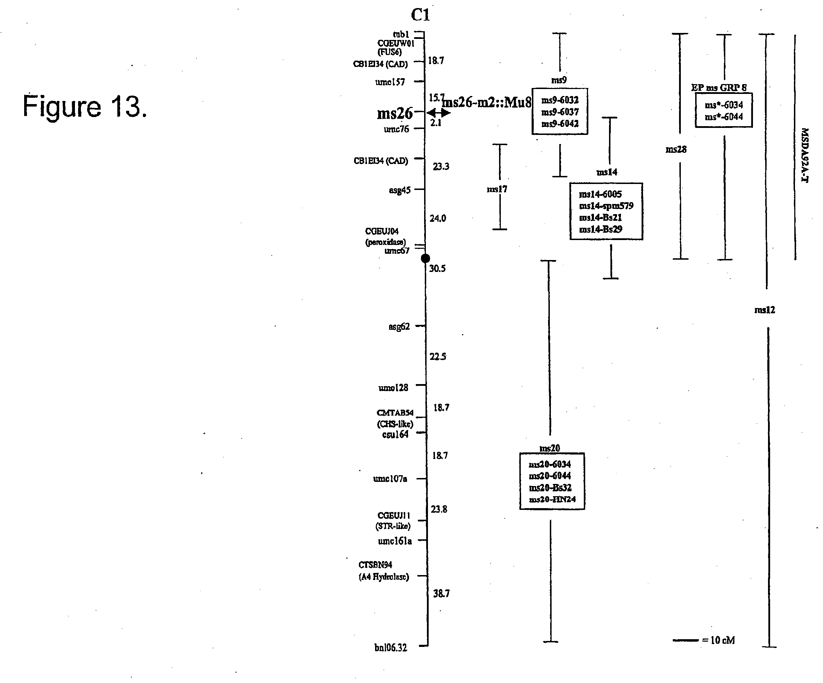

The *Pst*I probe was used to map the *ms**-SBMu200 mutation in an RFLP mapping population. The mutant mapped to the short arm of chromosome 1, near the male sterile locus, *Ms26* (Loukides et al., (1995) Amer. J. Bot 82, 1017-1023). To test whether *ms**-SBMu200 was an allele of *ms26-ref, ms**-SBMu200 and *ms26-ref* were crossed with each other using a known heterozygote as the pollen donor. The testcross progeny segregated male-sterile and wild-type plants in a 1:1 ratio, indicating allelism between *ms**-SBMu200 and *ms26-ref.* The *ms**-SBMu200 allele was designated *ms26-m2::Mu8.* The map location is shown in Figure 13.

### REFERENCE EXAMPLE 3

### Identification and Cloning of Additional ms26 alleles

An additional *Mu* insertion mutations in *Ms26* was identified by using a polymerase chain reaction (PCR) primer for *Mu* and a gene specific primer for *Ms26* and screening a population of *Mu* F₁ families. Sequence analyses of the PCR products showed that all three *Mu* insertions occurred in the second exon (Figure 1). The F₂ seeds from one of these families were grown and examined for male fertility/sterility. Southern blot analyses of this family confirmed the cosegregation of the *Mu* insertion in *Ms26* with the male-sterile phenotype and the allele was designated *ms26-m3::Mu.*

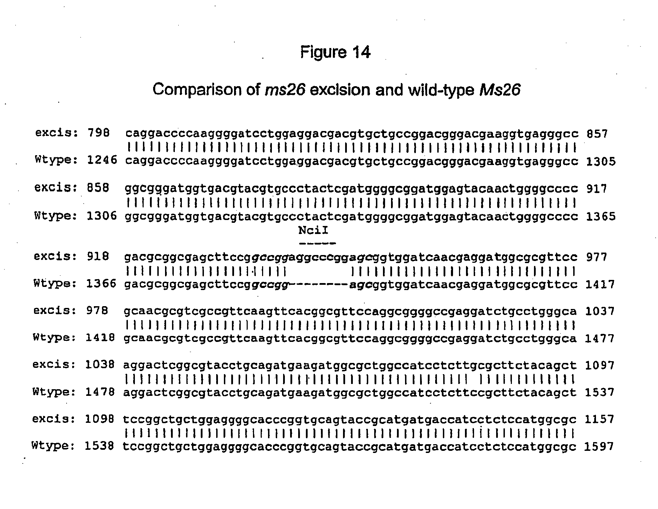

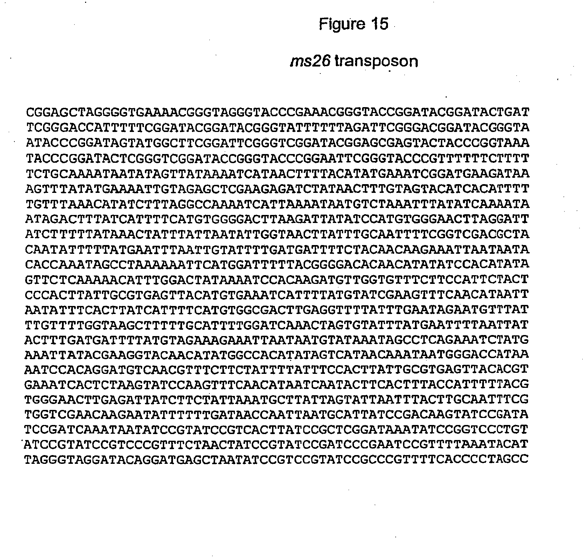

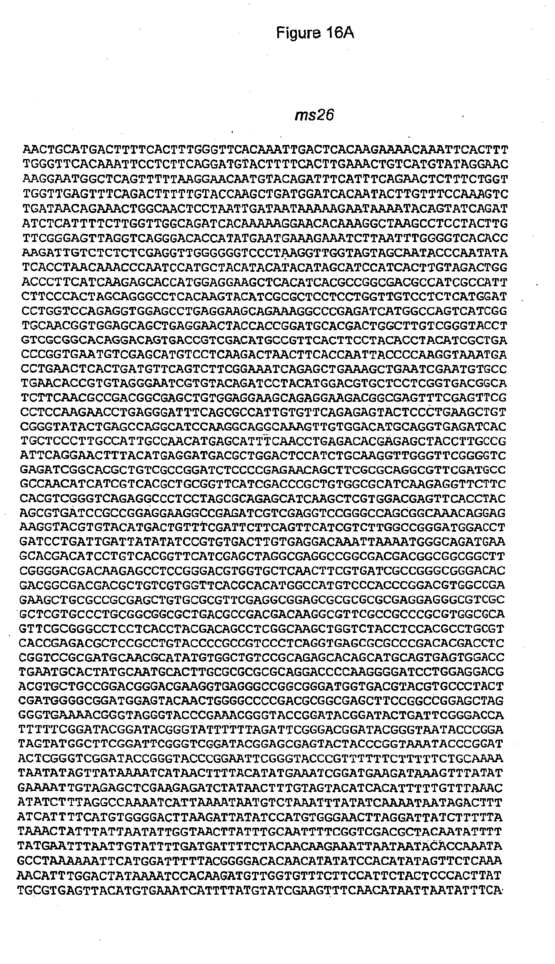

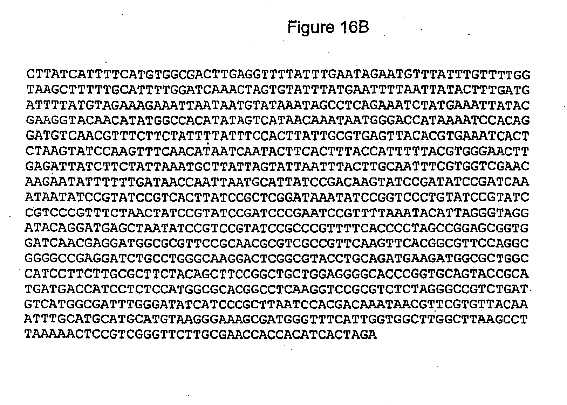

The *ms26* allele described in Loukides et al., (1995) Amer. J. Bot 82, 1017-1023 and designated *ms26-ref* was also investigated. To analyze the mutation in *ms26-ref, Ms26* genomic sequences were cloned from *ms26-ref* sterile and fertile plants. *Ms26* was cloned as a -4.2 kb *Eco*RI fragment and *ms26-ref* cloned as a -6 kb *Hind*II fragment and an overlapping -2.3 kb *Eco*RI fragment from the sterile plant. Sequence analysis revealed the presence of a new segment (1,430 bp) in the last exon of the *ms26-ref* allele shown in Figure 1. An 8 bp host site duplication (GCCGGAGC) was found that flanks the inserted element and the element also contains a 15 bp terminal inverted repeat (TIR) (TAGGGGTGAAAACGG; SEQ ID NO: 23). The transposon sequence is shown in Figure 15 (SEQ ID NO: 10). The *ms26-ref* genomic sequence in its entirety is shown in Figure 16, SEQ ID NO: 11. A variant of the *ms26-ref* allele was also found. Sequence analysis of this allele, designated *my26'-0406,* was found to have lost the 1430 bp segment found in the last exon of the *ms26-ref* allele but left an 8bp footprint at the site of insection. Plants homozygous for the *ms26'-0406* allele were male sterile. A comparison of the excision allele, *ms26'-0406* (SEQ ID NO: 8) with the region in the wild-type *Ms26* gene (SEQ ID NO: 9) is shown in Figure 14.

### REFERENCE EXAMPLE 4

### Expression Analysis and cDNA Isolation

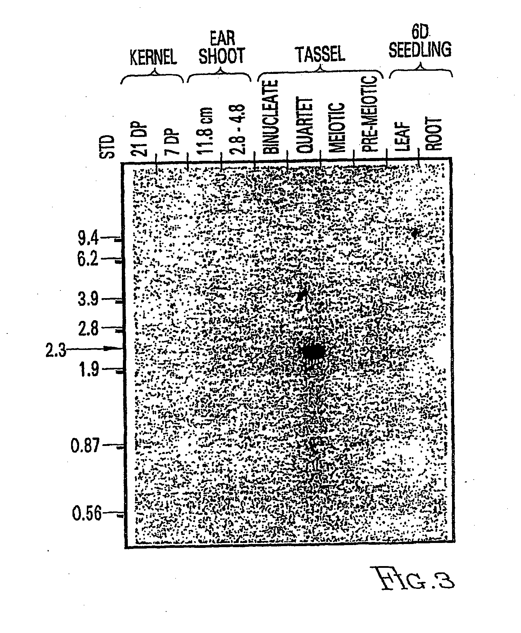

Northern analysis can be used to detect expression of genes characteristic of anther development at various states of microsporogenesis. Northern analysis is also a commonly used technique known to those skilled in the art and is similar to Southern analysis except that mRNA rather than DNA is isolated and placed on the gel. The RNA is then hybridzed with the labeled probe. Potter, E., et al., "Thyrotrotropin Releasing Hormone Exerts Rapid Nuclear Effects to Increase Production of the Primary Prolactin in RNA Transcript," Proc. Nat. Acad. Sci. USA 78:6662-6666 (1981), Lechelt, et al., "Isolation & Molecular Analysis of the Plows," Mol. Gen. Genet. 219:225-234 (1989). The *Pst*I fragment from the SBMu200-3.1 clone was used to probe a Northern blot containing kernel, immature ear, seedling and tassel RNA. A signal was seen only in tassel RNA at approximately the quartet stage of microsporogenesis, as reflected in Figure 3. The transcript is about 2.3 kb in length. The same probe was also used to screen a cDNA library constructed from mRNA isolated from meiotic to late uninucleate staged anthers. One clone, designated *Ms26*-8.1, was isolated from the library.

### REFERENCE EXAMPLE 5

### Sequence and Expression Analysis

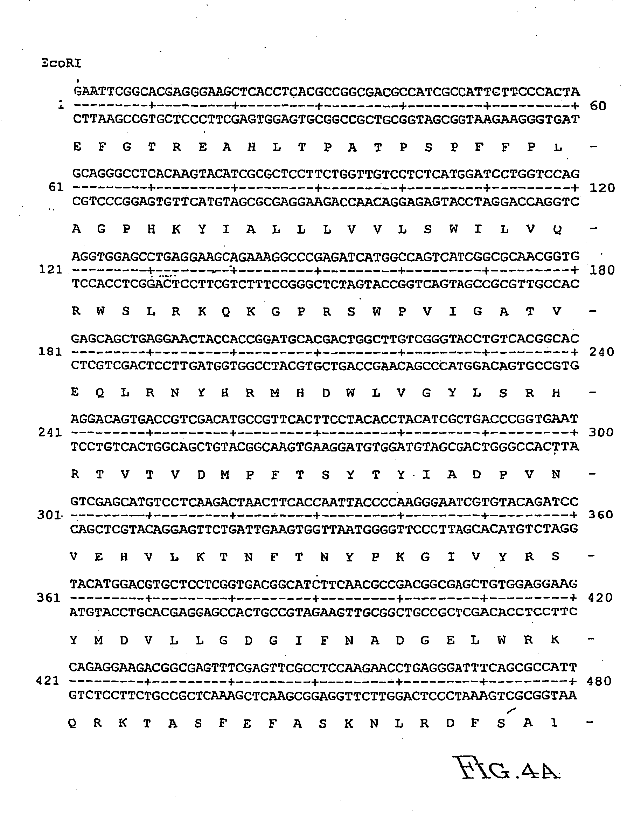

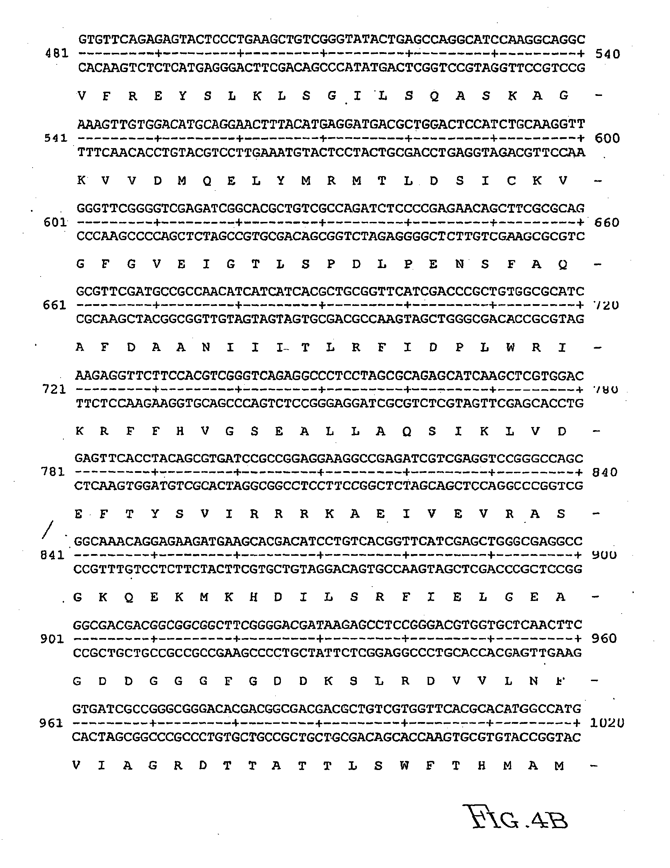

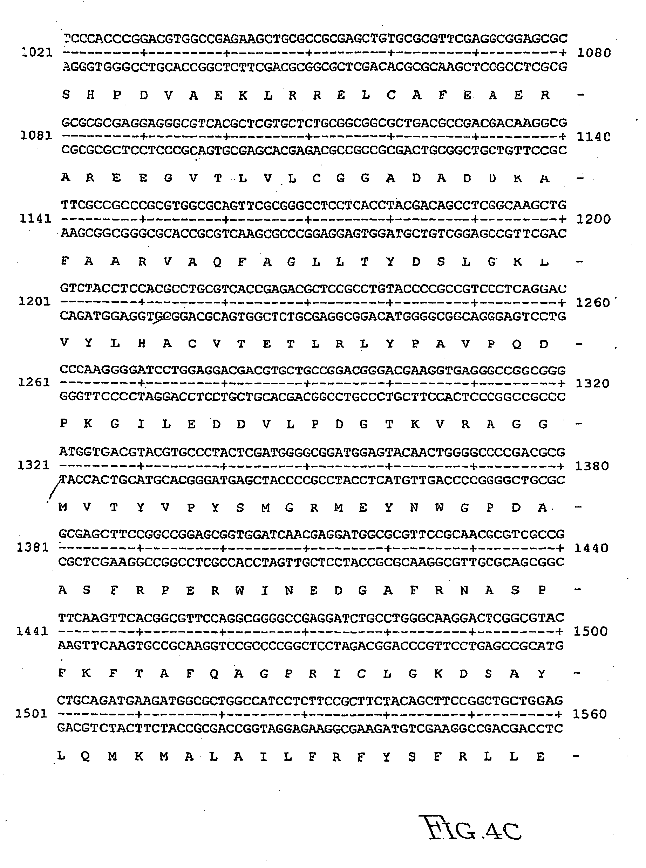

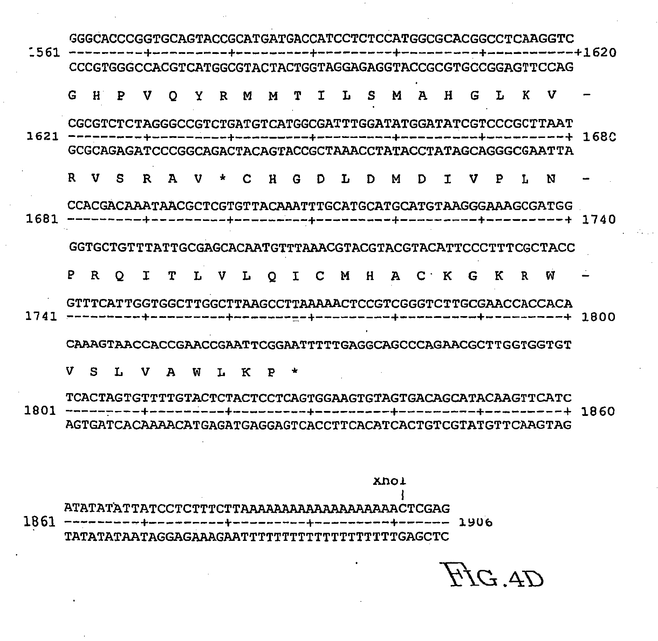

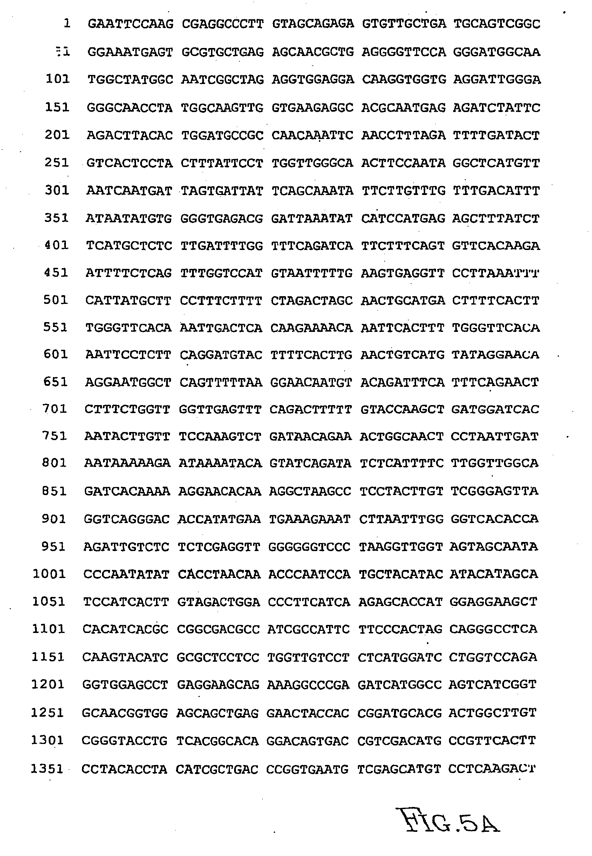

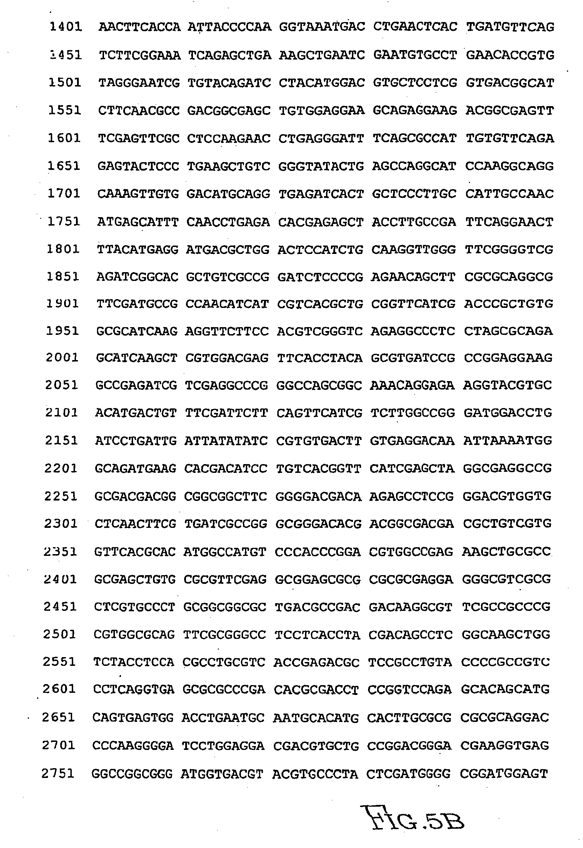

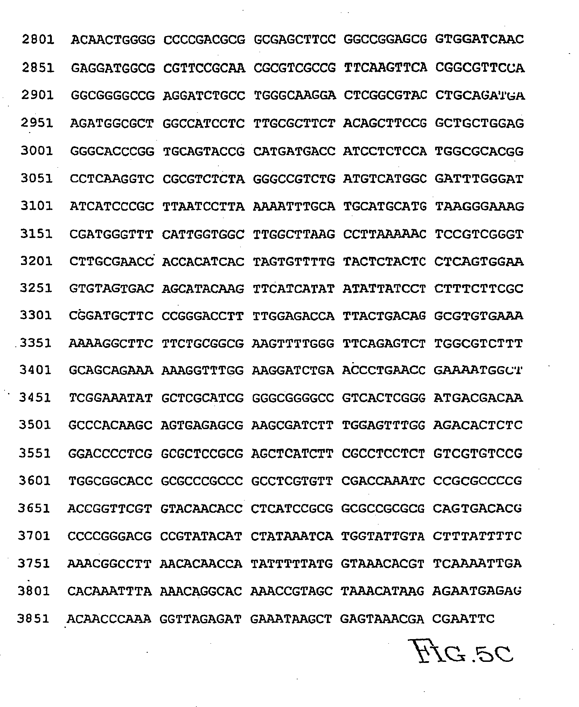

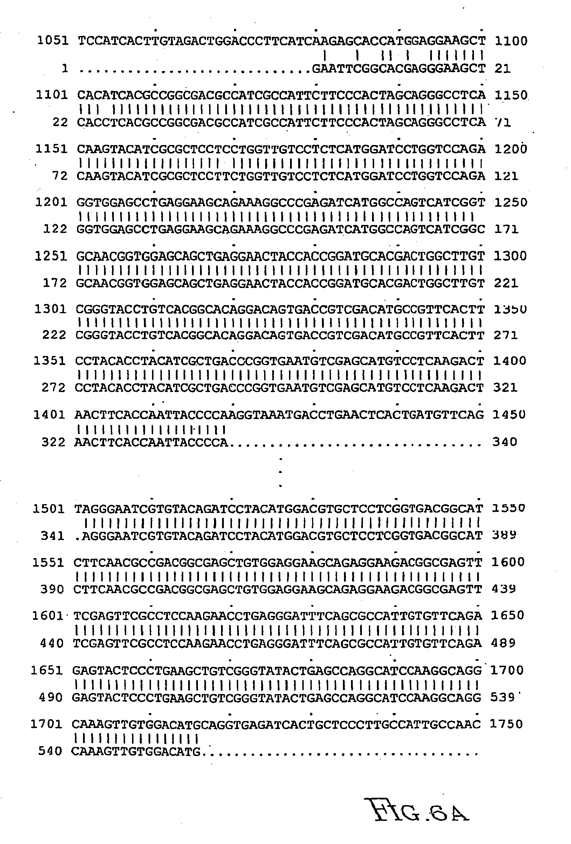

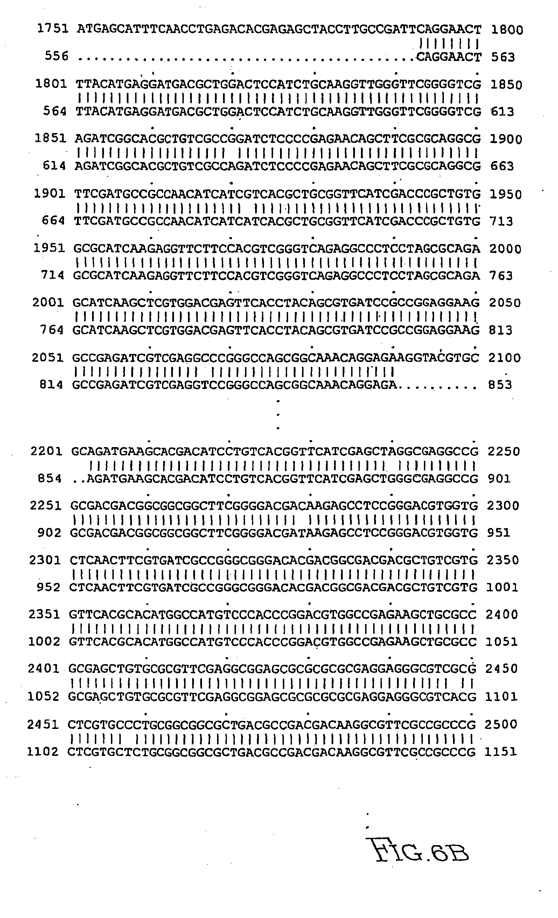

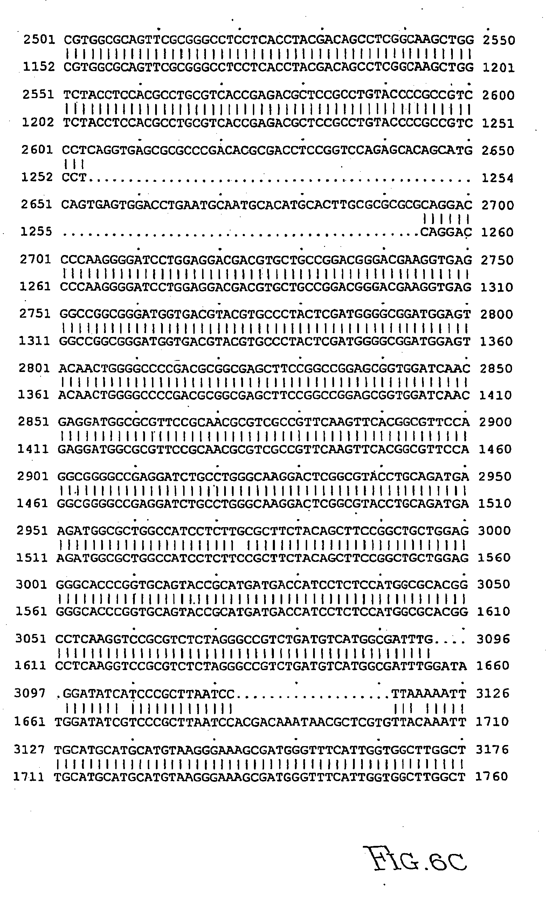

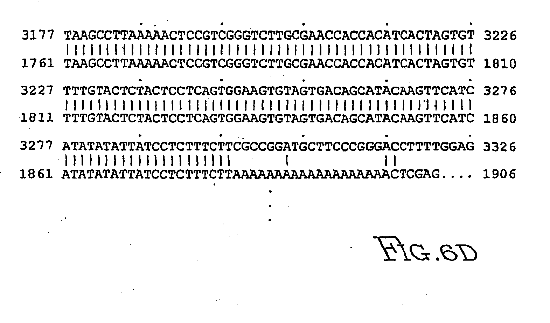

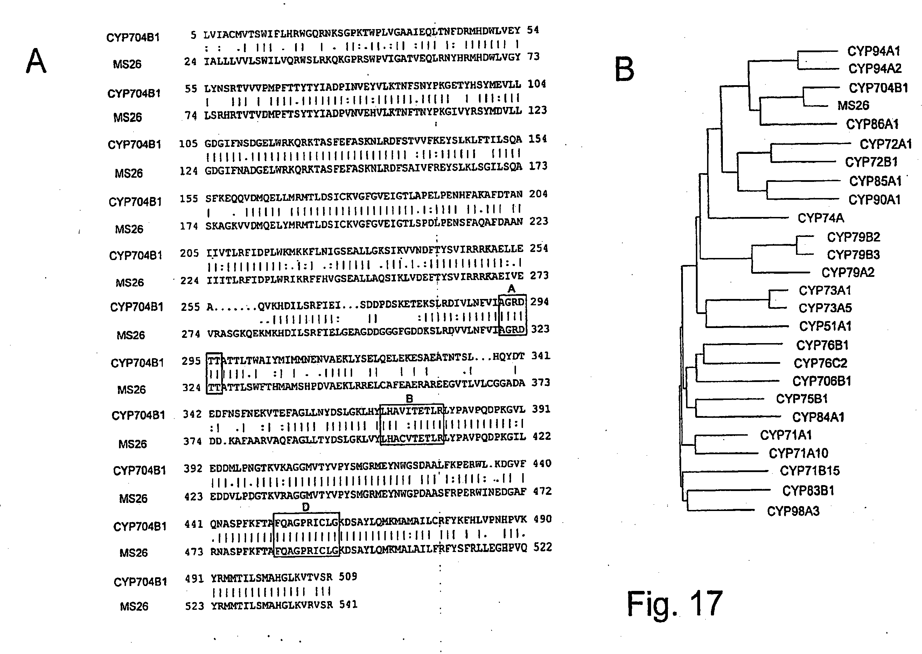

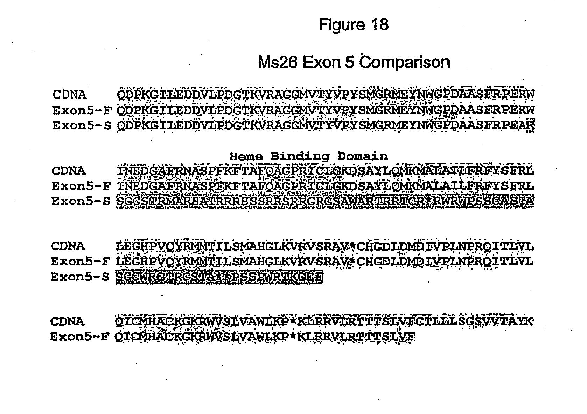

The SBMu200-3.1 genomic clone and the *Ms26-8.1* cDNA clone were sequenced by Loftstrand Labs Limited. Sanger, F., Nicklen, S., Coulson A.R. (1977) "DNA sequencing with chain terminating inhibitors" Proc. Natl. Acad. Sci. USA 74:5463-5467. The sequences are set forth in Figure 4 and 5 and the comparison is at Figure 6. The cDNA/genomic comparison reveals five introns are present in the genomic clone. The *Mu8* insertion occurs in exon 1. Testing for codon preference and non-randomness in the third position of each codon was consistent with the major ORF in the cDNA being the likely protein-coding ORF. There is a putative Met start codon at position 1089 in the genomic clone. The cDNA homology with respect to the genomic clone begins at nucleotide 1094. Thus *Ms26-*8.1 does not represent a full length clone and lacks 5 bases up to the putative Met start codon. A database search revealed significant homology to P450 enzymes found in yeast, plants and mammals. P450 enzymes have been widely studied and three characteristic protein domains have been elucidated. The *Ms26* protein contains several structural motifs characteristic of eukaryotic P450's, including the heme-binding domain FxxGxRxCxG (domain D; SEQ ID NO: 24), domain A A/GGXD/ETT/S (dioxygen-binding), domain B (steroid-binding), and domain C. The highly conserved heme-binding motif was found in *MS26* as FQAGPRICLG (SEQ ID NO: 25), 51 amino acids away from C-terminus. The dioxygen binding domain AGRDTT (SEQ ID NO: 26) was located between amino acids 320-325. The steroid-binding domain was found as LVYLHACVTETLR (SEQ ID NO: 27), amino acids 397-409. The most significant homologous sequence detected in Genebank database is a deduced protein sequence from rice (GeneBank accession number 19071651). The second highest homologous sequence is a putative *Arabidopsis* P450 gene (CYP704B1) whose function is also unknown. Figure 17A shows a sequence alignment between CYP704B1 (SEQ ID NO: 12) and *Ms26* (SEQ ID NO: 13). Phylogenetic tree analysis of some P450 genes revealed that *Ms26* is most closely related to P450s involved in fatty acid omega-hydroxylation found in *Arabidopsis thaliana* and *Vicia sativa* (Figure 17B). The translational frame shift caused in the *ms26'-0406* excision mutation is believed to destroy the activity of the heme binding domain, thus resulting in sterility. See the comparison at Figure 18 (*Ms26* cDNA at SEQ ID NO: 14; fertile exon 5 region at SEQ ID NO: 15 and sterile exon 5 region is SEQ ID NO: 16).

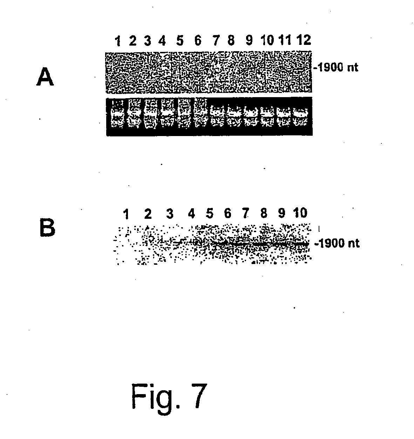

Further expression studies were done using the *Ms26* cDNA probe against a northern containing mRNA at discrete stages of microsporogenesis. Figure 7A shows a Northern blot with RNA samples from different tissues including root (1), leaf (2), husk (3), cob (4), ear spikelet (5), silk (6), immature embryo (7) mature embryo (8), and tassel from, fertile plant (9), *ms26-m2::Mu8* sterile plant (10), *ms26-ref* sterile plant (11) and fertile plant (12). A hybridization signal using *Ms26* cDNA was detected only in tassel tissues. Figure 7B shows a Northern blot containing mRNA at discrete stages of microsporogenesis. Hybridization signals using *Ms26* cDNA were detected from meiosis II/ quartet stage (4) to late-uninucleate stage (10), with the maximal signal being observed from early-uninucleate through late-uninucleate stage (10).

### REFERENCE EXAMPLE 6

### Identification of Promoter and its Essential Regions

A putative TATA box can be identified by primer extension analysis as described in by Current Protocols in Molecular Biology, Ausubel, F.M. et al. eds; John Wiley and Sons, New York pp.4.8.1 - 4.8.5 (1987).

Regulatory regions of anther genes, such as promoters, may be identified in genomic subclones using functional analysis, usually verified by the observation of reporter gene expression in anther tissue and a lower level or absence of reporter gene expression in non-anther tissue. The possibility of the regulatory regions residing "upstream" or 5' ward of the translational start site can be tested by subcloning a DNA fragment that contains the upstream region into expression vectors for transient expression experiments. It is expected that smaller subgenomic fragments may contain the regions essential for male-tissue preferred expression. For example, the essential regions of the CaMV 19S and 35S promoters have been identified in relatively small fragments derived from larger genomic pieces as described in U.S. Pat. No. 5,352,605.

The selection of an appropriate expression vector with which to test for functional expression will depend upon the host and the method of introducing the expression vector into the host and such methods are well known to one skilled in the art. For eukaryotes, the regions in the vector include regions that control initiation of transcription and control processing. These regions are operably linked to a reporter gene such as UidA, encoding -glucuronidase (GUS), or luciferase. General descriptions and examples of plant expression vectors and reporter genes can be found in Gruber, et al., "Vectors for Plant Transformation" in Methods in Plant Molecular Biology and Biotechnology; Glick, et al. eds; CRC Press; pp. 89-119; (1993). GUS expression vectors and GUS gene cassettes are commercially available from Clonetech, Palo Alto, CA, while luciferase expression vectors and luciferase gene cassettes are available from Promega Corporation, Madison, WI. Ti plasmids and other *Agrobacterium* vectors are described in Ishida, Y., et al., Nature Biotechnology; Vol. 14; pp. 745-750; (1996) and in U.S. Pat. No. 5,591,616 "Method for Transforming Monocotyledons" (1994).

Expression vectors containing putative regulatory regions located in genomic fragments can be introduced into intact tissues such as staged anthers, embryos or into callus. Methods of DNA delivery include microprojectile bombardment, DNA injection, electroporation and *Agrobacterium-*mediated gene transfer (*see* Gruber, et al., "Vectors for Plant Transformation," in Methods in Plant Molecular Biology and Biotechnology, Glick, et al. eds.; CRC Press; (1993); U.S Pat. No. 5,591,616; and Ishida, Y., et al., Nature Biotechnology; Vol. 14; pp. 745-750; (1996)). General methods of culturing plant tissues are found in Gruber, et al., *supra* and Glick, *supra.*

For the transient assay system, staged, isolated anthers are immediately placed onto tassel culture medium (Pareddy, D.R. and J.F. Petelino, Crop Sci. J.; Vol. 29; pp. 1564-1566; (1989)) solidified with 0.5% Phytagel (Sigma, St. Louis) or other solidifying media. The expression vector DNA is introduced within 5 hours preferably by microprojectile-mediated delivery with 1.2 µm particles at 1000 -1100 Psi. After DNA delivery, the anthers are incubated at 26°C upon the same tassel culture medium for 17 hours and analyzed by preparing a whole tissue homogenate and assaying for GUS or for lucifierase activity (see Gruber, et al., *supra*)*.*

Upstream of the likely translational start codon of *Ms26,* 1088 bp of DNA was present in the genomic clone *ms26-m2::Mu8.* Translational fusions via an engineered *NcoI* site were generated with reporter genes encoding luciferase and β-glucuronidase to test whether this fragment of DNA had promoter activity in transient expression assays of bombarded plant tissues. Activity was demonstrated in anthers and not in coleoptiles, roots and calli, suggesting anther-preferred or anther-specific promoter activity.

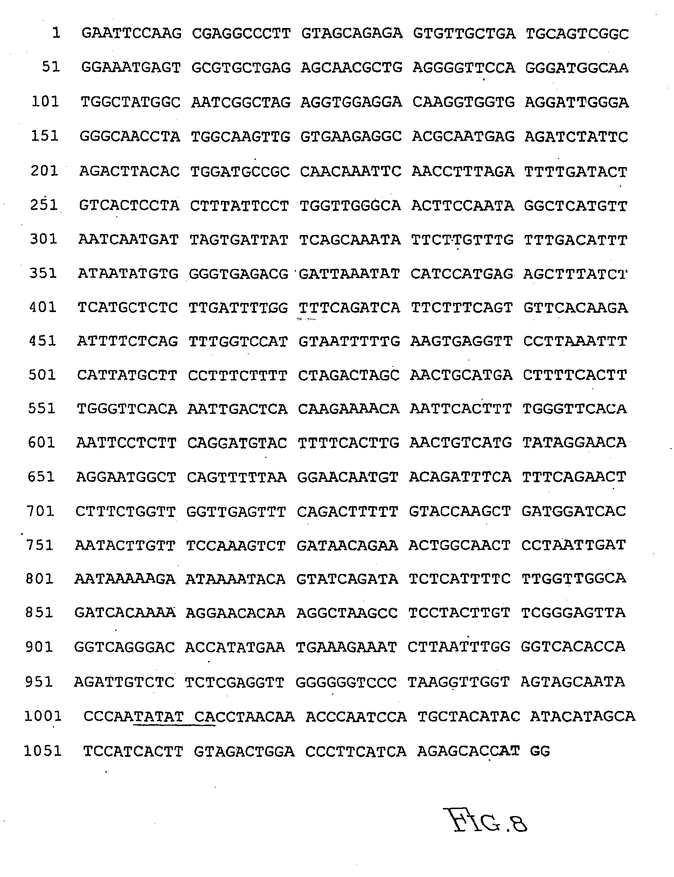

A reasonable TATA box was observed by inspection, about 83-77 bp upstream of the translational start codon. The genomic clone *ms26-m2::Mu8* thus includes about 1005 bp upstream of the possible TATA box. For typical plant genes, the start of transcription is 26-36 bp downstream of the TATA box, which would give the *Ms26* mRNA a 5'-nontranslated leader of about 48-58 nt. The total *ms26-m2::Mu8* subgenomic fragment of 1088 bp, including nontranslated leader, start of transcription, TATA box and sequences upstream of the TATA box, was thus shown to be sufficient for promoter activity. See Figure 8, which is SEQ. ID NO.5. The putative TATA box (TATATCA) is underlined. Thus, the present disclosure encompasses a DNA molecule having a nucleotide sequence of SEQ ID NO: 5 (or those with sequence identity) and having the function of a male tissue-preferred regulatory region.

Deletion analysis can occur from both the 5' and 3' ends of the regulatory region: fragments can be obtained by site-directed mutagenesis, mutagenesis using the polymerase chain reaction, and the like (Directed Mutagenesis: A Practical Approach; IRL Press; (1991)). The 3' end of the male tissue-preferred regulatory region can be delineated by proximity to the putative TATA box or by 3' deletions if necessary. The essential region may then be operably linked to a core promoter of choice. Once the essential region is identified, transcription of an exogenous gene may be controlled by the male tissue-preferred region of *Ms26* plus a core promoter. The core promoter can be any one of known core promoters such as a Cauliflower Mosaic Virus 35S or 19S promoter (U.S. Pat. No. 5,352,605), Ubiquitin (U.S. Pat. No. 5,510,474), the IN2 core promoter (U.S. Pat. No. 5,364,780), or a Figwort Mosaic Virus promoter (Gruber, et al., "Vectors for Plant Transformation" in Methods in Plant Molecular Biology and Biotechnology; Glick, et al. eds.; CRC Press; pp. 89-119; (1993)). Preferably, the promoter is the core promoter of a male tissue-preferred gene or the CaMV 35S core promoter. More preferably, the promoter is a promoter of a male tissue-preferred gene and in particular, the *Ms26* core promoter.

Further mutational analysis, for example by linker scanning, a method well known to the art, can identify small segments containing sequences required for anther-preferred expression. These mutations may introduce modifications of functionality such as in the levels of expression, in the timing of expression, or in the tissue of expression. Mutations may also be silent and have no observable effect.

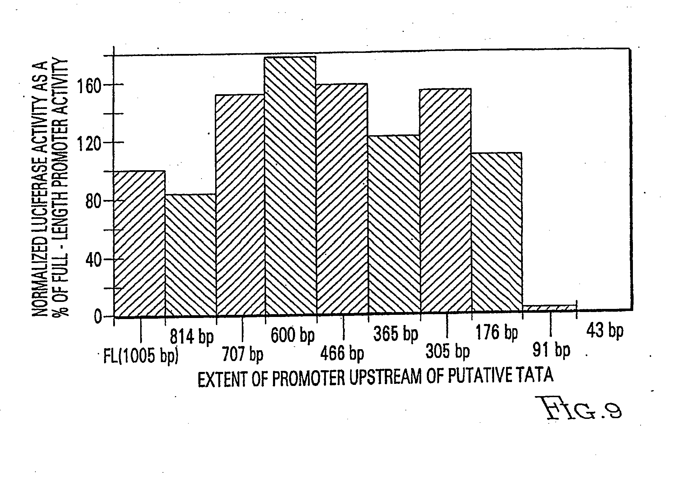

The foregoing procedures were used to identify essential regions of the Ms26 promoter. After linking the promoter with the luciferase marker gene deletion analysis was performed on the regions of the promoter upstream of the putative TATA box, as represented in Figure 9. The x-axis of the bar graph indicates the number of base pairs immediately upstream of the putative TATA box retained in a series of deletion derivatives starting from the 5' end of the promoter. The y-axis shows the normalized luciferase activity as a percent of full-length promoter activity.

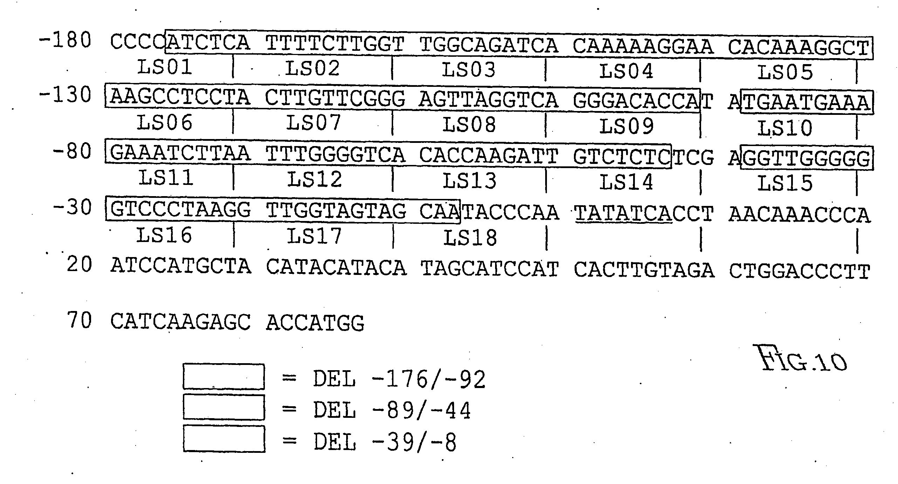

As is evident from the graph, approximately 176 bp immediately upstream of the TATA box was sufficient, when coupled to the core promoter (putative TATA box through start of transcription), plus 5' nontranslated leader, for transient expression in anthers. By contrast, luciferase activity was minimal upon further deletion from the 5' end to 91 bp upstream of the putative TATA box. This 176 bp upstream of the putative TATA box through the nontranslated leader can be considered a minimal promoter, which is further represented at Figure 10. The TATA box is underlined. Deletion within the full-length promoter from -176 through -92 relative to the TATA box reduced activity to about 1% of wild type. Deletion of -39 through -8 did not greatly reduce activity. Therefore the -176 to - 44bp region contains an essential region and thus would constitute an upstream enhancer element conferring anther expression on the promoter, which we refer to as an "anther box".

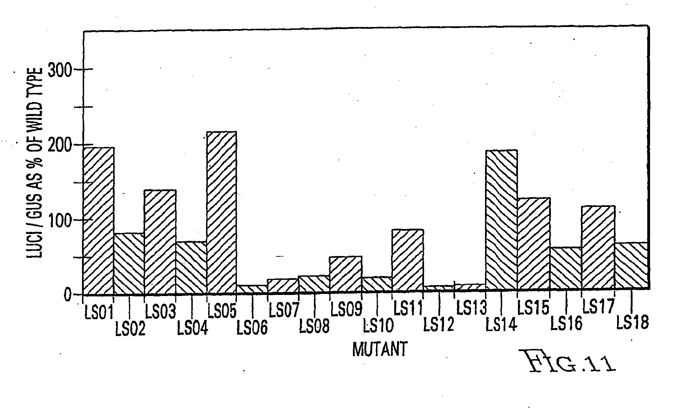

Linker scanning analysis was conducted across the anther box in 9-10 bp increments. The locations of the linker scanning substitutions in this region are shown in Figure 10, and the expression levels of the mutants relative to the wild type sequence are shown in Figure 11. The most drastic effect on transient expression in anthers was observed for mutants LS12 and LS13, in the region 52-71 bp upstream of the putative TATA box. A major effect on transient expression in anthers was also observed for mutants LS06, LS07, LS08 and LS10, within the region 82-131 bp upstream of the putative TATA box. Sequences within the anther box required for wild type levels of transient expression in anthers are thus demonstrated in the -52 to -131 region relative to the putative TATA box, particularly the -52 to -71 region. The essential regions are shown at SEQ ID NO: 6 (Figure 10) and, as compared to the genomic sequence, SEQ ID NO: 7 (Figure 5) are bases 1-1088; 830-962; 830-914; 917-962; 875-954; 935-954; and 875-924.

### REFERENCE EXAMPLE 7

### Ms26 Sorghum, Rice and Maize Comparison

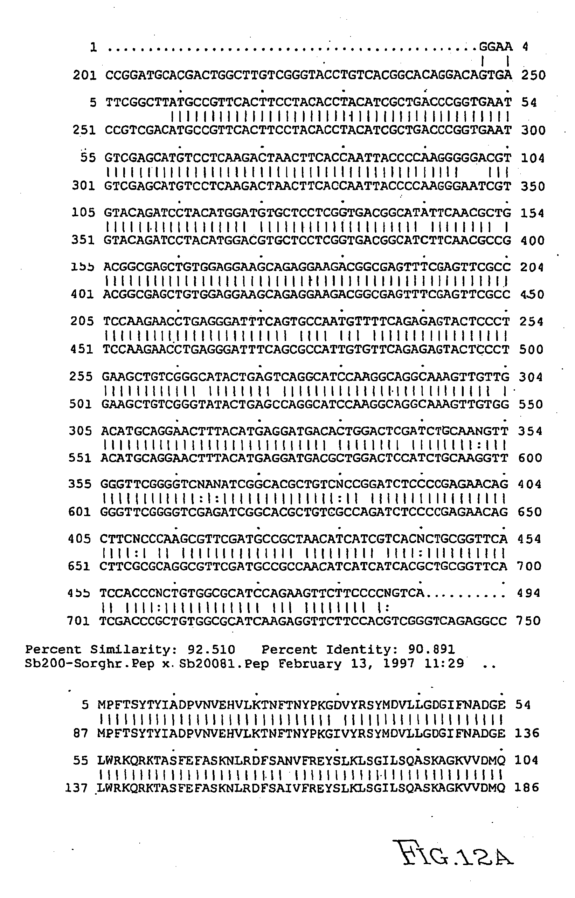

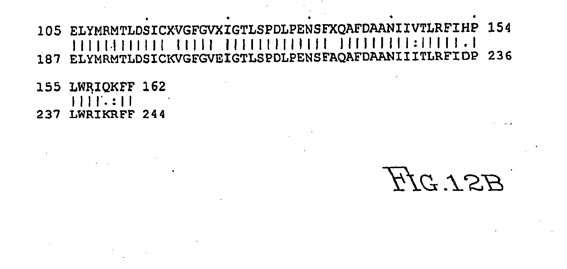

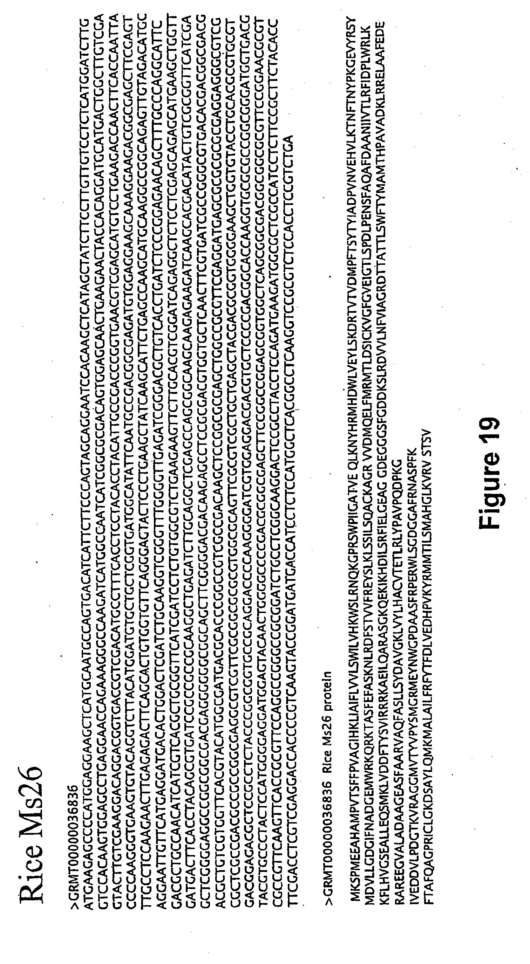

As noted above, *Ms26* is a male fertility gene in maize. When it is mutated, and made homozygous recessive, male sterility will result. An orthologue of *Ms26* was identified in sorghum. The sorghum orthologue of the *Ms26* _{C}DNA was isolated by using the maize *Ms26* gene primers in a polymerase chain reaction with sorghum tassel cDNA as the template. The resultant cDNA fragment was sequenced by methods described *supra* and then compared to the *Ms26* cDNA from maize. Nucleotide sequence comparisons are set forth in Figure 12 and show 90% identity. An orthologue from rice was also identified and the predicted coding sequence (SEQ ID NO: 17) and protein (SEQ ID NO: 18) is set forth in Figure 19. It has one intron less than the maize and sorghum *Ms26,* and the coding sequences are highly conserved.

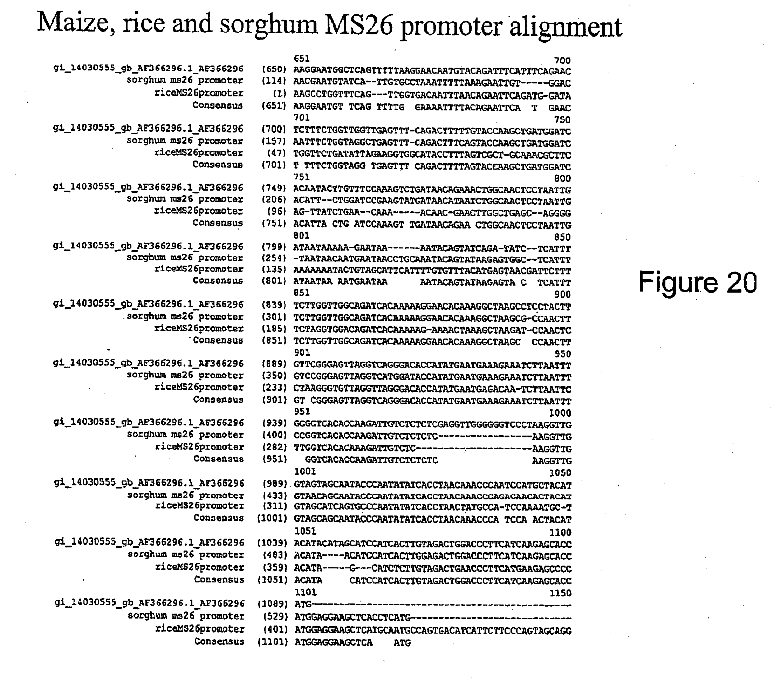

Identification of the sorghum and rice promoters was accomplished. Figure 20 shows an alignment of the *Ms26* promoter of corn (SEQ ID NO: 5), sorghum (SEQ ID NO: 19) and rice (SEQ ID NO: 20). The last three bases of the corn promoter shown in the figure is the ATG start of translation.

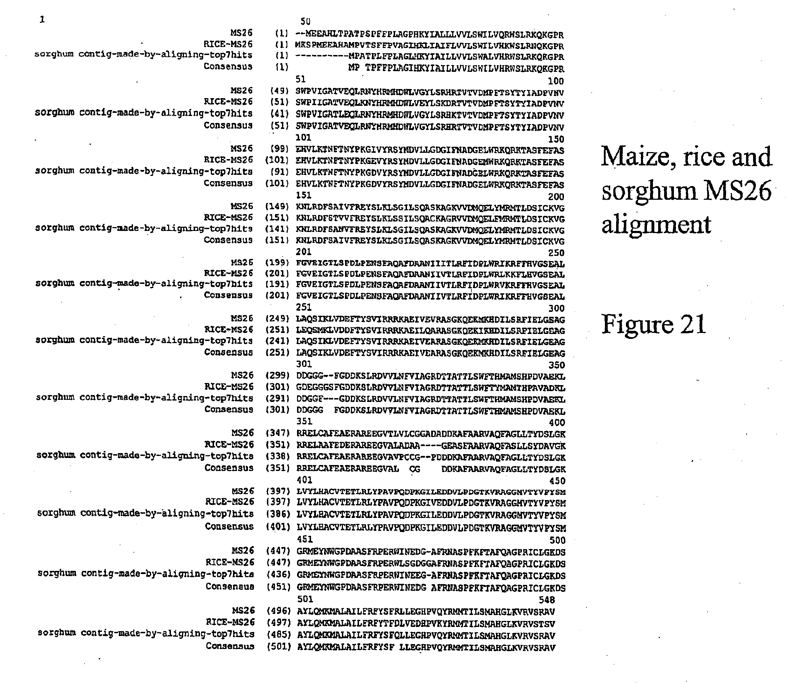

Alignment as reflected in Figure 21 of the maize *Ms26* protein (SEQ ID NO: 2), rice *Ms26* protein(SEQ ID NO: 18) and sorghum *Ms26* protein (SEQ ID NO: 4), and a consensus sequence (SEQ ID NO: 21). The comparison of protein sequences shows the protein is highly conserved among the orthologues, with the rice protein sharing 92% similarity and 86% identity when compared to the maize orthologue. The predicted tissue specificity in rice and sorghum is further reflected in a comparison of the *Ms26* protein in the sorghum and rice EST database derived from panicle (flower) libraries. Sorghum sequences producing significant alignments (GenBank accession numbers BI075441.1; BI075273.1; BI246000.1; BI246162.1; BG948686.1; BI099541.1 and BG948366.1, among others) all were sequences from immature panicle of sorghum, and sequences showing significant alignment in rice (GenBank accession numbers C73892.1; CR290740.1, among others) were also from rice immature panicle.

As is evident from the above, nucleotide sequences which map to the short arm of chromosome 1 of the *Zea mays* genome, at the same site as the *Ms26* gene, *ms26-m2::Mu8* and its alleles, are genes critical to male fertility in plants, that is, are necessary for fertility of a plant, or, when mutated from the sequence found in a fertile plant, cause sterility in the plant.

### REFERENCE EXAMPLE 8

### Construction of a plant transformation vector comprising a selectable marker, a male fertility gene Ms45 and a pollen cytotoxin gene.

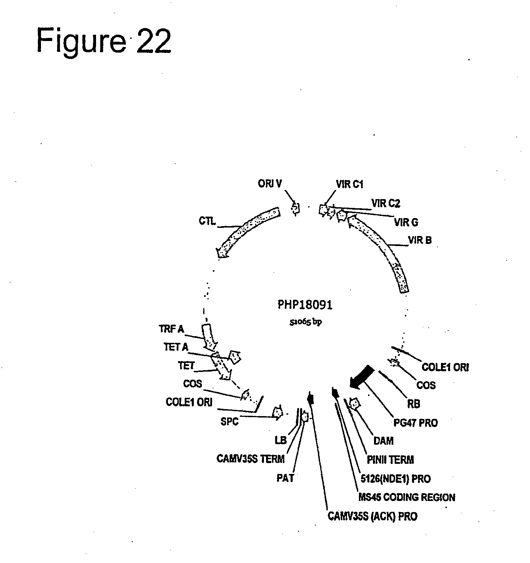

A construct designated PHP18091, shown in Figure 22 is made by assembling following DNA components:
1. The plasmid pSB11 backbone DNA (pSB31 lacking the EcoRI fragment carrying the 35SGUS and 35SBAR genes, Ishida et al., Nature Biotechnol. (1996) 14:745-750). This DNA backbone contains T-DNA border sequences and the replication origin from pBR322.
2. The 35S:PAT gene which encodes the enzyme phosphinothricin acetyltransferase (PAT) from *Streptomyces viridochomagenes* ( nucleotides 6-557 from accession number A02774, Strauch et al. 1988, EP 0275957-A; SEQ ID NO: 22) under the transcriptional control of the cauliflower mosaic virus (CaMV) 35S promoter and terminator (nucleotides 6906-7439, and 7439-7632, respectively from Franck et al. 1980, Cell 21: 285-294; SEQ ID NO: 23 and SEQ ID NO: 24).
3. The 5126:*Ms45* gene which contains the maize male fertility gene coding region (nucleotides 1392-3343, accession number AF360356, Albertsen et a Am. J. Bot.. (1993) 80:16; SEQ ID NO: 25) under the control of the maize anther-specific promoter 5126 (nucleotides 985-1490, accession number I75204; SEQ ID NO: 26).
4. The PG47:DAM gene which contains the *E*. *coli* DNA (Adenosine-N⁶) methyltransferase (DAM) coding region (nucleotides 195-1132, Brooks et al., Nucleic. Acids Res (1983) 11: 837-851; SEQ ID NO: 26) driven by the maize pollen-specific promoter PG47 (nucleotides 1-2870, accession number X66692, Allen and Lonsdale, Plant J. (1993)3:261-271; SEQ ID NO: 27). The transcription of this gene is terminated by the potato proteinase inhibitor II (PinII) terminator (nucleotides 2-310, An et al., Plant Cell (1989) 1: 115-122; SEQ ID NO: 28).
5. A 3.34 kb Ncol DNA fragment containing *Ms45:Ms45* was cloned upstream of the 35S:PAT gene in pUC8, creating PHP6641. A 4.7 kb HindIII/EcoRI DNA fragment containing *Ms45:Ms45*-35S:PAT from PHP6641 was cloned into pSB11, creating PHP10890 (Cigan et al, Sex. Plant Reprod. (2001)14: 135-142). The native *Ms45* promoter in PHP10890 was replaced by a 528 bp HindIII/NcoI fragment containing the maize 5126 promoter, creating PHP11943.
6. A 2.87 kb Hindlll/Ncol fragment containing PG47 promoter was ligated with a 0.8 kb NcoI/HindIII fragment containing the DAM coding region, PinII terminator and 35S enhancer which was from PHP10404 (Unger, et al., Transgenic Res. (2001)10: 409-422), creating a 3.67 kb fragment HindIII fragment containing PG47:DAM gene fusion (with the 35S enhancer). This 3.67 kbp HindIII fragment was then cloned into the HindIII site of PHP11943, creating PHP20005. The 35S enhancer in PHP20005 was removed, creating PHP18071. The PHP18071 was introduced into *Agrobacterium* strain LBA4404 carrying plasmid pSB1 by triparental mating (Ishida et al., Nature Biotechnol. (1996) 14:745-750). The co-integrate of PHP18071 and pSB1 was named PHP18091.

### REFERENCE EXAMPLE 9

### Transformation of corn with the restoring transgene construct of Example 8.

A male-sterile female which was homozygous for an *ms45* mutant *Ac* excision allele, *ms45'*-9301 *(ms45)* was repeatedly crossed with bulked pollen from maize Hi-type II plants (Armstrong 1994, In: Freeling and Walbot (eds). The Maize Handbook. Springer, New York, pp 663-671) resulting in the introgression of this *ms45* allele in transformation amenable maize germplasm over multiple generations. The resultant source of material for transformation consisted of embryos segregating (1:1 or 3:1) for *ms45* and allowed for both transformation directly into a homozygous *ms45* background and to test the genetic complementation of the *ms45* mutation in To plants. *Agrobacterum*-mediated transformation was performed according to Zhao et al. 1999, (United States Patent number 5,981,840). Genotyping and molecular analysis (integration and PTU) of transformants were done according Cigan et al., (Sex. Plant. Reprod. (2001)14:135-142 ). Transformants with single-integration and complete PTU were selected for further studies.

### REFERENCE EXAMPLE 10

### Analysis of maize transformants.

Transgenic plants (To) from Example 9 were evaluated for the whole plant morphology and analyzed for transgene transmission through both pollen and egg cells. No morphological difference was observed between the transgenic plants and the non-transgenic control plants except for the degree of male fertility. Transformants with single-integration and intact PTU were partial male fertile while non-transgenic control plants were completely male sterile, indicating that the expression of *Ms45* gene complemented the homozygous recessive ms45 male sterile phenotype. This also demonstrated that the expression of the DAM gene caused partial male sterility by eliminating the pollen grains carrying the transgenes. Without the DAM gene, *Ms45* transgene can completely recover the *ms45* male sterile mutation (Cigan et al., Sex. Plant. Reprod. (2001) 14: 135-142). The correct function of DAM gene was further determined by controlled pollinations between To transgenic plants and non-transgenic plants. Pollen grains from To transgenic plants were used to pollinate non-transgenic plants control plants. Immature embryos were harvested from ears of these non-transgenic plants 18 days after pollination and cultured either on MS media or MS media containing 3.0 mg/L of biataphos (Murashige, T. and Skoog, F. A revised medium for rapid growth and bioassays with tobacco tissue cultures. Physiol. Plant (1962) 15: 437-439). 100% of the embryos were able to germinate on control medium while none of the embryos were able to germinate on media containing 3 mg/L of bialaphos, indicating that the restoring transgene construct was not transmitted through pollen to progeny.

In addition, pollen from non-transgenic plants was used to pollinate the To transgenic maintainer plants. Immature embryos were harvested from ears of these T₀ transgenic maintainer plants 18 days after pollination and cultured as above control media or media containing 3 mg/L of bialaphos. All embryos were able to germinate on control medium while 50% of the embryos were able to germinate on the medium containing bialaphos, indicating that the restoring transgene construct was transmitted through the ovule to progeny at the expected frequency. The results of embryo rescues are summarized in Tables 1 and 2.

**Table 1: Transgene transmission through pollen**

| Transgenic plants | Pollen to non-transgenic plants | | | | | |
|---|---|---|---|---|---|---|
| | Control medium | | | Medium + 3mg/l bialaphos | | |
| | # embryo cultured | # embryo germinated | % | # embryo cultured | # embryo germinated | % |
| 14089263 | 40 | 40 | 100 | 60 | 0 | 0 |
| 14089277 | 100 | 100 | 100 | 100 | 0 | 0 |
| 14089839 | 40 | 40 | 100 | 60 | 0 | 0 |

**Table 2: Transgene transmission through egg cells**

| Transgenic plants | Pollen from non-transgenic plants | | |
|---|---|---|---|
| | Medium + 3mg/l bialaphos | | |
| | # embryo cultured | # embryo germinated | % |
| 14089262 | 20 | 8 | 40 |
| 14089277 | 40 | 22 | 55 |
| 14089839 | 40 | 21 | 53 |

### REFERENCE EXAMPLE 11

### Conversion of T₀ plants into different inbred lines and analysis of Tn plants.

To transgenic maintainer plants from Example 9 were converted into different inbred backgrounds through repeated backcross by pollination from inbred lines such as PH09B. To accomplish this, pollen produced by PH09B that is *ms45* heterozygous background were used to pollinate the ears of To maintainer plants that were homozygous for the *ms45* mutant alleles. T₁ seed harvested from these T₀ plants segregated for both transgenes and *ms45* alleles. T₁ plants that did not contain the restoring transgene construct were eliminated by herbicide selection. T₁ plants containing transgenes were analyzed for *ms45* background and male fertility according to Cigan et al., (Sex. Plant. Reprod., (2001) 14: 135-142). In general, T₁ plants in homozygous *ms45* condition that contained the restoring transgene construct showed partial male fertility like that observed for the T₀ parent plants, while the T₁ plants in homozygous ms45 condition but containing no transgenes were complete male sterile. This suggested that the *Ms45* transgene continued to function correctly in a different genetic background. Pollen grains from T₁ plants were examined for viability using microscopic and histochemical staining. Pollen grains at different developmental stages were collected and stained with fluorescein diacetate (FDA), 4', 6-diamidino-2-phenylindole (DAPI) and ethidium bromide (EB). About 50% of the pollen grains from the transgenic T₁ plants lost their viability as judged by the absence of fluorescence after staining with FDA after first pollen mitosis, while the pollen grains from non-transgenic control plants showed uniform FDA staining. This was further supported by in vitro pollen germination studies. The germination rate of the pollen grains from the transgenic T₁ plants were about half of that from non-transgenic control plants. Pollen grains from transgenic T₁ plant were also used to pollinate non-transgenic plants to test transgene transmission thought pollen. For instance, none of 248 embryos from a non-transgenic plant pollinated by a T₁ plant (20118954) were able to germinate on the medium containing 3 mg/l bialaphos. These experiments confirmed both the correct function of the *Ms45* and DAM transgenes in different genetic backgrounds. The T₁ plants with desired performance were used for the next backcross iteration using pollen from the paternal inbred parent which was heterozygous for the mutant *ms45* allele. This process will be repeated until sixth generation.

### REFERENCE EXAMPLE 12

### Large scale transmission and maintenance of ms45 male sterility using the construct of Example 8.

T₁ plants derived from T₀ 14089277 as described in example 9 were used as males to pollinate either wild type inbred plants or *ms45*/*ms45* male sterile inbred plants. The 10,117 T₂ progeny from the wild type crosses and 6688 T₂ progeny from the *ms45*/*ms45* crosses were evaluated for transgene transmission by screening for herbicide resistance. For both types of crosses a total of 16786 T₂ plants were found to be herbicide sensitive, yielding a non-transmission frequency of 99.89%. All T₂ plants from the *ms45*/*ms45* crosses that did not contain the transgene, were completely male sterile, indicating that this transgenic line can maintain *ms45* sterility.

### EXAMPLE 13

### Construction of a plant transformation vector comprising a screenable marker, a male fertility gene Ms26 and a pollen cytotoxin gene.

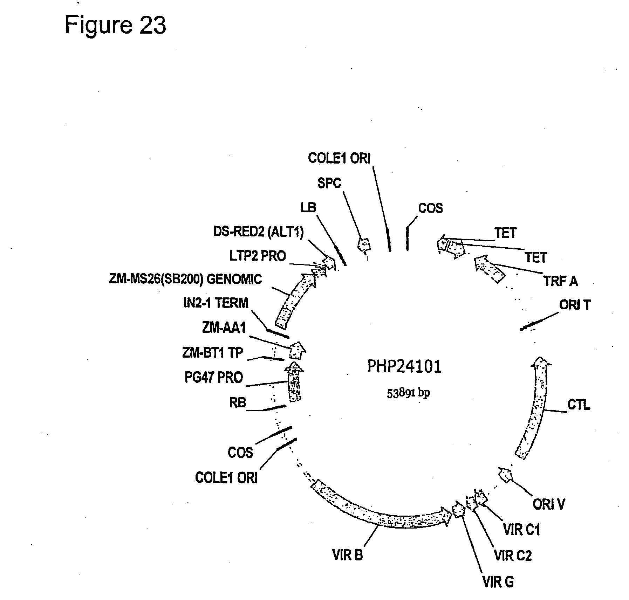

A construct designated PHP24101, shown in Figure 23, is made by assembling following DNA components:
1. The plasmid pSB11 backbone DNA (pSB31 lacking the EcoRI fragment carrying the 35SGUS and 35SBAR genes, Ishida et al., Nature Biotechnol. (1996) 14:745-750). This DNA backbone contains T-DNA border sequences and the replication origin from pBR322.
2. The PG47PRO:ZM-AA1 gene which contains alpha-amylase 1 coding region from *Zea mays* as set forth in Figure 24. (SEQ ID NO: 26). The transcription of this gene is terminated by IN2-1 terminator (U.S. Patent No. 5,364,780).
3. The *Ms26* (SB200) GENOMIC gene (SEQ ID NO: 7) which contains the maize male fertility gene coding region.
4. LTP2:DS-RED2 (ALT1) which contains red florescence coding region (a variant of *Discosoma* sp. red fluorescent protein (DsRed), from Clontech mutated to remove BstEII site, codon sequence unchanged) driven by LTP2 promoter, *supra.*
5. A 2.143 kb EcoRV/DraI DNA fragment containing LTP2PRO:DS-RED2 (ALT1) from PHP21737 was cloned into downstream of the *Ms26* GENOMIC gene in SK vector, creating SK-*Ms26* GENOMIC-LTP2PRO:DS-RED2 (ALT1).
6. A 2.143 kb EcoRV/DraI DNA fragment containing LTP2PRO:DS-RED2 (ALT1) from PHP21737 was cloned into downstream of the *Ms45*PRO:*Ms45* GENOMIC gene in SK vector, creating SK-*Ms45*-LTP2PRO:DS-RED2 (ALT1).
7. A 5.429 kb NotI fragment containing 5126PRO:*Ms45* GENOMIC-UBI:MOPAT:PINII in PHP20532 was replaced by A 4.318 kb Notl fragment containing *Ms45*-LTP2PRO:DS-RED2 (ALT1) from SK-*Ms45*-LTP2PRO:DS-RED2 (ALT1), creating PHP22623.
8. A 4.318 kb NotI fragment containing *Ms45*-LTP2PRO:DS-RED2 (ALT1) in PHP22623 was replaced by A 5.960 kb NotI DNA fragment containing *Ms26* GENOMIC-LTP2PRO:DS-RED2(ALT1) from SK-*Ms26* GENOMIC-LTP2PRO:DS-RED2 (ALT1), creating PHP24014. The PHP24014 was introduced into *Agrobacterium* strain LBA4404 carrying plasmid pSB1 by Electrophoresis. Co-integrate of PHPPHP24014 and pSB1 was named PHP24101.

### EXAMPLE 14

### Transformation of corn with the restoring transgene construct of Example13.

A male-sterile female which was homozygous for a *ms26* mutant excision allele, (*ms26*) was repeatedly crossed with bulked pollen from maize Hi-type II plants (Armstrong 1994, In: Freeling and Walbot (eds). The Maize Handbook. Springer, New York, pp 663-671) resulting in the introgression of this *ms26* allele in transformation amenable maize germplasm over multiple generations. The resultant source of material for transformation consisted of embryos segregating (1:1 or 3:1) for *ms26* and allowed for both transformation directly into a homozygous *ms26* background and to test the genetic complementation of the *ms26* mutation in T₀ plants. *Agrobacterum*-mediated transformation was performed according to Zhao et al. 1999, (United States Patent number 5,981,840). Genotyping and molecular analysis (integration and PTU) of transformants were done according Cigan et al., (Sex. Plant. Reprod. 1(2001) 4:135-142). Transformants with single-integration -and complete PTU were selected for further studies.

### EXAMPLE 15 - Analysis of maize transformants.

Transgenic plants (To) from Example 14 were evaluated for the whole plant morphology and analyzed for transgene transmission through pollen. No morphological difference was observed between the transgenic plants and the non-transgenic control plants except for the degree of male fertility. Transformants with single-integration and intact PTU were partial male fertile while non-transgenic control plants were completely male sterile, indicating that the expression of the *Ms26* gene complemented the homozygous recessive *ms26* male sterile phenotype. This also suggested that the pollen expression of the alpha amylase (AA) gene caused partial male sterility by disrupting the normal function of the pollen grains carrying the transgenes. Staining pollen from transformants with potassium iodide (KI), which stains starch granules, showed that approximately half of the pollen grains contained starch (black grains, non-transgenic) and the other half did not contain starch (gold grains, transgenic). The correct function of AA gene was further determined by controlled pollinations between T₀ transgenic plants and non-transgenic plants. Resultant T₁ kernels were evaluated for a red fluorescence phenotype. If the transgenes were transmitted through the pollen then the T₁ seed would contain red fluorescent kernels due to the expression of RFP in the aleurone layer. For four independent events shown in Table 3, no RFP expression was found in the T₁ seed, whereas seed from the T₀ ears themselves (T₁ seed) contained approximately 50% red fluorescent kernels.

**Table 3: Transgene transmission through pollen**

| Transgenic plants | Pollen to non-transgenic plants | | |
|---|---|---|---|
| | Kernel Red Fluoresence | | |
| | # Yellow Kernels | # Red Kernels | % |
| 42772379 | 338 | 0 | 100 |
| 42772385 | 277 | 0 | 100 |
| 42772400 | 268 | 0 | 100 |
| 42772411 | 598 | 0 | 100 |

Thus it can be seen that the invention achieves at least all of its objectives.

## Claims

1. An expression cassette comprising an alpha-amylase 1 sequence comprising:
(a) the alpha-amylase 1 coding region from *Zea mays* as set forth in Figure 24 (SEQ ID NO: 26); or
(b) a sequence having at least 90% sequence identity thereto operably linked to a male tissue-preferred promoter.

2. An expression cassette of claim 1, part (b), wherein said sequence identity is at least 95%.

3. A plant cell comprising an expression cassette of claim 1 or 2.

4. A plant comprising an expression cassette of claim 1 or 2.

5. A plant of claim 4 which is a maize, sorghum or soybean plant.

6. Use of an expression cassette of claim 1 or 2 in the repression of pollen development in a plant as defined in claim 4 or 5.

## Patentansprüche

1. Expressionskassette umfassend eine Alpha-1-Amylase-Sequenz, folgendes umfassend:
(a) die Alpha-1-Amylase-Kodierungsregion von *Zea mays,* wie in Figur 24 (SEQ ID Nr.: 26) dargelegt; oder
(b) eine Sequenz, die mindestens 90% Sequenzidentität damit aufweist, funktionsfähig mit einem männlichen Gewebe bevorzugenden Promotor verknüpft.

2. Expressionskassette nach Anspruch 1, Teil (b), wobei die Sequenzidentität mindestens 95% beträgt.

3. Pflanzenzelle umfassend eine Expressionskassette nach Anspruch 1 oder 2.

4. Pflanze umfassend eine Expressionskassette nach Anspruch 1 oder 2.

5. Pflanze nach Anspruch 4, die eine Mais-, Sorghum- oder Sojapflanze ist.

6. Verwendung einer Expressionskassette nach Anspruch 1 oder 2 bei der Hemmung der Pollenentwicklung in einer Pflanze, wie wie in Anspruch 4 oder 5 definiert ist.

## Revendications

1. Cassette d'expression comprenant une séquence d'alpha-amylase 1 comprenant :
(a) la région codant l'alpha-amylase 1 de *Zea mays* telle que définie dans la figure 24 (SEQ ID NO : 26) ; ou
(b) une séquence ayant au moins 90% d'identité séquentielle avec celle-ci, opérativement liée à un promoteur mâle à préférence tissulaire.

2. Cassette d'expression selon la revendication 1, partie (b), dans laquelle ladite identité séquentielle est d'au moins 95%.

3. Cellule de plante comprenant une cassette d'expression selon la revendication 1 ou 2.

4. Plante comprenant une cassette d'expression selon la revendication 1 ou 2.

5. Plante selon la revendication 4 qui est un plant de maïs, de sorghum ou de soja.

6. Utilisation d'une cassette d'expression selon la revendication 1 ou 2 dans la répression du développment du pollen dans une plante telle que définie dans la revendication 4 ou 5.


### SEQUENCE LISTING

<110> PIONEER HI-BRED INTERNATIONAL, INC.
<120> NUCLEOTIDE SEQUENCES MEDIATING PLANT MALE FERTILITY AND METHOD OF USING SAME
<130> 1148CR-PCT
<140> PCT/US2006/024273
   <141> 2006-06-22
<150> 11/166,609
   <151> 2005-06-24
<160> 36
<170> PatentIn Ver. 3.3
<210> 1
   <211> 1906
   <212> DNA
   <213> Zea mays
<220>
   <221> CDS
   <222> (1)..(1638)
<220>
   <221> CDS
   <222> (1642)..(1767)
<400> 1
   gaa ttc ggc acg agg gaa gct cac ctc acg ccg gcg acg cca tcg cca      48
   Glu Phe Gly Thr Arg Glu Ala His Leu Thr Pro Ala Thr Pro Ser Pro
    1               5                  10                  15
   ttc ttc cca cta gca ggg cct cac aag tac atc gcg ctc ctt ctg gtt      96
   Phe Phe Pro Leu Ala Gly Pro His Lys Tyr Ile Ala Leu Leu Leu Val
               20                  25                  30
   gtc ctc tca tgg atc ctg gtc cag agg tgg agc ctg agg aag cag aaa     144
   Val Leu Ser Trp Ile Leu Val Gln Arg Trp Ser Leu Arg Lys Gln Lys
           35                  40                  45
   ggc ccg aga tca tgg cca gtc atc ggc gca acg gtg gag cag ctg agg     192
   Gly Pro Arg Ser Trp Pro Val Ile Gly Ala Thr Val Glu Gln Leu Arg
       50                  55                  60
   aac tac cac cgg atg cac gac tgg ctt gtc ggg tac ctg tca cgg cac     240
   Asn Tyr His Arg Met His Asp Trp Leu Val Gly Tyr Leu Ser Arg His
   65                  70                  75                  80
   agg aca gtg acc gtc gac atg ccg ttc act tcc tac acc tac atc gct     288
   Arg Thr Val Thr Val Asp Met Pro Phe Thr Ser Tyr Thr Tyr Ile Ala
                   85                  90                  95
   gac ccg gtg aat gtc gag cat gtc ctc aag act aac ttc acc aat tac     336
   Asp Pro Val Asn Val Glu His Val Leu Lys Thr Asn Phe Thr Asn Tyr
               100                 105                 110
   ccc aag gga atc gtg tac aga tcc tac atg gac gtg ctc ctc ggt gac      384
   Pro Lys Gly Ile Val Tyr Arg Ser Tyr Met Asp Val Leu Leu Gly Asp
           115                 120                 125
   ggc atc ttc aac gcc gac ggc gag ctg tgg agg aag cag agg aag acg      432
   Gly Ile Phe Asn Ala Asp Gly Glu Leu Trp Arg Lys Gln Arg Lys Thr
       130                 135                 140
   gcg agt ttc gag ttc gcc tcc aag aac ctg agg gat ttc agc gcc att      480
   Ala Ser Phe Glu Phe Ala Ser Lys Asn Leu Arg Asp Phe Ser Ala Ile
   145                 150                 155                 160
   gtg ttc aga gag tac tcc ctg aag ctg tcg ggt ata ctg agc cag gca      528
   Val Phe Arg Glu Tyr Ser Leu Lys Leu Ser Gly Ile Leu Ser Gln Ala
                   165                 170                 175
   tcc aag gca ggc aaa gtt gtg gac atg cag gaa ctt tac atg agg atg      576
   Ser Lys Ala Gly Lys Val Val Asp Met Gln Glu Leu Tyr Met Arg Met
               180                 185                 190
   acg ctg gac tcc atc tgc aag gtt ggg ttc ggg gtc gag atc ggc acg      624
   Thr Leu Asp Ser Ile Cys Lys Val Gly Phe Gly Val Glu Ile Gly Thr
           195                 200                 205
   ctg tcg cca gat ctc ccc gag aac agc ttc gcg cag gcg ttc gat gcc      672
   Leu Ser Pro Asp Leu Pro Glu Asn Ser Phe Ala Gln Ala Phe Asp Ala
       210                 215                 220
   gcc aac atc atc atc acg ctg cgg ttc atc gac ccg ctg tgg cgc atc      720
   Ala Asn Ile Ile Ile Thr Leu Arg Phe Ile Asp Pro Leu Trp Arg Ile
   225                 230                 235                 240
   aag agg ttc ttc cac gtc ggg tca gag gcc ctc cta gcg cag agc atc      768
   Lys Arg Phe Phe His Val Gly Ser Glu Ala Leu Leu Ala Gln Ser Ile
                   245                 250                 255
   aag ctc gtg gac gag ttc acc tac agc gtg atc cgc cgg agg aag gcc      816
   Lys Leu Val Asp Glu Phe Thr Tyr Ser Val Ile Arg Arg Arg Lys Ala
               260                 265                 270
   gag atc gtc gag gtc cgg gcc agc ggc aaa cag gag aag atg aag cac      864
   Glu Ile Val Glu Val Arg Ala Ser Gly Lys Gln Glu Lys Met Lys His
           275                 280                 285
   gac atc ctg tca cgg ttc atc gag ctg ggc gag gcc ggc gac gac ggc      912
   Asp Ile Leu Ser Arg Phe Ile Glu Leu Gly Glu Ala Gly Asp Asp Gly
       290                 295                 300
   ggc ggc ttc ggg gac gat aag agc ctc cgg gac gtg gtg ctc aac ttc      960
   Gly Gly Phe Gly Asp Asp Lys Ser Leu Arg Asp Val Val Leu Asn Phe
   305                 310                 315                 320
   gtg atc gcc ggg cgg gac acg acg gcg acg acg ctg tcg tgg ttc acg      1008
   Val Ile Ala Gly Arg Asp Thr Thr Ala Thr Thr Leu Ser Trp Phe Thr
                   325                 330                 335
   cac atg gcc atg tcc cac ccg gac gtg gcc gag aag ctg cgc cgc gag      1056
   His Met Ala Met Ser His Pro Asp Val Ala Glu Lys Leu Arg Arg Glu
               340                 345                 350
   ctg tgc gcg ttc gag gcg gag cgc gcg cgc gag gag ggc gtc acg ctc    1104
   Leu Cys Ala Phe Glu Ala Glu Arg Ala Arg Glu Glu Gly Val Thr Leu
           355                 360                 365
   gtg ctc tgc ggc ggc gct gac gcc gac gac aag gcg ttc gcc gcc cgc    1152
   Val Leu Cys Gly Gly Ala Asp Ala Asp Asp Lys Ala Phe Ala Ala Arg
       370                 375                 380
   gtg gcg cag ttc gcg ggc ctc ctc acc tac gac agc ctc ggc aag ctg    1200
   Val Ala Gln Phe Ala Gly Leu Leu Thr Tyr Asp Ser Leu Gly Lys Leu
   385                 390                 395                 400
   gtc tac ctc cac gcc tgc gtc acc gag acg ctc cgc ctg tac ccc gcc    1248
   Val Tyr Leu His Ala Cys Val Thr Glu Thr Leu Arg Leu Tyr Pro Ala
                   405                 410                 415
   gtc cct cag gac ccc aag ggg atc ctg gag gac gac gtg ctg ccg gac    1296
   Val Pro Gln Asp Pro Lys Gly Ile Leu Glu Asp Asp Val Leu Pro Asp
               420                 425                 430
   ggg acg aag gtg agg gcc ggc ggg atg gtg acg tac gtg ccc tac tcg    1344
   Gly Thr Lys Val Arg Ala Gly Gly Met Val Thr Tyr Val Pro Tyr Ser
           435                 440                 445
   atg ggg cgg atg gag tac aac tgg ggc ccc gac gcg gcg agc ttc cgg    1392
   Met Gly Arg Met Glu Tyr Asn Trp Gly Pro Asp Ala Ala Ser Phe Arg
       450                 455                 460
   ccg gag cgg tgg atc aac gag gat ggc gcg ttc cgc aac gcg tcg ccg    1440
   Pro Glu Arg Trp Ile Asn Glu Asp Gly Ala Phe Arg Asn Ala Ser Pro
   465                 470                 475                 480
   ttc aag ttc acg gcg ttc cag gcg ggg ccg agg atc tgc ctg ggc aag    1488
   Phe Lys Phe Thr Ala Phe Gln Ala Gly Pro Arg Ile Cys Leu Gly Lys
                   485                 490                 495
   gac tcg gcg tac ctg cag atg aag atg gcg ctg gcc atc ctc ttc cgc    1536
   Asp Ser Ala Tyr Leu Gln Met Lys Met Ala Leu Ala Ile Leu Phe Arg
               500                 505                 510
   ttc tac agc ttc cgg ctg ctg gag ggg cac ccg gtg cag tac cgc atg    1584
   Phe Tyr Ser Phe Arg Leu Leu Glu Gly His Pro Val Gln Tyr Arg Met
           515                 520                 525
   atg acc atc ctc tcc atg gcg cac ggc ctc aag gtc cgc gtc tct agg    1632
   Met Thr Ile Leu Ser Met Ala His Gly Leu Lys Val Arg Val Ser Arg
       530                 535                 540
   gcc gtc tga tgt cat ggc gat ttg gat atg gat atc gtc ccg ctt aat    1680
   Ala Val     Cys His Gly Asp Leu Asp Met Asp Ile Val Pro Leu Asn
   545                 550                 555
   cca cga caa ata acg ctc gtg tta caa att tgc atg cat gca tgt aag    1728
   Pro Arg Gln Ile Thr Leu Val Leu Gln Ile Cys Met His Ala Cys Lys
   560                 565                 570                 575
   gga aag cga tgg gtt tca ttg gtg gct tgg ctt aag cct taaaaactcc      1777
   Gly Lys Arg Trp Val Ser Leu Val Ala Trp Leu Lys Pro
                   580                 585
   gtcgggtctt gcgaaccacc acatcactag tgttttgtac tctactcctc agtggaagtg 1837
   tagtgacagc atacaagttc atcatatata ttatcctctt tcttaaaaaa aaaaaaaaaa 1897
   aaactcgag 1906
<210> 2
   <211> 546
   <212> PRT
   <213> Zea mays
<400> 2
   Glu Phe Gly Thr Arg Glu Ala His Leu Thr Pro Ala Thr Pro Ser Pro
   1 5 10 15
   Phe Phe Pro Leu Ala Gly Pro His Lys Tyr Ile Ala Leu Leu Leu Val
   20 25 30
   Val Leu Ser Trp Ile Leu Val Gln Arg Trp Ser Leu Arg Lys Gln Lys
   35 40 45
   Gly Pro Arg Ser Trp Pro Val Ile Gly Ala Thr Val Glu Gln Leu Arg
   50 55 60
   Asn Tyr His Arg Met His Asp Trp Leu Val Gly Tyr Leu Ser Arg His
   65 70 75 80
   Arg Thr Val Thr Val Asp Met Pro Phe Thr Ser Tyr Thr Tyr Ile Ala
   85 90 95
   Asp Pro Val Asn Val Glu His Val Leu Lys Thr Asn Phe Thr Asn Tyr
   100 105 110
   Pro Lys Gly Ile Val Tyr Arg Ser Tyr Met Asp Val Leu Leu Gly Asp
   115 120 125
   Gly Ile Phe Asn Ala Asp Gly Glu Leu Trp Arg Lys Gln Arg Lys Thr
   130 135 140
   Ala Ser Phe Glu Phe Ala Ser Lys Asn Leu Arg Asp Phe Ser Ala Ile
   145 150 155 160
   Val Phe Arg Glu Tyr Ser Leu Lys Leu Ser Gly Ile Leu Ser Gln Ala
   165 170 175
   Ser Lys Ala Gly Lys Val Val Asp Met Gln Glu Leu Tyr Met Arg Met
   180 185 190
   Thr Leu Asp Ser Ile Cys Lys Val Gly Phe Gly Val Glu Ile Gly Thr
   195 200 205
   Leu Ser Pro Asp Leu Pro Glu Asn Ser Phe Ala Gln Ala Phe Asp Ala
   210 215 220
   Ala Asn Ile Ile Ile Thr Leu Arg Phe Ile Asp Pro Leu Trp Arg Ile
   225 230 235 240
   Lys Arg Phe Phe His Val Gly Ser Glu Ala Leu Leu Ala Gln Ser Ile
   245 250 255
   Lys Leu Val Asp Glu Phe Thr Tyr Ser Val Ile Arg Arg Arg Lys Ala
               260                 265                 270
   Glu Ile Val Glu Val Arg Ala Ser Gly Lys Gln Glu Lys Met Lys His
           275                 280                 285
   Asp Ile Leu Ser Arg Phe Ile Glu Leu Gly Glu Ala Gly Asp Asp Gly
       290                 295                 300
   Gly Gly Phe Gly Asp Asp Lys Ser Leu Arg Asp Val Val Leu Asn Phe
   305                 310                 315                 320
   Val Ile Ala Gly Arg Asp Thr Thr Ala Thr Thr Leu Ser Trp Phe Thr
                   325                 330                 335
   His Met Ala Met Ser His Pro Asp Val Ala Glu Lys Leu Arg Arg Glu
               340                 345                 350
   Leu Cys Ala Phe Glu Ala Glu Arg Ala Arg Glu Glu Gly Val Thr Leu
           355                 360                 365
   Val Leu Cys Gly Gly Ala Asp Ala Asp Asp Lys Ala Phe Ala Ala Arg
       370                 375                 380
   Val Ala Gln Phe Ala Gly Leu Leu Thr Tyr Asp Ser Leu Gly Lys Leu
   385                 390                 395                 400
   Val Tyr Leu His Ala Cys Val Thr Glu Thr Leu Arg Leu Tyr Pro Ala
                   405                 410                 415
   Val Pro Gln Asp Pro Lys Gly Ile Leu Glu Asp Asp Val Leu Pro Asp
               420                 425                 430
   Gly Thr Lys Val Arg Ala Gly Gly Met Val Thr Tyr Val Pro Tyr Ser
           435                 440                 445
   Met Gly Arg Met Glu Tyr Asn Trp Gly Pro Asp Ala Ala Ser Phe Arg
       450                 455                 460
   Pro Glu Arg Trp Ile Asn Glu Asp Gly Ala Phe Arg Asn Ala Ser Pro
   465                 470                 475                 480
   Phe Lys Phe Thr Ala Phe Gln Ala Gly Pro Arg Ile Cys Leu Gly Lys
                   485                 490                 495
   Asp Ser Ala Tyr Leu Gln Met Lys Met Ala Leu Ala Ile Leu Phe Arg
               500                 505                 510
   Phe Tyr Ser Phe Arg Leu Leu Glu Gly His Pro Val Gln Tyr Arg Met
           515                 520                 525
   Met Thr Ile Leu Ser Met Ala His Gly Leu Lys Val Arg Val Ser Arg
       530                 535                 540
   Ala Val
   545
<210> 3
   <211> 494
   <212> DNA
   <213> Sorghum sp.
<220>
   <221> modified_base
   <222> (351)
   <223> a, c, g, t, unknown or other
<220>
   <221> modified_base
   <222> (367)
   <223> a, c, g, t, unknown or other
<220>
   <221> modified_base
   <222> (369)
   <223> a, c, g, t, unknown or other
<220>
   <221> modified_base
   <222> (384)
   <223> a, c, g, t, unknown or other
<220>
   <221> modified_base
   <222> (409)
   <223> a, c, g, t, unknown or other
<220>
   <221> modified_base
   <222> (444)
   <223> a, c, g, t, unknown or other
<220>
   <221> modified_base
   <222> (462)
   <223> a, c, g, t, unknown or other
<220>
   <221> modified_base
   <222> (490)
   <223> a, c, g, t, unknown or other
<400> 3
   ggaattcggc ttatgccgtt cacttcctac acctacatcg ctgacccggt gaatgtcgag 60
   catgtcctca agactaactt caccaattac cccaaggggg acgtgtacag atcctacatg 120
   gatgtgctcc tcggtgacgg catattcaac gctgacggcg agctgtggag gaagcagagg 180
   aagacggcga gtttcgagtt cgcctccaag aacctgaggg atttcagtgc caatgttttc 240
   agagagtact ccctgaagct gtcgggcata ctgagtcagg catccaaggc aggcaaagtt 300
   gttgacatgc aggaacttta catgaggatg acactggact cgatctgcaa ngttgggttc 360
   ggggtcnana tcggcacgct gtcnccggat ctccccgaga acagcttcnc ccaagcgttc 420
   gatgccgcta acatcatcgt cacnctgcgg ttcatccacc cnctgtggcg catccagaag 480
   ttcttccccn gtca                                                   494
<210> 4
   <211> 158
   <212> PRT
   <213> Sorghum sp.
<220>
   <221> MOD_RES
   <222> (113)
   <223> Variable amino acid
<220>
   <221> MOD_RES
   <222> (119)
   <223> Variable amino acid
<220>
   <221> MOD_RES
   <222> (133)
   <223> Variable amino acid
<400> 4
   Met Pro Phe Thr Ser Tyr Thr Tyr Ile Ala Asp Pro Val Asn Val Glu
    1               5                  10                  15
   His Val Leu Lys Thr Asn Phe Thr Asn Tyr Pro Lys Gly Asp Val Tyr
               20                  25                  30
   Arg Ser Tyr Met Asp Val Leu Leu Gly Asp Gly Ile Phe Asn Ala Asp
           35                  40                  45
   Gly Glu Leu Trp Arg Lys Gln Arg Lys Thr Ala Ser Phe Glu Phe Ala
       50                  55                  60
   Ser Lys Asn Leu Arg Asp Phe Ser Ala Asn Val Phe Arg Glu Tyr Ser
   65                  70                  75                  80
   Leu Lys Leu Ser Gly Ile Leu Ser Gln Ala Ser Lys Ala Gly Lys Val
                   85                  90                  95
   Val Asp Met Gln Glu Leu Tyr Met Arg Met Thr Leu Asp Ser Ile Cys
               100                 105                 110
   Xaa Val Gly Phe Gly Val Xaa Ile Gly Thr Leu Ser Pro Asp Leu Pro
           115                 120                 125
   Glu Asn Ser Phe Xaa Gln Ala Phe Asp Ala Ala Asn Ile Ile Val Thr
       130                 135                 140
   Leu Arg Phe Ile His Pro Leu Trp Arg Ile Gln Lys Phe Phe
   145                 150                 155
<210> 5
   <211> 1092
   <212> DNA
   <213> Zea mays
<400> 5
   gaattccaag cgaggccctt gtagcagaga gtgttgctga tgcagtcggc ggaaatgagt 60
   gcgtgctgag agcaacgctg aggggttcca gggatggcaa tggctatggc aatcggctag 120
   aggtggagga caaggtggtg aggattggga gggcaaccta tggcaagttg gtgaagaggc 180
   acgcaatgag agatctattc agacttacac tggatgccgc caacaaattc aacctttaga 240
   ttttgatact gtcactccta ctttattcct tggttgggca acttccaata ggctcatgtt 300
   aatcaatgat tagtgattat tcagcaaata ttcttgtttg tttgacattt ataatatgtg 360
   gggtgagacg gattaaatat catccatgag agctttatct tcatgctctc ttgattttgg 420
   tttcagatca ttctttcagt gttcacaaga attttctcag tttggtccat gtaatttttg 480
   aagtgaggtt ccttaaattt cattatgctt cctttctttt ctagactagc aactgcatga 540
   cttttcactt tgggttcaca aattgactca caagaaaaca aattcacttt tgggttcaca 600
   aattcctctt caggatgtac ttttcacttg aactgtcatg tataggaaca aggaatggct 660
   cagtttttaa ggaacaatgt acagatttca tttcagaact ctttctggtt ggttgagttt 720
   cagacttttt gtaccaagct gatggatcac aatacttgtt tccaaagtct gataacagaa 780
   actggcaact cctaattgat aataaaaaga ataaaataca gtatcagata tctcattttc 840
   ttggttggca gatcacaaaa aggaacacaa aggctaagcc tcctacttgt tcgggagtta 900
   ggtcagggac accatatgaa tgaaagaaat cttaatttgg ggtcacacca agattgtctc 960
   tctcgaggtt ggggggtccc taaggttggt agtagcaata cccaatatat cacctaacaa 1020
   acccaatcca tgctacatac atacatagca tccatcactt gtagactgga cccttcatca 1080
   agagcaccat gg 1092
<210> 6
   <211> 267
   <212> DNA
   <213> Zea mays
<400> 6
   ccccatctca ttttcttggt tggcagatca caaaaaggaa cacaaaggct aagcctccta 60
   cttgttcggg agttaggtca gggacaccat atgaatgaaa gaaatcttaa tttggggtca 120
   caccaagatt gtctctctcg aggttggggg gtccctaagg ttggtagtag caatacccaa 180
   tatatcacct aacaaaccca atccatgcta catacataca tagcatccat cacttgtaga 240
   ctggaccctt catcaagagc accatgg 267
<210> 7
   <211> 3897
   <212> DNA
   <213> Zea mays
<400> 7
   gaattccaag cgaggccctt gtagcagaga gtgttgctga tgcagtcggc ggaaatgagt 60
   gcgtgctgag agcaacgctg aggggttcca gggatggcaa tggctatggc aatcggctag 120
   aggtggagga caaggtggtg aggattggga gggcaaccta tggcaagttg gtgaagaggc 180
   acgcaatgag agatctattc agacttacac tggatgccgc caacaaattc aacctttaga 240
   ttttgatact gtcactccta ctttattcct tggttgggca acttccaata ggctcatgtt 300
   aatcaatgat tagtgattat tcagcaaata ttcttgtttg tttgacattt ataatatgtg 360
   gggtgagacg gattaaatat catccatgag agctttatct tcatgctctc ttgattttgg 420
   tttcagatca ttctttcagt gttcacaaga attttctcag tttggtccat gtaatttttg 480
   aagtgaggtt ccttaaattt cattatgctt cctttctttt ctagactagc aactgcatga 540
   cttttcactt tgggttcaca aattgactca caagaaaaca aattcacttt tgggttcaca 600
   aattcctctt caggatgtac ttttcacttg aactgtcatg tataggaaca aggaatggct 660
   cagtttttaa ggaacaatgt acagatttca tttcagaact ctttctggtt ggttgagttt 720
   cagacttttt gtaccaagct gatggatcac aatacttgtt tccaaagtct gataacagaa 780
   actggcaact cctaattgat aataaaaaga ataaaataca gtatcagata tctcattttc 840
   ttggttggca gatcacaaaa aggaacacaa aggctaagcc tcctacttgt tcgggagtta 900
   ggtcagggac accatatgaa tgaaagaaat cttaatttgg ggtcacacca agattgtctc 960
   tctcgaggtt ggggggtccc taaggttggt agtagcaata cccaatatat cacctaacaa 1020
   acccaatcca tgctacatac atacatagca tccatcactt gtagactgga cccttcatca 1080
   agagcaccat ggaggaagct cacatcacgc cggcgacgcc atcgccattc ttcccactag 1140
   cagggcctca caagtacatc gcgctcctcc tggttgtcct ctcatggatc ctggtccaga 1200
   ggtggagcct gaggaagcag aaaggcccga gatcatggcc agtcatcggt gcaacggtgg 1260
   agcagctgag gaactaccac cggatgcacg actggcttgt cgggtacctg tcacggcaca 1320
   ggacagtgac cgtcgacatg ccgttcactt cctacaccta catcgctgac ccggtgaatg 1380
   tcgagcatgt cctcaagact aacttcacca attaccccaa ggtaaatgac ctgaactcac 1440
   tgatgttcag tcttcggaaa tcagagctga aagctgaatc gaatgtgcct gaacaccgtg 1500
   tagggaatcg tgtacagatc ctacatggac gtgctcctcg gtgacggcat cttcaacgcc 1560
   gacggcgagc tgtggaggaa gcagaggaag acggcgagtt tcgagttcgc ctccaagaac 1620
   ctgagggatt tcagcgccat tgtgttcaga gagtactccc tgaagctgtc gggtatactg 1680
   agccaggcat ccaaggcagg caaagttgtg gacatgcagg tgagatcact gctcccttgc 1740
   cattgccaac atgagcattt caacctgaga cacgagagct accttgccga ttcaggaact 1800
   ttacatgagg atgacgctgg actccatctg caaggttggg ttcggggtcg agatcggcac 1860
   gctgtcgccg gatctccccg agaacagctt cgcgcaggcg ttcgatgccg ccaacatcat 1920
   cgtcacgctg cggttcatcg acccgctgtg gcgcatcaag aggttcttcc acgtcgggtc 1980
   agaggccctc ctagcgcaga gcatcaagct cgtggacgag ttcacctaca gcgtgatccg 2040
   ccggaggaag gccgagatcg tcgaggcccg ggccagcggc aaacaggaga aggtacgtgc 2100
   acatgactgt ttcgattctt cagttcatcg tcttggccgg gatggacctg atcctgattg 2160
   attatatatc cgtgtgactt gtgaggacaa attaaaatgg gcagatgaag cacgacatcc 2220
   tgtcacggtt catcgagcta ggcgaggccg gcgacgacgg cggcggcttc ggggacgaca 2280
   agagcctccg ggacgtggtg ctcaacttcg tgatcgccgg gcgggacacg acggcgacga 2340
   cgctgtcgtg gttcacgcac atggccatgt cccacccgga cgtggccgag aagctgcgcc 2400
   gcgagctgtg cgcgttcgag gcggagcgcg cgcgcgagga gggcgtcgcg ctcgtgccct 2460
   gcggcggcgc tgacgccgac gacaaggcgt tcgccgcccg cgtggcgcag ttcgcgggcc 2520
   tcctcaccta cgacagcctc ggcaagctgg tctacctcca cgcctgcgtc accgagacgc 2580
   tccgcctgta ccccgccgtc cctcaggtga gcgcgcccga cacgcgacct ccggtccaga 2640
   gcacagcatg cagtgagtgg acctgaatgc aatgcacatg cacttgcgcg cgcgcaggac 2700
   cccaagggga tcctggagga cgacgtgctg ccggacggga cgaaggtgag ggccggcggg 2760
   atggtgacgt acgtgcccta ctcgatgggg cggatggagt acaactgggg ccccgacgcg 2820
   gcgagcttcc ggccggagcg gtggatcaac gaggatggcg cgttccgcaa cgcgtcgccg 2880
   ttcaagttca cggcgttcca ggcggggccg aggatctgcc tgggcaagga ctcggcgtac 2940
   ctgcagatga agatggcgct ggccatcctc ttgcgcttct acagcttccg gctgctggag 3000
   gggcacccgg tgcagtaccg catgatgacc atcctctcca tggcgcacgg cctcaaggtc 3060
   cgcgtctcta gggccgtctg atgtcatggc gatttgggat atcatcccgc ttaatcctta 3120
   aaaatttgca tgcatgcatg taagggaaag cgatgggttt cattggtggc ttggcttaag 3180
   ccttaaaaac tccgtcgggt cttgcgaacc accacatcac tagtgttttg tactctactc 3240
   ctcagtggaa gtgtagtgac agcatacaag ttcatcatat atattatcct ctttcttcgc 3300
   cggatgcttc ccgggacctt ttggagacca ttactgacag gcgtgtgaaa aaaaggcttc 3360
   ttctgcggcg aagttttggg ttcagagtct tggcgtcttt gcagcagaaa aaaggtttgg 3420
   aaggatctga accctgaacc gaaaatggct tcggaaatat gctcgcatcg ggcgggggcc 3480
   gtcactcggg atgacgacaa gcccacaagc agtgagagcg aagcgatctt tggagtttgg 3540
   agacactctc ggacccctcg gcgctccgcg agctcatctt cgcctcctct gtcgtgtccg 3600
   tggcggcacc gcgcccgccc gcctcgtgtt cgaccaaatc ccgcgccccg accggttcgt 3660
   gtacaacacc ctcatccgcg gcgccgcgcg cagtgacacg ccccgggacg ccgtatacat 3720
   ctataaatca tggtattgta ctttattttc aaacggcctt aacacaacca tatttttatg 3780
   gtaaacacgt tcaaaattga cacaaattta aaacaggcac aaaccgtagc taaacataag 3840
   agaatgagag acaacccaaa ggttagagat gaaataagct gagtaaacga cgaattc    3897
<210> 8
   <211> 360
   <212> DNA
   <213> Zea mays
<400> 8
   caggacccca aggggatcct ggaggacgac gtgctgccgg acgggacgaa ggtgagggcc 60
   ggcgggatgg tgacgtacgt gccctactcg atggggcgga tggagtacaa ctggggcccc 120
   gacgcggcga gcttccggcc ggaggcccgg agcggtggat caacgaggat ggcgcgttcc 180
   gcaacgcgtc gccgttcaag ttcacggcgt tccaggcggg gccgaggatc tgcctgggca 240
   aggactcggc gtacctgcag atgaagatgg cgctggccat cctcttgcgc ttctacagct 300
   tccggctgct ggaggggcac ccggtgcagt accgcatgat gaccatcctc tccatggcgc 360
<210> 9
   <211> 352
   <212> DNA
   <213> Zea mays
<400> 9
   caggacccca aggggatcct ggaggacgac gtgctgccgg acgggacgaa ggtgagggcc 60
   ggcgggatgg tgacgtacgt gccctactcg atggggcgga tggagtacaa ctggggcccc 120
   gacgcggcga gcttccggcc ggagcggtgg atcaacgagg atggcgcgtt ccgcaacgcg 180
   tcgccgttca agttcacggc gttccaggcg gggccgagga tctgcctggg caaggactcg 240
   gcgtacctgc agatgaagat ggcgctggcc atcctcttcc gcttctacag cttccggctg 300
   ctggaggggc acccggtgca gtaccgcatg atgaccatcc tctccatggc gc 352
<210> 10
   <211> 1440
   <212> DNA
   <213> Zea mays
<400> 10
   cggagctagg ggtgaaaacg ggtagggtac ccgaaacggg taccggatac ggatactgat 60
   tcgggaccat ttttcggata cggatacggg tattttttag attcgggacg gatacgggta 120
   atacccggat agtatggctt cggattcggg tcggatacgg agcgagtact acccggtaaa 180
   tacccggata ctcgggtcgg ataccgggta cccggaattc gggtacccgt tttttctttt 240
   tctgcaaaat aatatagtta taaaatcata acttttacat atgaaatcgg atgaagataa 300
   agtttatatg aaaattgtag agctcgaaga gatctataac tttgtagtac atcacatttt 360
   tgtttaaaca tatctttagg ccaaaatcat taaaataatg tctaaattta tatcaaaata 420
   atagacttta tcattttcat gtggggactt aagattatat ccatgtggga acttaggatt 480
   atctttttat aaactattta ttaatattgg taacttattt gcaattttcg gtcgacgcta 540
   caatattttt atgaatttaa ttgtattttg atgattttct acaacaagaa attaataata 600
   caccaaatag cctaaaaaat tcatggattt ttacggggac acaacatata tccacatata 660
   gttctcaaaa acatttggac tataaaatcc acaagatgtt ggtgtttctt ccattctact 720
   cccacttatt gcgtgagtta catgtgaaat cattttatgt atcgaagttt caacataatt 780
   aatatttcac ttatcatttt catgtggcga cttgaggttt tatttgaata gaatgtttat 840
   ttgttttggt aagctttttg cattttggat caaactagtg tatttatgaa ttttaattat 900
   actttgatga ttttatgtag aaagaaatta ataatgtata aatagcctca gaaatctatg 960
   aaattatacg aaggtacaac atatggccac atatagtcat aacaaataat gggaccataa 1020
   aatccacagg atgtcaacgt ttcttctatt ttatttccac ttattgcgtg agttacacgt 1080
   gaaatcactc taagtatcca agtttcaaca taatcaatac ttcactttac catttttacg 1140
   tgggaacttg agattatctt ctattaaatg cttattagta ttaatttact tgcaatttcg 1200
   tggtcgaaca agaatatttt ttgataacca attaatgcat tatccgacaa gtatccgata 1260
   tccgatcaaa taatatccgt atccgtcact tatccgctcg gataaatatc cggtccctgt 1320
   atccgtatcc gtcccgtttc taactatccg tatccgatcc cgaatccgtt ttaaatacat 1380
   tagggtagga tacaggatga gctaatatcc gtccgtatcc gcccgttttc acccctagcc 1440
<210> 11
   <211> 4182
   <212> DNA
   <213> Zea mays
<400> 11
   aactgcatga cttttcactt tgggttcaca aattgactca caagaaaaca aattcacttt 60
   tgggttcaca aattcctctt caggatgtac ttttcacttg aaactgtcat gtataggaac 120
   aaggaatggc tcagttttta aggaacaatg tacagatttc atttcagaac tctttctggt 180
   tggttgagtt tcagactttt tgtaccaagc tgatggatca caatacttgt ttccaaagtc 240
   tgataacaga aactggcaac tcctaattga taataaaaag aataaaatac agtatcagat 300
   atctcatttt cttggttggc agatcacaaa aaggaacaca aaggctaagc ctcctacttg 360
   ttcgggagtt aggtcaggga caccatatga atgaaagaaa tcttaatttg gggtcacacc 420
   aagattgtct ctctcgaggt tggggggtcc ctaaggttgg tagtagcaat acccaatata 480
   tcacctaaca aacccaatcc atgctacata catacatagc atccatcact tgtagactgg 540
   acccttcatc aagagcacca tggaggaagc tcacatcacg ccggcgacgc catcgccatt 600
   cttcccacta gcagggcctc acaagtacat cgcgctcctc ctggttgtcc tctcatggat 660
   cctggtccag aggtggagcc tgaggaagca gaaaggcccg agatcatggc cagtcatcgg 720
   tgcaacggtg gagcagctga ggaactacca ccggatgcac gactggcttg tcgggtacct 780
   gtcgcggcac aggacagtga ccgtcgacat gccgttcact tcctacacct acatcgctga 840
   cccggtgaat gtcgagcatg tcctcaagac taacttcacc aattacccca aggtaaatga 900
   cctgaactca ctgatgttca gtcttcggaa atcagagctg aaagctgaat cgaatgtgcc 960
   tgaacaccgt gtagggaatc gtgtacagat cctacatgga cgtgctcctc ggtgacggca 1020
   tcttcaacgc cgacggcgag ctgtggagga agcagaggaa gacggcgagt ttcgagttcg 1080
   cctccaagaa cctgagggat ttcagcgcca ttgtgttcag agagtactcc ctgaagctgt 1140
   cgggtatact gagccaggca tccaaggcag gcaaagttgt ggacatgcag gtgagatcac 1200
   tgctcccttg ccattgccaa catgagcatt tcaacctgag acacgagagc taccttgccg 1260
   attcaggaac tttacatgag gatgacgctg gactccatct gcaaggttgg gttcggggtc 1320
   gagatcggca cgctgtcgcc ggatctcccc gagaacagct tcgcgcaggc gttcgatgcc 1380
   gccaacatca tcgtcacgct gcggttcatc gacccgctgt ggcgcatcaa gaggttcttc 1440
   cacgtcgggt cagaggccct cctagcgcag agcatcaagc tcgtggacga gttcacctac 1500
   agcgtgatcc gccggaggaa ggccgagatc gtcgaggtcc gggccagcgg caaacaggag 1560
   aaggtacgtg tacatgactg tttcgattct tcagttcatc gtcttggccg ggatggacct 1620
   gatcctgatt gattatatat ccgtgtgact tgtgaggaca aattaaaatg ggcagatgaa 1680
   gcacgacatc ctgtcacggt tcatcgagct aggcgaggcc ggcgacgacg gcggcggctt 1740
   cggggacgac aagagcctcc gggacgtggt gctcaacttc gtgatcgccg ggcgggacac 1800
   gacggcgacg acgctgtcgt ggttcacgca catggccatg tcccacccgg acgtggccga 1860
   gaagctgcgc cgcgagctgt gcgcgttcga ggcggagcgc gcgcgcgagg agggcgtcgc 1920
   gctcgtgccc tgcggcggcg ctgacgccga cgacaaggcg ttcgccgccc gcgtggcgca 1980
   gttcgcgggc ctcctcacct acgacagcct cggcaagctg gtctacctcc acgcctgcgt 2040
   caccgagacg ctccgcctgt accccgccgt ccctcaggtg agcgcgcccg acacgacctc 2100
   cggtccgcga tgcaacgcat atgtggctgt ccgcagagca cagcatgcag tgagtggacc 2160
   tgaatgcact atgcaatgca cttgcgcgcg cgcaggaccc caaggggatc ctggaggacg 2220
   acgtgctgcc ggacgggacg aaggtgaggg ccggcgggat ggtgacgtac gtgccctact 2280
   cgatggggcg gatggagtac aactggggcc ccgacgcggc gagcttccgg ccggagctag 2340
   gggtgaaaac gggtagggta cccgaaacgg gtaccggata cggatactga ttcgggacca 2400
   tttttcggat acggatacgg gtatttttta gattcgggac ggatacgggt aatacccgga 2460
   tagtatggct tcggattcgg gtcggatacg gagcgagtac tacccggtaa atacccggat 2520
   actcgggtcg gataccgggt acccggaatt cgggtacccg tttttctttt ttctgcaaaa 2580
   taatatagtt ataaaatcat aacttttaca tatgaaatcg gatgaagata aagtttatat 2640
   gaaaattgta gagctcgaag agatctataa ctttgtagta catcacattt ttgtttaaac 2700
   atatctttag gccaaaatca ttaaaataat gtctaaattt atatcaaaat aatagacttt 2760
   atcattttca tgtggggact taagattata tccatgtggg aacttaggat tatcttttta 2820
   taaactattt attaatattg gtaacttatt tgcaattttc ggtcgacgct acaatatttt 2880
   tatgaattta attgtatttt gatgattttc tacaacaaga aattaataat acaccaaata 2940
   gcctaaaaaa ttcatggatt tttacgggga cacaacatat atccacatat agttctcaaa 3000
   aacatttgga ctataaaatc cacaagatgt tggtgtttct tccattctac tcccacttat 3060
   tgcgtgagtt acatgtgaaa tcattttatg tatcgaagtt tcaacataat taatatttca 3120
   cttatcattt tcatgtggcg acttgaggtt ttatttgaat agaatgttta tttgttttgg 3180
   taagcttttt gcattttgga tcaaactagt gtatttatga attttaatta tactttgatg 3240
   attttatgta gaaagaaatt aataatgtat aaatagcctc agaaatctat gaaattatac 3300
   gaaggtacaa catatggcca catatagtca taacaaataa tgggaccata aaatccacag 3360
   gatgtcaacg tttcttctat tttatttcca cttattgcgt gagttacacg tgaaatcact 3420
   ctaagtatcc aagtttcaac ataatcaata cttcacttta ccatttttac gtgggaactt 3480
   gagattatct tctattaaat gcttattagt attaatttac ttgcaatttc gtggtcgaac 3540
   aagaatattt tttgataacc aattaatgca ttatccgaca agtatccgat atccgatcaa 3600
   ataatatccg tatccgtcac ttatccgctc ggataaatat ccggtccctg tatccgtatc 3660
   cgtcccgttt ctaactatcc gtatccgatc ccgaatccgt tttaaataca ttagggtagg 3720
   atacaggatg agctaatatc cgtccgtatc cgcccgtttt cacccctagc cggagcggtg 3780
   gatcaacgag gatggcgcgt tccgcaacgc gtcgccgttc aagttcacgg cgttccaggc 3840
   ggggccgagg atctgcctgg gcaaggactc ggcgtacctg cagatgaaga tggcgctggc 3900
   catccttctt gcgcttctac agcttccggc tgctggaggg gcacccggtg cagtaccgca 3960
   tgatgaccat cctctccatg gcgcacggcc tcaaggtccg cgtctctagg gccgtctgat 4020
   gtcatggcga tttgggatat catcccgctt aatccacgac aaataacgtt cgtgttacaa 4080
   atttgcatgc atgcatgtaa gggaaagcga tgggtttcat tggtggcttg gcttaagcct 4140
   taaaaactcc gtcgggttct tgcgaaccac cacatcacta ga                   4182
<210> 12
   <211> 505
   <212> PRT
   <213> Arabidopsis thaliana
<400> 12
   Leu Val Ile Ala Cys Met Val Thr Ser Trp Ile Phe Leu His Arg Trp
   1               5                   10                  15
   Gly Gln Arg Asn Lys Ser Gly Pro Lys Thr Trp Pro Leu Val Gly Ala
   20 25 30
   Ala Ile Glu Gln Leu Thr Asn Phe Asp Arg Met His Asp Trp Leu Val
   35 40 45
   Glu Tyr Leu Tyr Asn Ser Arg Thr Val Val Val Pro Met Pro Phe Thr
   50 55 60
   Thr Tyr Thr Tyr Ile Ala Asp Pro Ile Asn Val Glu Tyr Val Leu Lys
   65 70 75 80
   Thr Asn Phe Ser Asn Tyr Pro Lys Gly Glu Thr Tyr His Ser Tyr Met
   85 90 95
   Glu Val Leu Leu Gly Asp Gly Ile Phe Asn Ser Asp Gly Glu Leu Trp
   100 105 110
   Arg Lys Gln Arg Lys Thr Ala Ser Phe Glu Phe Ala Ser Lys Asn Leu
   115 120 125
   Arg Asp Phe Ser Thr Val Val Phe Lys Glu Tyr Ser Leu Lys Leu Phe
   130 135 140
   Thr Ile Leu Ser Gln Ala Ser Phe Lys Glu Gln Gln Val Asp Met Gln
   145 150 155 160
   Glu Leu Leu Met Arg Met Thr Leu Asp Ser Ile Cys Lys Val Gly Phe
   165 170 175
   Gly Val Glu Ile Gly Thr Leu Ala Pro Glu Leu Pro Glu Asn His Phe
   180 185 190
   Ala Lys Ala Phe Asp Thr Ala Asn Ile Ile Val Thr Leu Arg Phe Ile
   195 200 205
   Asp Pro Leu Trp Lys Met Lys Lys Phe Leu Asn Ile Gly Ser Glu Ala
   210 215 220
   Leu Leu Gly Lys Ser Ile Lys Val Val Asn Asp Phe Thr Tyr Ser Val
   225 230 235 240
   Ile Arg Arg Arg Lys Ala Glu Leu Leu Glu Ala Gln Val Lys His Asp
   245 250 255
   Ile Leu Ser Arg Phe Ile Glu Ile Ser Asp Asp Pro Asp Ser Lys Glu
   260 265 270
   Thr Glu Lys Ser Leu Arg Asp Ile Val Leu Asn Phe Val Ile Ala Gly
   275 280 285
   Arg Asp Thr Thr Ala Thr Thr Leu Thr Trp Ala Ile Tyr Met Ile Met
   290 295 300
   Met Asn Glu Asn Val Ala Glu Lys Leu Tyr Ser Glu Leu Gln Glu Leu
   305 310 315 320
   Glu Lys Glu Ser Ala Glu Ala Thr Asn Thr Ser Leu His Gln Tyr Asp
   325 330 335
   Thr Glu Asp Phe Asn Ser Phe Asn Glu Lys Val Thr Glu Phe Ala Gly
               340                 345                 350
   Leu Leu Asn Tyr Asp Ser Leu Gly Lys Leu His Tyr Leu His Ala Val
           355                 360                 365
   Ile Thr Glu Thr Leu Arg Leu Tyr Pro Ala Val Pro Gln Asp Pro Lys
       370                 375                 380
   Gly Val Leu Glu Asp Asp Met Leu Pro Asn Gly Thr Lys Val Lys Ala
   385                 390                 395                 400
   Gly Gly Met Val Thr Tyr Val Pro Tyr Ser Met Gly Arg Met Glu Tyr
                   405                 410                 415
   Asn Trp Gly Ser Asp Ala Ala Leu Phe Lys Pro Glu Arg Trp Leu Lys
               420                 425                 430
   Asp Gly Val Phe Gln Asn Ala Ser Pro Phe Lys Phe Thr Ala Phe Gln
           435                 440                 445
   Ala Gly Pro Arg Ile Cys Leu Gly Lys Asp Ser Ala Tyr Leu Gln Met
       450                 455                 460
   Lys Met Ala Met Ala Ile Leu Cys Arg Phe Tyr Lys Phe His Leu Val
   465                 470                 475                 480
   Pro Asn His Pro Val Lys Tyr Arg Met Met Thr Ile Leu Ser Met Ala
                   485                 490                 495
   His Gly Leu Lys Val Thr Val Ser Arg
               500                 505
<210> 13
   <211> 518
   <212> PRT
   <213> Zea mays
<400> 13
   Ile Ala Leu Leu Leu Val Val Leu Ser Trp Ile Leu Val Gln Arg Trp
   1               5                   10                  15
   Ser Leu Arg Lys Gln Lys Gly Pro Arg Ser Trp Pro Val Ile Gly Ala
               20                  25                  30
   Thr Val Glu Gln Leu Arg Asn Tyr His Arg Met His Asp Trp Leu Val
           35                  40                  45
   Gly Tyr Leu Ser Arg His Arg Thr Val Thr Val Asp Met Pro Phe Thr
       50                  55                  60
   Ser Tyr Thr Tyr Ile Ala Asp Pro Val Asn Val Glu His Val Leu Lys
   65                  70                  75                  80
   Thr Asn Phe Thr Asn Tyr Pro Lys Gly Ile Val Tyr Arg Ser Tyr Met
                   85                  90                  95
   Asp Val Leu Leu Gly Asp Gly Ile Phe Asn Ala Asp Gly Glu Leu Trp
               100                 105                 110
   Arg Lys Gln Arg Lys Thr Ala Ser Phe Glu Phe Ala Ser Lys Asn Leu
           115                 120                 125
   Arg Asp Phe Ser Ala Ile Val Phe Arg Glu Tyr Ser Leu Lys Leu Ser
       130                 135                 140
   Gly Ile Leu Ser Gln Ala Ser Lys Ala Gly Lys Val Val Asp Met Gln
   145                 150                 155                 160
   Glu Leu Tyr Met Arg Met Thr Leu Asp Ser Ile Cys Lys Val Gly Phe
                   165                 170                 175
   Gly Val Glu Ile Gly Thr Leu Ser Pro Asp Leu Pro Glu Asn Ser Phe
               180                 185                 190
   Ala Gln Ala Phe Asp Ala Ala Asn Ile Ile Ile Thr Leu Arg Phe Ile
           195                 200                 205
   Asp Pro Leu Trp Arg Ile Lys Arg Phe Phe His Val Gly Ser Glu Ala
       210                 215                 220
   Leu Leu Ala Gln Ser Ile Lys Leu Val Asp Glu Phe Thr Tyr Ser Val
   225                 230                 235                 240
   Ile Arg Arg Arg Lys Ala Glu Ile Val Glu Val Arg Ala Ser Gly Lys
                   245                 250                 255
   Gln Glu Lys Met Lys His Asp Ile Leu Ser Arg Phe Ile Glu Leu Gly
               260                 265                 270
   Glu Ala Gly Asp Asp Gly Gly Gly Phe Gly Asp Asp Lys Ser Leu Arg
           275                 280                 285
   Asp Val Val Leu Asn Phe Val Ile Ala Gly Arg Asp Thr Thr Ala Thr
       290                 295                 300
   Thr Leu Ser Trp Phe Thr His Met Ala Met Ser His Pro Asp Val Ala
   305                 310                 315                 320
   Glu Lys Leu Arg Arg Glu Leu Cys Ala Phe Glu Ala Glu Arg Ala Arg
                   325                 330                 335
   Glu Glu Gly Val Thr Leu Val Leu Cys Gly Gly Ala Asp Ala Asp Asp
               340                 345                 350
   Lys Ala Phe Ala Ala Arg Val Ala Gln Phe Ala Gly Leu Leu Thr Tyr
           355                 360                 365
   Asp Ser Leu Gly Lys Leu Val Tyr Leu His Ala Cys Val Thr Glu Thr
       370                 375                 380
   Leu Arg Leu Tyr Pro Ala Val Pro Gln Asp Pro Lys Gly Ile Leu Glu
   385                 390                 395                 400
   Asp Asp Val Leu Pro Asp Gly Thr Lys Val Arg Ala Gly Gly Met Val
                   405                 410                 415
   Thr Tyr Val Pro Tyr Ser Met Gly Arg Met Glu Tyr Asn Trp Gly Pro
               420                 425                 430
   Asp Ala Ala Ser Phe Arg Pro Glu Arg Trp Ile Asn Glu Asp Gly Ala
           435                 440                 445
   Phe Arg Asn Ala Ser Pro Phe Lys Phe Thr Ala Phe Gln Ala Gly Pro
       450                 455                 460
   Arg Ile Cys Leu Gly Lys Asp Ser Ala Tyr Leu Gln Met Lys Met Ala
   465                 470                 475                 480
   Leu Ala Ile Leu Phe Arg Phe Tyr Ser Phe Arg Leu Leu Glu Gly His
                   485                 490                 495
   Pro Val Gln Tyr Arg Met Met Thr Ile Leu Ser Met Ala His Gly Leu
               500                 505                 510
   Lys Val Arg Val Ser Arg
           515
<210> 14
   <211> 128
   <212> PRT
   <213> Zea mays
<400> 14
   Gln Asp Pro Lys Gly Ile Leu Glu Asp Asp Val Leu Pro Asp Gly Thr
   1               5                   10                  15
   Lys Val Arg Ala Gly Gly Met Val Thr Tyr Val Pro Tyr Ser Met Gly
               20                  25                  30
   Arg Met Glu Tyr Asn Trp Gly Pro Asp Ala Ala Ser Phe Arg Pro Glu
           35                  40                  45
   Arg Trp Ile Asn Glu Asp Gly Ala Phe Arg Asn Ala Ser Pro Phe Lys
       50                  55                  60
   Phe Thr Ala Phe Gln Ala Gly Pro Arg Ile Cys Leu Gly Lys Asp Ser
   65                  70                  75                  80
   Ala Tyr Leu Gln Met Lys Met Ala Leu Ala Ile Leu Phe Arg Phe Tyr
                   85                  90                  95
   Ser Phe Arg Leu Leu Glu Gly His Pro Val Gln Tyr Arg Met Met Thr
               100                 105                 110
   Ile Leu Ser Met Ala His Gly Leu Lys Val Arg Val Ser Arg Ala Val
           115                 120                 125
<210> 15
   <211> 128
   <212> PRT
   <213> Zea mays
<400> 15
   Gln Asp Pro Lys Gly Ile Leu Glu Asp Asp Val Leu Pro Asp Gly Thr
    1               5                  10                  15
   Lys Val Arg Ala Gly Gly Met Val Thr Tyr Val Pro Tyr Ser Met Gly
               20                  25                  30
   Arg Met Glu Tyr Asn Trp Gly Pro Asp Ala Ala Ser Phe Arg Pro Glu
           35                  40                  45
   Arg Trp Ile Asn Glu Asp Gly Ala Phe Arg Asn Ala Ser Pro Phe Lys
       50                  55                  60
   Phe Thr Ala Phe Gln Ala Gly Pro Arg Ile Cys Leu Gly Lys Asp Ser
   65                  70                  75                  80
   Ala Tyr Leu Gln Met Lys Met Ala Leu Ala Ile Leu Phe Arg Phe Tyr
                   85                  90                  95
   Ser Phe Arg Leu Leu Glu Gly His Pro Val Gln Tyr Arg Met Met Thr
               100                 105                 110
   Ile Leu Ser Met Ala His Gly Leu Lys Val Arg Val Ser Arg Ala Val
           115                 120                 125
<210> 16
   <211> 87
   <212> PRT
   <213> Zea mays
<400> 16
   Gln Asp Pro Lys Gly Ile Leu Glu Asp Asp Val Leu Pro Asp Gly Thr
    1               5                  10                  15
   Lys Val Arg Ala Gly Gly Met Val Thr Tyr Val Pro Tyr Ser Met Gly
               20                  25                  30
   Arg Met Glu Tyr Asn Trp Gly Pro Asp Ala Ala Ser Phe Arg Pro Glu
           35                  40                  45
   Ala Arg Ser Gly Gly Ser Thr Arg Met Ala Arg Ser Ala Thr Arg Arg
       50                  55                  60
   Arg Ser Ser Ser Arg Arg Ser Arg Arg Gly Arg Gly Ser Ala Trp Ala
   65                  70                  75                  80
   Arg Thr Arg Arg Thr Cys Arg
                   85
<210> 17
   <211> 1635
   <212> DNA
   <213> Oryza sativa
<400> 17
   atgaagagcc ccatggagga agctcatgca atgccagtga catcattctt cccagtagca 60
   ggaatccaca agctcatagc tatcttcctt gttgtcctct catggatctt ggtccacaag 120
   tggagcctga ggaaccagaa agggccaaga tcatggccaa tcatcggcgc gacagtggag 180
   caactgaaga actaccacag gatgcatgac tggcttgtcg agtacttgtc gaaggacagg 240
   acggtgaccg tcgacatgcc tttcacctcc tacacctaca ttgccgaccc ggtgaacgtc 300
   gagcatgtcc tgaagaccaa cttcaccaat taccccaagg gtgaagtgta caggtcttac 360
   atggatgtgc tgctcggtga tggcatattc aatgccgacg gcgagatgtg gaggaagcaa 420
   aggaagacgg cgagcttcga gtttgcctcc aagaacttga gagacttcag cactgtggtg 480
   ttcagggagt actccctgaa gctatcaagc attctgagcc aagcatgcaa ggccggcaga 540
   gttgtagaca tgcaggaatt gttcatgagg atgacactgg actcgatctg caaggtcggg 600
   tttggggttg agatcgggac gctgtcacct gatctcccgg agaacagctt tgcccaggca 660
   ttcgacgctg ccaacatcat cgtcacgctg cggttcatcg atcctctgtg gcgtctgaag 720
   aagttcttgc acgtcggatc agaggctctc ctcgagcaga gcatgaagct ggttgatgac 780
   ttcacctaca gcgtgatccg ccgccgcaag gctgagatct tgcaggctcg agccagcggc 840
   aagcaagaga agatcaagca cgacatactg tcgcggttca tcgagctcgg ggaggccggc 900
   ggcgacgagg ggggcggcag cttcggggac gacaagagcc tccgcgacgt ggtgctcaac 960
   ttcgtgatcg ccgggcgtga cacgacggcg acgacgctgt cgtggttcac gtacatggcg 1020
   atgacgcacc cggccgtcgc cgacaagctc cggcgcgagc tggccgcgtt cgaggatgag 1080
   cgcgcgcgcg aggagggcgt cgcgctcgcc gacgccgccg gcgaggcgtc gttcgcggcg 1140
   cgcgtggcgc agttcgcgtc gctgctgagc tacgacgcgg tggggaagct ggtgtacctg 1200
   cacgcgtgcg tgacggagac gctccgcctc tacccggcgg tgccgcagga ccccaagggg 1260
   atcgtggagg acgacgtgct ccccgacggc accaaggtgc gcgccggcgg gatggtgacg 1320
   tacgtgccct actccatggg gaggatggag tacaactggg gccccgacgc ggcgagcttc 1380
   cggccggagc ggtggctcag cggcgacggc ggcgcgttcc ggaacgcgtc gccgttcaag 1440
   ttcaccgcgt tccaggccgg gccgcggatc tgcctcggca aggactccgc ctacctccag 1500
   atgaagatgg cgctcgccat cctcttccgc ttctacacct tcgacctcgt cgaggaccac 1560
   cccgtcaagt accggatgat gaccatcctc tccatggctc acggcctcaa ggtccgcgtc 1620
   tccacctccg tctga                                                  1635
<210> 18
   <211> 544
   <212> PRT
   <213> Oryza sativa
<400> 18
   Met Lys Ser Pro Met Glu Glu Ala His Ala Met Pro Val Thr Ser Phe
    1               5                  10                  15
   Phe Pro Val Ala Gly Ile His Lys Leu Ile Ala Ile Phe Leu Val Val
               20                  25                  30
   Leu Ser Trp Ile Leu Val His Lys Trp Ser Leu Arg Asn Gln Lys Gly
           35                  40                  45
   Pro Arg Ser Trp Pro Ile Ile Gly Ala Thr Val Glu Gln Leu Lys Asn
       50                  55                  60
   Tyr His Arg Met His Asp Trp Leu Val Glu Tyr Leu Ser Lys Asp Arg
   65                  70                  75                  80
   Thr Val Thr Val Asp Met Pro Phe Thr Ser Tyr Thr Tyr Ile Ala Asp
                   85                  90                  95
   Pro Val Asn Val Glu His Val Leu Lys Thr Asn Phe Thr Asn Tyr Pro
               100                 105                 110
   Lys Gly Glu Val Tyr Arg Ser Tyr Met Asp Val Leu Leu Gly Asp Gly
           115                 120                 125
   Ile Phe Asn Ala Asp Gly Glu Met Trp Arg Lys Gln Arg Lys Thr Ala
       130                 135                 140
   Ser Phe Glu Phe Ala Ser Lys Asn Leu Arg Asp Phe Ser Thr Val Val
   145                 150                 155                 160
   Phe Arg Glu Tyr Ser Leu Lys Leu Ser Ser Ile Leu Ser Gln Ala Cys
                   165                 170                 175
   Lys Ala Gly Arg Val Val Asp Met Gln Glu Leu Phe Met Arg Met Thr
               180                 185                 190
   Leu Asp Ser Ile Cys Lys Val Gly Phe Gly Val Glu Ile Gly Thr Leu
           195                 200                 205
   Ser Pro Asp Leu Pro Glu Asn Ser Phe Ala Gln Ala Phe Asp Ala Ala
       210                 215                 220
   Asn Ile Ile Val Thr Leu Arg Phe Ile Asp Pro Leu Trp Arg Leu Lys
   225                 230                 235                 240
   Lys Phe Leu His Val Gly Ser Glu Ala Leu Leu Glu Gln Ser Met Lys
                   245                 250                 255
   Leu Val Asp Asp Phe Thr Tyr Ser Val Ile Arg Arg Arg Lys Ala Glu
               260                 265                 270
   Ile Leu Gln Ala Arg Ala Ser Gly Lys Gln Glu Lys Ile Lys His Asp
           275                 280                 285
   Ile Leu Ser Arg Phe Ile Glu Leu Gly Glu Ala Gly Gly Asp Glu Gly
       290                 295                 300
   Gly Gly Ser Phe Gly Asp Asp Lys Ser Leu Arg Asp Val Val Leu Asn
   305                 310                 315                 320
   Phe Val Ile Ala Gly Arg Asp Thr Thr Ala Thr Thr Leu Ser Trp Phe
                   325                 330                 335
   Thr Tyr Met Ala Met Thr His Pro Ala Val Ala Asp Lys Leu Arg Arg
               340                 345                 350
   Glu Leu Ala Ala Phe Glu Asp Glu Arg Ala Arg Glu Glu Gly Val Ala
           355                 360                 365
   Leu Ala Asp Ala Ala Gly Glu Ala Ser Phe Ala Ala Arg Val Ala Gln
       370                 375                 380
   Phe Ala Ser Leu Leu Ser Tyr Asp Ala Val Gly Lys Leu Val Tyr Leu
   385                 390                 395                 400
   His Ala Cys Val Thr Glu Thr Leu Arg Leu Tyr Pro Ala Val Pro Gln
                   405                 410                 415
   Asp Pro Lys Gly Ile Val Glu Asp Asp Val Leu Pro Asp Gly Thr Lys
               420                 425                 430
   Val Arg Ala Gly Gly Met Val Thr Tyr Val Pro Tyr Ser Met Gly Arg
           435                 440                 445
   Met Glu Tyr Asn Trp Gly Pro Asp Ala Ala Ser Phe Arg Pro Glu Arg
       450                 455                 460
   Trp Leu Ser Gly Asp Gly Gly Ala Phe Arg Asn Ala Ser Pro Phe Lys
   465                 470                 475                 480
   Phe Thr Ala Phe Gln Ala Gly Pro Arg Ile Cys Leu Gly Lys Asp Ser
                   485                 490                 495
   Ala Tyr Leu Gln Met Lys Met Ala Leu Ala Ile Leu Phe Arg Phe Tyr
               500                 505                 510
   Thr Phe Asp Leu Val Glu Asp His Pro Val Lys Tyr Arg Met Met Thr
           515                 520                 525
   Ile Leu Ser Met Ala His Gly Leu Lys Val Arg Val Ser Thr Ser Val
       530                 535                 540
<210> 19
   <211> 436
   <212> DNA
   <213> Sorghum sp.
<400> 19
   aacgaatgta tcattgtgcc taaattttta aagaattgtg gacaatttct ggtaggctga 60
   gtttcagact ttcagtacca agctgatgga tcacattctg gatccgaagt atgataacat 120
   aatctggcaa ctcctaattg taataacaat gaataacctg caaatacagt ataagagtgg 180
   ctcattttct tggttggcag atcacaaaaa ggaacacaaa ggctaagcgc caacttgtcc 240
   gggagttagg tcatggatac catatgaatg aaagaaatct taatttccgg tcacaccaag 300
   attgtctctc tcaaggttgg taacagcaat acccaatata tcacctaaca aacccagaca 360
   acactacata cataacatcc atcacttgga gactggaccc ttcatcaaga gcaccatgga 420
   ggaagctcac ctcatg                                                  436
<210> 20
   <211> 450
   <212> DNA
   <213> Oryza sativa
<400> 20
   aagcctggtt tcagttggtg acaatttaac agaattcaga tggatatggt tctgatatta 60
   gaaggtggca tacctttagt cgctgcaaac gcttcagtta tctgaacaaa acaacgaact 120
   tggctgagca ggggaaaaaa atactgtagc attcattttg tgtttacatg agtaacgatt 180
   cttttctagg tggacagatc acaaaaagaa aactaaagct aagatccaac tcctaagggt 240
   gttaggttag ggacaccata tgaatgagac aatcttaatt cttggtcaca caaagattgt 300
   ctcaaggttg gtagcatcag tgcccaatat atcacctaac tatgccatcc aaaatgctac 360
   atagcatctc ttgtagactg aacccttcat gaagagcccc atggaggaag ctcatgcaat 420
   gccagtgaca tcattcttcc cagtagcagg                                   450
<210> 21
   <211> 543
   <212> PRT
   <213> Zea mays
<400> 21
   Met Glu Glu Ala His Leu Thr Pro Ala Thr Pro Ser Pro Phe Phe Pro
   1               5                   10                  15
   Leu Ala Gly Pro His Lys Tyr Ile Ala Leu Leu Leu Val Val Leu Ser
               20                  25                  30
   Trp Ile Leu Val Gln Arg Trp Ser Leu Arg Lys Gln Lys Gly Pro Arg
           35                  40                  45
   Ser Trp Pro Val Ile Gly Ala Thr Val Glu Gln Leu Arg Asn Tyr His
       50                  55                  60
   Arg Met His Asp Trp Leu Val Gly Tyr Leu Ser Arg His Arg Thr Val
   65                  70                  75                  80
   Thr Val Asp Met Pro Phe Thr Ser Tyr Thr Tyr Ile Ala Asp Pro Val
                   85                  90                  95
   Asn Val Glu His Val Leu Lys Thr Asn Phe Thr Asn Tyr Pro Lys Gly
               100                 105                 110
   Ile Val Tyr Arg Ser Tyr Met Asp Val Leu Leu Gly Asp Gly Ile Phe
           115                 120                 125
   Asn Ala Asp Gly Glu Leu Trp Arg Lys Gln Arg Lys Thr Ala Ser Phe
       130                 135                 140
   Glu Phe Ala Ser Lys Asn Leu Arg Asp Phe Ser Ala Ile Val Phe Arg
   145                 150                 155                 160
   Glu Tyr Ser Leu Lys Leu Ser Gly Ile Leu Ser Gln Ala Ser Lys Ala
                   165                 170                 175
   Gly Lys Val Val Asp Met Gln Glu Leu Tyr Met Arg Met Thr Leu Asp
               180                 185                 190
   Ser Ile Cys Lys Val Gly Phe Gly Val Glu Ile Gly Thr Leu Ser Pro
           195                 200                 205
   Asp Leu Pro Glu Asn Ser Phe Ala Gln Ala Phe Asp Ala Ala Asn Ile
       210                 215                 220
   Ile Ile Thr Leu Arg Phe Ile Asp Pro Leu Trp Arg Ile Lys Arg Phe
   225                 230                 235                 240
   Phe His Val Gly Ser Glu Ala Leu Leu Ala Gln Ser Ile Lys Leu Val
                   245                 250                 255
   Asp Glu Phe Thr Tyr Ser Val Ile Arg Arg Arg Lys Ala Glu Ile Val
               260                 265                 270
   Glu Val Arg Ala Ser Gly Lys Gln Glu Lys Met Lys His Asp Ile Leu
           275                 280                 285
   Ser Arg Phe Ile Glu Leu Gly Glu Ala Gly Asp Asp Gly Gly Gly Phe
       290                 295                 300
   Gly Asp Asp Lys Ser Leu Arg Asp Val Val Leu Asn Phe Val Ile Ala
   305                 310                 315                 320
   Gly Arg Asp Thr Thr Ala Thr Thr Leu Ser Trp Phe Thr His Met Ala
                   325                 330                 335
   Met Ser His Pro Asp Val Ala Glu Lys Leu Arg Arg Glu Leu Cys Ala
               340                 345                 350
   Phe Glu Ala Glu Arg Ala Arg Glu Glu Gly Val Thr Leu Val Leu Cys
           355                 360                 365
   Gly Gly Ala Asp Ala Asp Asp Lys Ala Phe Ala Ala Arg Val Ala Gln
       370                 375                 380
   Phe Ala Gly Leu Leu Thr Tyr Asp Ser Leu Gly Lys Leu Val Tyr Leu
   385                 390                 395                 400
   His Ala Cys Val Thr Glu Thr Leu Arg Leu Tyr Pro Ala Val Pro Gln
                   405                 410                 415
   Asp Pro Lys Gly Ile Leu Glu Asp Asp Val Leu Pro Asp Gly Thr Lys
               420                 425                 430
   Val Arg Ala Gly Gly Met Val Thr Tyr Val Pro Tyr Ser Met Gly Arg
           435                 440                 445
   Met Glu Tyr Asn Trp Gly Pro Asp Ala Ala Ser Phe Arg Pro Glu Arg
       450                 455                 460
   Trp Ile Asn Glu Asp Gly Ala Phe Arg Asn Ala Ser Pro Phe Lys Phe
   465                 470                 475                 480
   Thr Ala Phe Gln Ala Gly Pro Arg Ile Cys Leu Gly Lys Asp Ser Ala
                   485                 490                 495
   Tyr Leu Gln Met Lys Met Ala Leu Ala Ile Leu Phe Arg Phe Tyr Ser
               500                 505                 510
   Phe Arg Leu Leu Glu Gly His Pro Val Gln Tyr Arg Met Met Thr Ile
           515                 520                 525
   Leu Ser Met Ala His Gly Leu Lys Val Arg Val Ser Arg Ala Val
       530                 535                 540
<210> 22
   <211> 532
   <212> PRT
   <213> Sorghum sp.
<400> 22
   Met Pro Ala Thr Pro Leu Phe Pro Leu Ala Gly Leu His Lys Tyr Ile
   1               5                   10                  15
   Ala Ile Leu Leu Val Val Leu Ser Trp Ala Leu Val His Arg Trp Ser
               20                  25                  30
   Leu Arg Lys Gln Lys Gly Pro Arg Ser Trp Pro Val Ile Gly Ala Thr
           35                  40                  45
   Leu Glu Gln Leu Arg Asn Tyr His Arg Met His Asp Trp Leu Val Gly
       50                  55                  60
   Tyr Leu Ser Arg His Lys Thr Val Thr Val Asp Met Pro Phe Thr Ser
   65                  70                  75                  80
   Tyr Thr Tyr Ile Ala Asp Pro Val Asn Val Glu His Val Leu Lys Thr
                   85                  90                  95
   Asn Phe Thr Asn Tyr Pro Lys Gly Asp Val Tyr Arg Ser Tyr Met Asp
               100                 105                 110
   Val Leu Leu Gly Asp Gly Ile Phe Asn Ala Asp Gly Glu Leu Trp Arg
           115                 120                 125
   Lys Gln Arg Lys Thr Ala Ser Phe Glu Phe Ala Ser Lys Asn Leu Arg
       130                 135                 140
   Asp Phe Ser Ala Asn Val Phe Arg Glu Tyr Ser Leu Lys Leu Ser Gly
   145                 150                 155                 160
   Ile Leu Ser Gln Ala Ser Lys Ala Gly Lys Val Val Asp Met Gln Glu
                   165                 170                 175
   Leu Tyr Met Arg Met Thr Leu Asp Ser Ile Cys Lys Val Gly Phe Gly
               180                 185                 190
   Val Glu Ile Gly Thr Leu Ser Pro Asp Leu Pro Glu Asn Ser Phe Ala
           195                 200                 205
   Gln Ala Phe Asp Ala Ala Asn Ile Ile Val Thr Leu Arg Phe Ile Asp
       210                 215                 220
   Pro Leu Trp Arg Val Lys Arg Phe Phe His Val Gly Ser Glu Ala Leu
   225                 230                 235                 240
   Leu Ala Gln Ser Ile Lys Leu Val Asp Glu Phe Thr Tyr Ser Val Ile
                   245                 250                 255
   Arg Arg Arg Lys Ala Glu Ile Val Glu Ala Arg Ala Ser Gly Lys Gln
               260                 265                 270
   Glu Lys Met Lys His Asp Ile Leu Ser Arg Phe Ile Glu Leu Gly Glu
           275                 280                 285
   Ala Gly Asp Asp Gly Gly Phe Gly Asp Asp Lys Ser Leu Arg Asp Val
       290                 295                 300
   Val Leu Asn Phe Val Ile Ala Gly Arg Asp Thr Thr Ala Thr Thr Leu
   305                 310                 315                 320
   Ser Trp Phe Thr His Met Ala Met Ser His Pro Asp Val Ala Glu Lys
                   325                 330                 335
   Leu Arg Arg Glu Leu Cys Ala Phe Glu Ala Glu Arg Ala Arg Glu Glu
               340                 345                 350
   Gly Val Ala Val Pro Cys Cys Gly Pro Asp Asp Asp Lys Ala Phe Ala
           355                 360                 365
   Ala Arg Val Ala Gln Phe Ala Gly Leu Leu Thr Tyr Asp Ser Leu Gly
       370                 375                 380
   Lys Leu Val Tyr Leu His Ala Cys Val Thr Glu Thr Leu Arg Leu Tyr
   385                 390                 395                 400
   Pro Ala Val Pro Gln Asp Pro Lys Gly Ile Leu Glu Asp Asp Val Leu
                   405                 410                 415
   Pro Asp Gly Thr Lys Val Arg Ala Gly Gly Met Val Thr Tyr Val Pro
               420                 425                 430
   Tyr Ser Met Gly Arg Met Glu Tyr Asn Trp Gly Pro Asp Ala Ala Ser
           435                 440                 445
   Phe Arg Pro Glu Arg Trp Ile Asn Glu Glu Gly Ala Phe Arg Asn Ala
       450                 455                 460
   Ser Pro Phe Lys Phe Thr Ala Phe Gln Ala Gly Pro Arg Ile Cys Leu
   465                 470                 475                 480
   Gly Lys Asp Ser Ala Tyr Leu Gln Met Lys Met Ala Leu Ala Ile Leu
                   485                 490                 495
   Phe Arg Phe Tyr Ser Phe Gln Leu Leu Glu Gly His Pro Val Gln Tyr
               500                 505                 510
   Arg Met Met Thr Ile Leu Ser Met Ala His Gly Leu Lys Val Arg Val
           515                 520                 525
   Ser Arg Ala Val
       530
<210> 23
   <211> 15
   <212> DNA
   <213> Artificial Sequence
<220>
   <223> Description of Artificial Sequence: Synthetic oligonucleotide
<400> 23
   taggggtgaa aacgg 15
<210> 24
   <211> 10
   <212> PRT
   <213> Artificial Sequence
<220>
   <223> Description of Artificial Sequence: Synthetic peptide
<220>
   <221> MOD_RES
   <222> (2)..(3)
   <223> Variable amino acid
<220>
   <221> MOD_RES
   <222> (5)
   <223> Variable amino acid
<220>
   <221> MOD_RES
   <222> (7)
   <223> Variable amino acid
<220>
   <221> MOD_RES
   <222> (9)
   <223> Variable amino acid
<400> 24
   Phe Xaa Xaa Gly Xaa Arg Xaa Cys Xaa Gly
   1 5 10
<210> 25
   <211> 10
   <212> PRT
   <213> Zea mays
<400> 25
   Phe Gln Ala Gly Pro Arg Ile Cys Leu Gly
   1 5 10
<210> 26
   <211> 1618
   <212> DNA
   <213> Zea mays
<220>
   <221> CDS
   <222> (108)..(1430)
<400> 26
   ggcacgagcc ggcgagccca ctcggcagtc ggcacaacca cacacacctc cacccactct 60
   ctgagataag tgaagcatct cgcgcactgt cgcagtcgca gacggag atg atg aag   116
                                                       Met Met Lys
                                                        1
   cac tcg agc agc ttg tgc ttg ctc ttc ctc ttg gcg ctc tgc acc acc   164
   His Ser Ser Ser Leu Cys Leu Leu Phe Leu Leu Ala Leu Cys Thr Thr
        5                  10                  15
   ctg ctg gcc tgc ggc ctg gtc cag gca caa gtc ctc ttc cag ggg ttt   212
   Leu Leu Ala Cys Gly Leu Val Gln Ala Gln Val Leu Phe Gln Gly Phe
    20                  25                  30                  35
   aac tgg gag tcg tgc aag cag cag gga ggc tgg tac aac agg ctc aag   260
   Asn Trp Glu Ser Cys Lys Gln Gln Gly Gly Trp Tyr Asn Arg Leu Lys
                    40                  45                  50
   gcc cag gtc gac gac atc gcc aag gcc ggc gtc acg cac gtc tgg ctg   308
   Ala Gln Val Asp Asp Ile Ala Lys Ala Gly Val Thr His Val Trp Leu
                55                  60                  65
   cct cca ccc tcg cac tcc gtc tcg cca caa ggc tac atg cca ggc cgc   356
   Pro Pro Pro Ser His Ser Val Ser Pro Gln Gly Tyr Met Pro Gly Arg
            70                  75                  80
   cta tac gac ctg gac gcg tcc aag tac ggc acg gcg gcg gag ctc aag   404
   Leu Tyr Asp Leu Asp Ala Ser Lys Tyr Gly Thr Ala Ala Glu Leu Lys
        85                  90                  95
   tcc ctg ata gcg gcg ttc cac ggc agg ggc gtg cag tgc gtg gcg gac   452
   Ser Leu Ile Ala Ala Phe His Gly Arg Gly Val Gln Cys Val Ala Asp
   100                 105                 110                 115
   atc gtc atc aac cac cgg tgc gcg gaa aag aag gac gcg cgc ggc gtg   500
   Ile Val Ile Asn His Arg Cys Ala Glu Lys Lys Asp Ala Arg Gly Val
                   120                 125                 130
   tac tgc atc ttc gag ggc ggg act ccc gac gac cgc ctg gac tgg ggc      548
   Tyr Cys Ile Phe Glu Gly Gly Thr Pro Asp Asp Arg Leu Asp Trp Gly
               135                 140                 145
   ccc ggg atg atc tgc agc gac gac acg cag tac tcg gac ggg acg ggg      596
   Pro Gly Met Ile Cys Ser Asp Asp Thr Gln Tyr Ser Asp Gly Thr Gly
           150                 155                 160
   cac cgc gac acg ggc gag ggg ttc gcg gcg gcg ccc gac atc gac cac      644
   His Arg Asp Thr Gly Glu Gly Phe Ala Ala Ala Pro Asp Ile Asp His
       165                 170                 175
   ctc aac ccg cgc gtg cag cgg gag ctc tcc gcc tgg ctc aac tgg ctc      692
   Leu Asn Pro Arg Val Gln Arg Glu Leu Ser Ala Trp Leu Asn Trp Leu
   180                 185                 190                 195
   agg tcc gac gcc gtg ggg ttc gac ggc tgg cgc ctc gac ttc gcc aag      740
   Arg Ser Asp Ala Val Gly Phe Asp Gly Trp Arg Leu Asp Phe Ala Lys
                   200                 205                 210
   ggc tac tcg ccg gcc gtc gcc aga atg tac gtg gag agc acg ggg ccg      788
   Gly Tyr Ser Pro Ala Val Ala Arg Met Tyr Val Glu Ser Thr Gly Pro
               215                 220                 225
   ccg agc ttc gtc gtc gcg gag ata tgg aac tcg ctg agc tac agc ggg      836
   Pro Ser Phe Val Val Ala Glu Ile Trp Asn Ser Leu Ser Tyr Ser Gly
           230                 235                 240
   gac ggc aag ccg gcg ccc aac cag gac cag tgc cgg cag gag ctg ctg      884
   Asp Gly Lys Pro Ala Pro Asn Gln Asp Gln Cys Arg Gln Glu Leu Leu
       245                 250                 255
   gac tgg acg cgg gcc gtc ggc ggg ccc gcc atg gcg ttc gac ttc ccc      932
   Asp Trp Thr Arg Ala Val Gly Gly Pro Ala Met Ala Phe Asp Phe Pro
   260                 265                 270                 275
   acc aag ggc ctg ctg cag gcg ggc gtg cag ggg gag ctg tgg cgg ctg      980
   Thr Lys Gly Leu Leu Gln Ala Gly Val Gln Gly Glu Leu Trp Arg Leu
                   280                 285                 290
   cgc gac agc tcc ggc aac gcg gcc ggc ctg atc ggg tgg gcg ccc gag      1028
   Arg Asp Ser Ser Gly Asn Ala Ala Gly Leu Ile Gly Trp Ala Pro Glu
               295                 300                 305
   aag gcc gtc acc ttc gtc gac aac cat gac acc ggg tcg acg cag aag      1076
   Lys Ala Val Thr Phe Val Asp Asn His Asp Thr Gly Ser Thr Gln Lys
           310                 315                 320
   ctc tgg ccg ttc cca tcc gac aag gtc atg cag ggc tac gcc tac atc      1124
   Leu Trp Pro Phe Pro Ser Asp Lys Val Met Gln Gly Tyr Ala Tyr Ile
       325                 330                 335
   ctc acc cat cca gga gtc ccc tgc att ttc tac gac cac atg ttc gac      1172
   Leu Thr His Pro Gly Val Pro Cys Ile Phe Tyr Asp His Met Phe Asp
   340                 345                 350                 355
   tgg aac ctg aag cag gag ata tcc acg ctg tct gcc atc agg gcg cgg      1220
   Trp Asn Leu Lys Gln Glu Ile Ser Thr Leu Ser Ala Ile Arg Ala Arg
                   360                 365                 370
   aac ggc atc cgc gcc ggg agc aag ctg cgg atc ctc gtg gcg gac gcg   1268
   Asn Gly Ile Arg Ala Gly Ser Lys Leu Arg Ile Leu Val Ala Asp Ala
               375                 380                 385
   gac gcg tac gtg gcc gtc gtc gac gag aag gtc atg gtg aag atc ggg   1316
   Asp Ala Tyr Val Ala Val Val Asp Glu Lys Val Met Val Lys Ile Gly
           390                 395                 400
   aca agg tac ggc gtg agc agc gtg gtc ccg tcg gat ttc cac ccg gcg   1364
   Thr Arg Tyr Gly Val Ser Ser Val Val Pro Ser Asp Phe His Pro Ala
       405                 410                 415
   gcg cac ggc aag gac tac tgc gtc tgg gag aaa gcg agc ctc cgc gtc   1412
   Ala His Gly Lys Asp Tyr Cys Val Trp Glu Lys Ala Ser Leu Arg Val
   420                 425                 430                 435
   ccg gcg ggg cgc cac ctc tagcagctca gattgctcag tcttgtgctg          1460
   Pro Ala Gly Arg His Leu
                   440
   cattgcaaac acagcagcac gacactgcat aacgtctttt ccttaatttc ctgaatttta 1520
   ccttttccta gttcaatttc atatatgtat ttctacatgt acacactatc acaatcagat 1580
   aaataaacaa gcttggtcaa aaaaaaaaaa aaaaaaaa                         1618
<210> 27
   <211> 13
   <212> PRT
   <213> Zea mays
<400> 27
   Leu Val Tyr Leu His Ala Cys Val Thr Glu Thr Leu Arg
   1               5                   10
<210> 28
   <211> 42
   <212> PRT
   <213> Zea mays
<400> 28
   Cys His Gly Asp Leu Asp Met Asp Ile Val Pro Leu Asn Pro Arg Gln
   1               5                   10                  15
   Ile Thr Leu Val Leu Gln Ile Cys Met His Ala Cys Lys Gly Lys Arg
               20                  25                  30
   Trp Val Ser Leu Val Ala Trp Leu Lys Pro
           35                  40
<210> 29
   <211> 28
   <212> PRT
   <213> Zea mays
<400> 29
   Lys Leu Arg Arg Val Leu Arg Thr Thr Thr Ser Leu Val Phe Cys Thr
   1 5 10 15
   Leu Leu Leu Ser Gly Ser Val Val Thr Ala Tyr Lys
   20 25
<210> 30
   <211> 42
   <212> PRT
   <213> Zea mays
<400> 30
   Cys His Gly Asp Leu Asp Met Asp Ile Val Pro Leu Asn Pro Arg Gln
   1 5 10 15
   Ile Thr Leu Val Leu Gln Ile Cys Met His Ala Cys Lys Gly Lys Arg
   20 25 30
   Trp Val Ser Leu Val Ala Trp Leu Lys Pro
   35 40
<210> 31
   <211> 14
   <212> PRT
   <213> Zea mays
<400> 31
   Lys Leu Arg Arg Val Leu Arg Thr Thr Thr Ser Leu Val Phe
   1 5 10
<210> 32
   <211> 24
   <212> PRT
   <213> Zea mays
<400> 32
   Arg Trp Arg Trp Pro Ser Ser Cys Ala Ser Thr Ala Ser Gly Cys Trp
   1 5 10 15
   Arg Gly Thr Arg Cys Ser Thr Ala
   20
<210> 33
   <211> 11
   <212> PRT
   <213> Zea mays
<400> 33
   Pro Ser Ser Pro Trp Arg Thr Lys Gly Glu Phe
   1 5 10
<210> 34
   <211> 42
   <212> PRT
   <213> Zea mays
<400> 34
   Cys His Gly Asp Leu Asp Met Asp Ile Val Pro Leu Asn Pro Arg Gln
   1               5                   10                  15
   Ile Thr Leu Val Leu Gln Ile Cys Met His Ala Cys Lys Gly Lys Arg
               20                  25                  30
   Trp Val Ser Leu Val Ala Trp Leu Lys Pro
           35                  40
<210> 35
   <211> 6
   <212> PRT
   <213> Zea mays
<400> 35
   Ala Gly Arg Asp Thr Thr
   1               5
<210> 36
   <211> 441
   <212> PRT
   <213> Zea mays
<400> 36
   Met Met Lys His Ser Ser Ser Leu Cys Leu Leu Phe Leu Leu Ala Leu
    1               5                  10                  15
   Cys Thr Thr Leu Leu Ala Cys Gly Leu Val Gln Ala Gln Val Leu Phe
               20                  25                  30
   Gln Gly Phe Asn Trp Glu Ser Cys Lys Gln Gln Gly Gly Trp Tyr Asn
           35                  40                  45
   Arg Leu Lys Ala Gln Val Asp Asp Ile Ala Lys Ala Gly Val Thr His
       50                  55                  60
   Val Trp Leu Pro Pro Pro Ser His Ser Val Ser Pro Gln Gly Tyr Met
   65                  70                  75                  80
   Pro Gly Arg Leu Tyr Asp Leu Asp Ala Ser Lys Tyr Gly Thr Ala Ala
                   85                  90                  95
   Glu Leu Lys Ser Leu Ile Ala Ala Phe His Gly Arg Gly Val Gln Cys
               100                 105                 110
   Val Ala Asp Ile Val Ile Asn His Arg Cys Ala Glu Lys Lys Asp Ala
           115                 120                 125
   Arg Gly Val Tyr Cys Ile Phe Glu Gly Gly Thr Pro Asp Asp Arg Leu
       130                 135                 140
   Asp Trp Gly Pro Gly Met Ile Cys Ser Asp Asp Thr Gln Tyr Ser Asp
   145                 150                 155                 160
   Gly Thr Gly His Arg Asp Thr Gly Glu Gly Phe Ala Ala Ala Pro Asp
                   165                 170                 175
   Ile Asp His Leu Asn Pro Arg Val Gln Arg Glu Leu Ser Ala Trp Leu
               180                 185                 190
   Asn Trp Leu Arg Ser Asp Ala Val Gly Phe Asp Gly Trp Arg Leu Asp
           195                 200                 205
   Phe Ala Lys Gly Tyr Ser Pro Ala Val Ala Arg Met Tyr Val Glu Ser
       210                 215                 220
   Thr Gly Pro Pro Ser Phe Val Val Ala Glu Ile Trp Asn Ser Leu Ser
   225                 230                 235                 240
   Tyr Ser Gly Asp Gly Lys Pro Ala Pro Asn Gln Asp Gln Cys Arg Gln
                   245                 250                 255
   Glu Leu Leu Asp Trp Thr Arg Ala Val Gly Gly Pro Ala Met Ala Phe
               260                 265                 270
   Asp Phe Pro Thr Lys Gly Leu Leu Gln Ala Gly Val Gln Gly Glu Leu
           275                 280                 285
   Trp Arg Leu Arg Asp Ser Ser Gly Asn Ala Ala Gly Leu Ile Gly Trp
       290                 295                 300
   Ala Pro Glu Lys Ala Val Thr Phe Val Asp Asn His Asp Thr Gly Ser
   305                 310                 315                 320
   Thr Gln Lys Leu Trp Pro Phe Pro Ser Asp Lys Val Met Gln Gly Tyr
                   325                 330                 335
   Ala Tyr Ile Leu Thr His Pro Gly Val Pro Cys Ile Phe Tyr Asp His
               340                 345                 350
   Met Phe Asp Trp Asn Leu Lys Gln Glu Ile Ser Thr Leu Ser Ala Ile
           355                 360                 365
   Arg Ala Arg Asn Gly Ile Arg Ala Gly Ser Lys Leu Arg Ile Leu Val
       370                 375                 380
   Ala Asp Ala Asp Ala Tyr Val Ala Val Val Asp Glu Lys Val Met Val
   385                 390                 395                 400
   Lys Ile Gly Thr Arg Tyr Gly Val Ser Ser Val Val Pro Ser Asp Phe
                   405                 410                 415
   His Pro Ala Ala His Gly Lys Asp Tyr Cys Val Trp Glu Lys Ala Ser
               420                 425                 430
   Leu Arg Val Pro Ala Gly Arg His Leu
           435                 440